(19) Europäisches Patentamt

European Patent Office

Office européen des brevets

(11) **EP 1 721 159 B1**

(12) **EUROPEAN PATENT SPECIFICATION**

(45) Date of publication and mention of the grant of the patent:
**10.12.2014 Bulletin 2014/50**

(21) Application number: **05732080.6**

(22) Date of filing: **18.02.2005**

(51) Int Cl.:
*C12Q 1/68* *(2006.01)*    *G01N 33/50* *(2006.01)*
*G01N 33/53* *(2006.01)*

(86) International application number:
**PCT/US2005/005711**

(87) International publication number:
**WO 2005/083429 (09.09.2005 Gazette 2005/36)**

(54) **BREAST CANCER PROGNOSTICS**

BRUSTKREBSPROGNOSE

PRONOSTICS DE CANCER DU SEIN

(84) Designated Contracting States:
**AT BE BG CH CY CZ DE DK EE ES FI FR GB GR HU IE IS IT LI LT LU MC NL PL PT RO SE SI SK TR**

(30) Priority: **20.02.2004   US 783271**
**08.12.2004   US 634430 P**

(43) Date of publication of application:
**15.11.2006   Bulletin 2006/46**

(60) Divisional application:
**10178199.5 / 2 333 112**

(73) Proprietor: **Janssen Diagnostics, LLC**
**Raritan, NJ 08869 (US)**

(72) Inventor: **WANG, Yixin**
**San Diego, CA 92130 (US)**

(74) Representative: **Bullett, Rachel Margaret et al**
**Carpmaels & Ransford LLP**
**One Southampton Row**
**London WC1B 5HA (GB)**

(56) References cited:
**WO-A-02/064741      WO-A-02/099421**
**WO-A-02/103320      WO-A-2004/078035**

• **VEER VAN 'T L J ET AL: "Gene expression profiling predicts clinical outcome of breast cancer" NATURE, NATURE PUBLISHING GROUP, LONDON, GB, vol. 415, no. 6871, 31 January 2002 (2002-01-31), pages 530-536, XP002259781 ISSN: 0028-0836 cited in the application**

EP 1 721 159 B1

**Description**

FIELD OF THE INVENTION

This invention relates to breast cancer patient prognosis based on the gene expression profiles of patient biological samples.

BACKGROUND ART

Approximately 60-70% of lymph-node-negative (LNN) breast cancer patients are cured by local or regional treatment alone. Lancet (1998a); and Lancet (1998b). Guidelines have been developed to assist clinicians in selecting patients that should receive adjuvant therapy. Recommendations most widely used are the St. Gallen criteria (Goldhirsch et al. (2003)) and the National Institutes of Health (NIH) consensus criteria. Eifel et al. (2001). These guidelines indicate 85%-90% of LNN patients as candidates for adjuvant systemic therapy.

There is a need to specifically identify a patient's risk of disease recurrence to ensure she receives appropriate therapy. Currently, there are few diagnostic tools available to identify at-risk patients. Gene expression patterns have been used to classify breast tumors into different clinically relevant subtypes. Perou et al. (2000); Sørlie et al. (2001); Sørlie et al. (2003); Gruvberger et al. (2001); van't Veer et al. (2002); van de Vijver et al. (2002); Ahr et al. (2002); Huang et al. (2003); Sotiriou et al. (2003); Woelfle et al. (2003); Ma et al. (2003); Ramaswamy et al. (2003); Chang et al. (2003); Sotiriou et al. (2003); and Hedenfalk et al. (2001).

Currently in LNN patients, the decision to apply adjuvant therapy after surgical removal of the primary tumor, and which type (endocrine- and/or chemotherapy), largely depends on patient's age, menopausal status, tumor size, tumor grade, and the steroid hormone-receptor status. These factors are accounted for in guidelines such as St. Gallen criteria and the National Institutes of Health (NIH) consensus criteria. Based on these criteria more than 85%-90% of the LNN patients would be candidates to receive adjuvant systemic therapy. There is clearly a need to identify better prognostic factors for guiding selection of treatment choices.

SUMMARY OF THE INVENTION

Recognizing the complexity of disease progression, we report here a comprehensive genome-wide assessment of gene expression to identify broadly applicable prognostic markers. Ntzani et al. (2003); and Wang et al. (2004).

The present invention encompasses a method of assessing breast cancer status in a biological sample obtained from a breast cancer patient as defined in the claims.

The present invention encompasses a method of staging breast cancer in a biological sample from a breast cancer patient as defined in the claims.

The present invention encompasses a method of determining breast cancer patient treatment protocol by in a biological sample from a breast cancer patient as defined in the claims.

The present disclosure provides a method of treating a breast cancer patient by obtaining a biological sample from a breast cancer patient; and measuring the expression levels in the sample of genes selected from those encoding mRNA: corresponding to SEQ ID NOs: 1-111; or recognized by the probe sets selected from psids corresponding to SEQ ID NOs: 1-111 as depicted in Table 10 where the gene expression levels above or below pre-determined cut-off levels are indicate a high risk of recurrence and; treating the patient with adjuvant therapy if they are a high risk patient.

The present disclosure provides a method of cross validating a prognostic gene expression profile for breast cancer patients by obtaining gene expression data from a statistically significant number of patient biological samples; randomizing sample order; setting aside data from about 10% - 50% of samples; computing, for the remaining samples, for factor of interest on all variables and selecting variables that meet a p-value cutoff (p); selecting variables that fit a prediction model using a forward search and evaluating the training error until it hits a predetermined error rate; testing the prediction model on the left-out 10-50% of samples; repeating steps c., -g. with a new set of samples removed; and continuing steps c) -g) until 100% of samples have been tested and record classification performance.

The present disclosure provides a method of independently validating a prognostic gene expression profile for breast patients by obtaining gene expression data from a statistically significant number of patient biological samples; normalizing the source variabilities in the gene expression data; computing for factor of interest on all variables that were selected previously; and testing the prediction model on the sample and record classification performance.

The present disclosure encompasses a method of generating a Relapse Hazard Score to enable prognosis of breast cancer patients by obtaining gene expression data from a statistically significant number of patient biological samples; applying univariate Cox's regression analysis to the data to obtain selected genes; applying weighted expression levels to the selected genes with standard Cox's coefficients to obtain a prediction model that can be applied as a relapse Hazard Score.

The present disclosure encompasses a method of generating a breast cancer prognostic patient report by obtaining a biological sample from the patient; measuring gene expression of the sample; applying a Relapse Hazard Score to the results of step b.; and using the results obtained in step c. to generate the report and patient reports generated thereby.

The present invention encompasses a composition comprising a probe set consisiting of all of SEQ ID NOs: 1-111 in Table 10

The present invention encompasses the use of a kit for conducting an assay to determine breast cancer prognosis in a biological sample containing: materials for detecting isolated nucleic acid sequences, their complements, or portions thereof of a combination of genes consisting of those encoding mRNA: identified by SEQ ID NOs: 1-11 in Table 10.

The present disclosure encompasses articles for assessing breast cancer status containing: materials for detecting isolated nucleic acid sequences, their complements, or portions thereof of a combination of genes selected from those encoding mRNA: corresponding to SEQ ID NOs: 1-111; or recognized by the probe sets selected from psids corresponding to SEQ ID NOs: 1-111 as depicted in Table 10.

The present disclosure encompasses a diagnostic/prognostic portfolio containing isolated nucleic acid sequences, their complements, or portions thereof of a combination of genes selected from those encoding mRNA: corresponding to SEQ ID NOs: 1-111; or recognized by the probe sets selected from psids corresponding to SEQ ID NOs: 1-111 as depicted in Table 10 where the combination is sufficient to characterize breast cancer status or risk of relapse in a biological sample.

BRIEF DESCRIPTION OF THE DRAWINGS

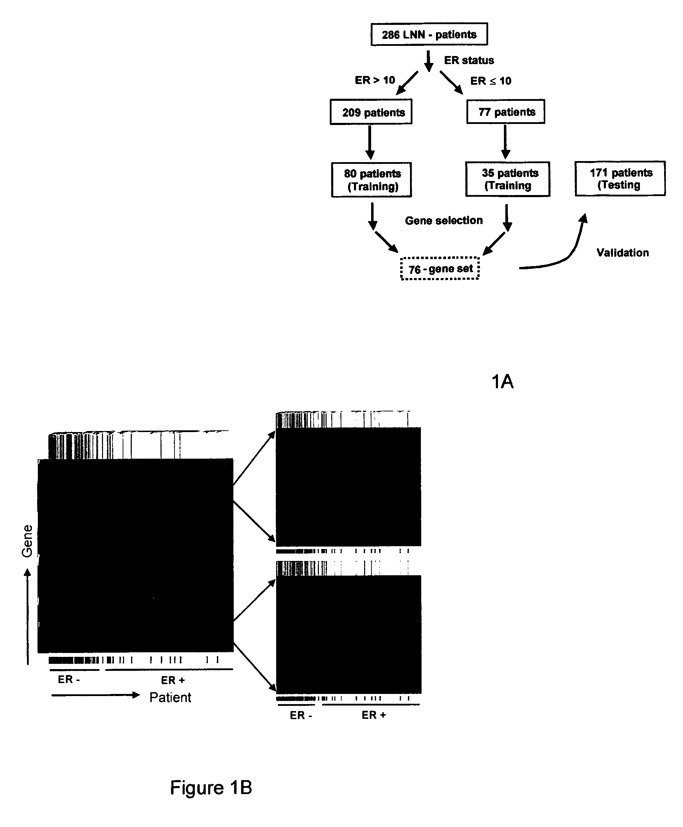

Figure 1 depicts the profile for selection of samples for analysis and unsupervised clustering analysis of gene expression data for 286 patients with lymph node-negative (LNN) breast cancer. (A). Flow chart for selecting patient samples for analysis. ER status was used to identify patient subgroups. Each subgroup was then analyzed separately in order to select markers. The patients in a subgroup were assigned to a training set or a testing set. The markers selected from each subgroup were combined to form a single signature to predict tumor recurrence for all patients in the testing set as a whole. (B). Unsupervised clustering analysis of gene expression data of 286 LNN breast cancer patients. Left panel is a view of the 17,819 informative genes. Red indicates high relative expression; green indicates relative low expression. Each column is a sample and each row is a gene. Right panel shows enlarged views of two dominant gene clusters identified from the left view that had drastic differential expression between the two patient subgroups. The upper gene cluster has a group of 282 down-regulated genes in the ER-positive subgroup. The bottom gene cluster is represented by a group of 339 up-regulated genes in the ER-positive subgroup. The label bar at the bottom of each dendrogram indicates the patient ER status measured by routine assays.

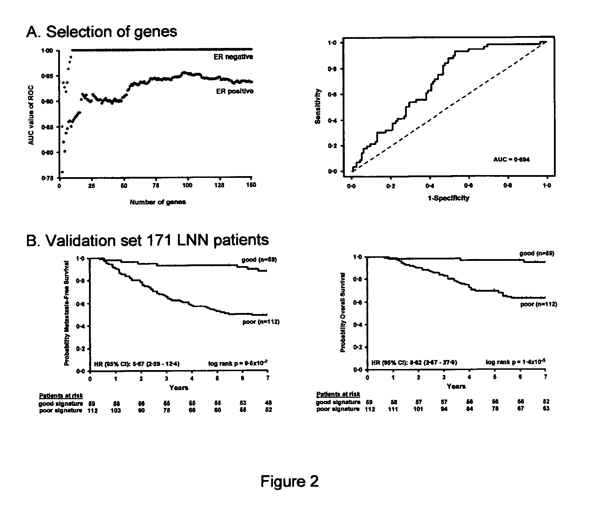

Figure 2. Establishment of the 76-gene profile and Kaplan-Meier analysis for distant-metastasis-free (DMFS) and overall survival (OS). A. Selection of genes for the prognostic signature. Gene markers for ER-positive and ER-negative groups were selected from two training sets using ROC analysis. Sixty genes were from the ER-positive group and 16 genes were from the ER-negative group (*left*), and ROC curve of the 76-gene signature derived from the analysis of the 171 patients in the testing set (*right*). B. DMFS analysis (*left*) and OS analysis (right) in the validation set of 171 LNN patients. The risk of failure for each patient was assessed based on the 76-gene signature and the threshold was determined by the training set. The log rank test was used to test for differences.

Figure 3. DMFS and OS analysis in subgroups of LNN patients. DMFS (*left*) and OS (*right*) in 84 premenopausal patients (A), 87 postmenopausal patients (B), and 79 patients with tumors of a size ranging from 10-20 mm (C). Results are from the independent patients in the validation set. The risk of recurrence for each patient was assessed based on the 76-gene signature and the threshold was determined by the training set. The log rank test was used to test for differences.

Figure 4 depicts the hierarchical clustering based on 5121 genes.

Figure 5 is a bar graph depicting the expression levels of 21 control genes (Table 7).

Figure 6 depicts a data analysis workflow.

Figure 7 depicts PCA analysis with filtered gene sets.

Figure 8 is a pie chart depicting ER status used to assign patient subgroups. Differentially expressed genes between ER-positive and ER-negative sub-clusters in both LCM and bulk tissue samples were defined by Student T-test.

Figure 9 is a series of bar graphs depicting the results of pathway analysis by Gene Ontology for genes exclusively associated with ER in LCM samples, exclusively in bulk tissues, and for those that are common to both LCM and bulk tissue.

Figure 10 shows the results of the independent validation. The flow chart in Figure 10 shows that the 132 patients were collected at four different sources. A relapse hazard score was calculated for each patient based on the expression levels of the 76-gene signature. The patients were classified into good and poor outcome groups.

Figure 11 depicts A. clinical and pathological characteristics of patients and their tumors for the validation study and B. an ROC curve of the 76-gene signature in the validation study.

Figure 12 depicts the classification result of the 132 patients in the validation study. Kaplan-Meier survival curve and log rank test on the predicted good and poor outcome groups are shown in the graph.

Figure 13 depicts the results of pathway analysis. The flow chart in Figure 13 shows the method used for selecting samples for statistical analyses. A. The 286 patients were randomly divided into a training set of 115 patients and a testing set. B. The 286 patients were randomly divided into a training set of 80% of the patients and a testing set of 20% of the patients. The training set was used to select gene markers and to build a prognostic signature. The testing set was used for validation. Both procedures were repeated 10 times.

Figure 14 depicts summaries of GO ontology analysis. Each bar represents a significantly over-represented pathway in the 76-gene signature ($p < 0.05$). The standard deviation is calculated from the results of the alternative signatures. A. Results from 10 signatures using training sets of 115 patients. B. Results from 10 signatures using training sets of 80% of the patients.

DETAILED DESCRIPTION

The present invention encompasses a method of assessing breast cancer status in a biological sample from a breast cancer patient as defined in the claims.

The present invention encompasses a method of staging breast cancer in a biological sample from a breast cancer patient; as defined by the claims. The method utilizes any classification known in the art including the TNM system American Joint Committee on Cancer www.cancerstaging.org) and comparison to stages corresponding to patients with similar gene expression profiles.

The present invention encompasses a method of determining breast cancer patient treatment protocol in a biological sample from a breast cancer patient; as defined in the claims.

The present invention encompasses a method of treating a breast cancer patient in a biological sample from a breast cancer patient; as defined in the claims.

Preferably, the SEQ ID NOs: are 36-95 for estrogen receptor (ER) patients and 96-111 for ER negative patients.

The sample can be prepared by any method known in the art including bulk tissue preparation and laser capture microdissection. Bulk tissue preparations can be obtained from a biopsy or a surgical specimen.

The above methods can further include measuring the expression level of at least one gene encoding mRNA identified by SEQ ID NOs: 112-132; Table 10. They can also include measuring the expression level of at least one gene constitutively expressed in the sample.

The above methods can further include determining the estrogen receptor (ER) status of the sample. ER status can be measured by any method known in the art including measuring the expression level of at least one gene indicative of ER status, measuring the presence of ER in the sample and measuring immunohistochemically.

The above methods can be used with samples from any biological source. Preferably, the sample is obtained from a primary tumor.

The above methods preferably have a specificity of at least 40% and a sensitivity of at least at least 90%.

The above methods can be used where the expression pattern of the genes is compared to an expression pattern indicative of a relapse patient. The comparison can be by any method known in the art including comparison of expression patterns is conducted with pattern recognition methods. Pattern recognition methods can be any known in the art including a Cox's proportional hazards analysis.

The above methods can be used where the pre-determined cut-off levels are at least 1.5-fold over- or under-expression in the sample relative to benign cells or normal tissue. Preferably, the pre-determined cut-off levels have at least a statistically significant p-value over-expression in the sample having metastatic cells relative to benign cells or normal tissue. More preferably, the p-value is less than 0.05.

The above methods can be used where gene expression is measured on a microarray or gene chip. Gene chips and microarrays suitable for use herein are also included in the invention. The microarray can be a cDNA array or an oligonucleotide array and can further contain one or more internal control reagents.

The above methods can be used where gene expression is determined by nucleic acid amplification conducted by polymerase chain reaction (PCR) of RNA extracted from the sample. The PCR can be reverse transcription polymerase chain reaction (RT-PCR). The RT-PCR can further contain one or more internal control reagents.

The above methods can be used where gene expression is detected by measuring or detecting a protein encoded by the gene. Any method known in the art can be used including detection by an antibody specific to the protein and measuring a characteristic of the gene. Suitable characteristics include, without limitation, DNA amplification, methylation, mutation and allelic variation.

The present disclosure encompasses a method of cross validating a prognostic gene expression profile for breast cancer patients by obtaining gene expression data from a statistically significant number of patient biological samples; randomizing sample order; setting aside data from about 10% - 50% of samples; computing, for the remaining samples, for factor of interest on all variables and selecting variables that meet a p-value cutoff (p); selecting variables that fit a prediction model using a forward search and evaluating the training error until it hits a predetermined error rate; testing the prediction model on the left-out 10-50% of samples; repeating steps c., -g. with a new set of samples removed; and continuing steps c) -g) until 100% of samples have been tested and record classification performance.

The cross validation method can be used where the gene expression data obtained in step h. is represented by genes selected from those encoding mRNA: corresponding to SEQ ID NOs: 3, 4, 10, 18, 37, 40, 42, 43, 45, 55, 58, 64, 67, 72-74, 76, 81, 85-86, 89, 97, 100-101, 110-111, 125 and 132-442, or 2, 3, 5, 12, 20, 25, 36, 37, 39, 40, 41, 42, 43, 45, 46, 51, 52, 53, 54, 55, 57, 58, 59, 60, 61, 62, 63, 64, 66, 67, 69, 70, 72, 73, 74, 76, 80, 81, 83, 84, 85, 86, 87, 88, 89, 90, 94, 97, 98, 101, 102, 104, 107, 110, 111, 132, 139, 142, 150, 151, 153, 154, 158, 161, 163, 167, 170, 171, 173, 175, 181, 182, 183, 186, 188, 190, 192, 204, 206, 207, 212, 215, 218, 221, 223, 225, 228, 231, 232, 236, 238, 239, 240, 241, 242, 243, 246, 248, 249, 255, 257, 267, 269, 270, 271, 273, 280, 281, 282, 288, 290, 291, 299, 301, 304, 306, 311, 314, 315, 318, 327, 328, 338, 342, 346, 348, 354, 366, 368, 371, 375, 385, 388, 391, 395, 397, 402, 405, 409, 422, 424, 428, 429, 435, 436, 440, 651 and 443-650; or recognized by the probe sets selected from the group consisting of psids corresponding to SEQ ID NOs: 3, 4, 10, 18, 37, 40, 42, 43, 45, 55, 58, 64, 67, 72-74, 76, 81, 85-86, 89, 97, 100-101, 110-111, 125 and 132-442, or 2, 3, 5, 12, 20, 25, 36, 37, 39, 40, 41, 42, 43, 45, 46, 51, 52, 53, 54, 55, 57, 58, 59, 60, 61, 62, 63, 64, 66, 67, 69, 70, 72, 73, 74, 76, 80, 81, 83, 84, 85, 86, 87, 88, 89, 90, 94, 97, 98, 101, 102, 104, 107, 110, 111, 132, 139, 142, 150, 151, 153, 154, 158, 161, 163, 167, 170, 171, 173, 175, 181, 182, 183, 186, 188, 190, 192, 204, 206, 207, 212, 215, 218, 221, 223, 225, 228, 231, 232, 236, 238, 239, 240, 241, 242, 243, 246, 248, 249, 255, 257, 267, 269, 270, 271, 273, 280, 281, 282, 288, 290, 291, 299, 301, 304, 306, 311, 314, 315, 318, 327, 328, 338, 342, 346, 348, 354, 366, 368, 371, 375, 385, 388, 391, 395, 397, 402, 405, 409, 422, 424, 428, 429, 435, 436, 440, 651 and 443-650 as depicted in Table 10.

The present disclosure encompasses a method of independently validating a prognostic gene expression profile for breast patients by obtaining gene expression data from a statistically significant number of patient biological samples; normalizing the source variabilities in the gene expression data; computing for factor of interest on all variables that were selected previously; and testing the prediction model on the sample and record classification performance.

The independent validation method can be used where the gene expression data obtained in step d. is represented by genes selected from those encoding mRNA: corresponding to SEQ ID NOs: 3, 4, 10, 18, 37, 40, 42, 43, 45, 55, 58, 64, 67, 72-74, 76, 81, 85-86, 89, 97, 100-101, 110-111, 125 and 132-442, or 2, 3, 5, 12, 20, 25, 36, 37, 39, 40, 41, 42, 43, 45, 46, 51, 52, 53, 54, 55, 57, 58, 59, 60, 61, 62, 63, 64, 66, 67, 69, 70, 72, 73, 74, 76, 80, 81, 83, 84, 85, 86, 87, 88, 89, 90, 94, 97, 98, 101, 102, 104, 107, 110, 111, 132, 139, 142, 150, 151, 153, 154, 158, 161, 163, 167, 170, 171, 173, 175, 181, 182, 183, 186, 188, 190, 192, 204, 206, 207, 212, 215, 218, 221, 223, 225, 228, 231, 232, 236, 238, 239, 240, 241, 242, 243, 246, 248, 249, 255, 257, 267, 269, 270, 271, 273, 280, 281, 282, 288, 290, 291, 299, 301, 304, 306, 311, 314, 315, 318, 327, 328, 338, 342, 346, 348, 354, 366, 368, 371, 375, 385, 388, 391, 395, 397, 402, 405, 409, 422, 424, 428, 429, 435, 436, 440, 651 and 443-650; or recognized by the probe sets selected from the group consisting of psids corresponding to SEQ ID NOs: 3, 4, 10, 18, 37, 40, 42, 43, 45, 55, 58, 64, 67, 72-74, 76, 81, 85-86, 89, 97, 100-101, 110-111, 125 and 132-442, or 2, 3, 5, 12, 20, 25, 36, 37, 39, 40, 41, 42, 43, 45, 46, 51, 52, 53, 54, 55, 57, 58, 59, 60, 61, 62, 63, 64, 66, 67, 69, 70, 72, 73, 74, 76, 80, 81, 83, 84, 85, 86, 87, 88, 89, 90, 94, 97, 98, 101, 102, 104, 107, 110, 111, 132, 139, 142, 150, 151, 153, 154, 158, 161, 163, 167, 170, 171, 173, 175, 181, 182, 183, 186, 188, 190, 192, 204, 206, 207, 212, 215, 218, 221, 223, 225, 228, 231, 232, 236, 238, 239, 240, 241, 242, 243, 246, 248, 249, 255, 257, 267, 269, 270, 271, 273, 280, 281, 282, 288, 290, 291, 299, 301, 304, 306, 311, 314, 315, 318, 327, 328, 338, 342, 346, 348, 354, 366, 368, 371, 375, 385, 388, 391, 395, 397, 402, 405, 409, 422, 424, 428, 429, 435, 436, 440, 651 and 443-650 as depicted in Table 10.

The present invention encompasses a method of generating a Relapse Hazard Score to enable prognosis of breast cancer patients by obtaining gene expression data from a statistically significant number of patient biological samples; applying univariate Cox's regression analysis to the data to obtain selected genes; applying weighted expression levels to the selected genes with standard Cox's coefficients to obtain a prediction model that can be applied as a relapse Hazard Score.

The Relapse Hazard Score can be obtained by the formula:

$$\text{Relapse Score} = A \cdot I + \sum_{i=1}^{60} I \cdot w_i x_i + B \cdot (1 - I) + \sum_{j=1}^{16} (1 - I) \cdot w_j x_j$$

where

$$I = \begin{cases} 1 & \text{if X gene level} > 10 \\ 0 & \text{if X gene level} \leq 10 \end{cases}$$

A and B are constants
$w_i$ is the standardized Cox regression coefficient for X gene + marker
$x_i$ is the expression value of ER + marker in log2 scale
$w_j$ is the standardized Cox regression coefficient for X gene - marker
$x_j$ is the expression value of ER - marker in log2 scale
X gene is selected from the group consisting of those encoding mRNA :

i. corresponding to SEQ ID NOs 1-111; or
ii. recognized by the probe sets selected from the group consisting of psids corresponding to SEQ ID NOs: 1-111 as depicted in Table 10.

The present invention encompasses a method of generating a breast cancer prognostic patient report by obtaining a biological sample from the patient; measuring gene expression of the sample; applying a Relapse Hazard Score to the results of step b.; and using the results obtained in step c. to generate the report and patient reports generated thereby. The report may contain an assessment of patient outcome and/or probability of risk relative to the patient population.

The present invention encompasses a composition comprising a probe set consisiting of all of SEQ ID NOs: 1-111 in Table 10. The composition can further contain reagents for conducting a microarray analysis, and a medium through which said nucleic acid sequences, their complements, or portions thereof are assayed.

The present invention encompasses the use of a kit for conducting an assay to determine breast cancer prognosis in a biological sample containing: materials for detecting isolated nucleic acid sequences, their complements, or portions thereof of a combination of genes consisting of those encoding mRNA: identified by SEQ ID NOs: 1-111 in Table 10. The kit can further contain reagents for conducting a microarray analysis, and a medium through which said nucleic acid sequences, their complements, or portions thereof are assayed.

The present disclosure provides articles for assessing breast cancer status containing: materials for detecting isolated nucleic acid sequences, their complements, or portions thereof of a combination of genes selected from those encoding mRNA: corresponding to SEQ ID NOs: 1-111; or recognized by the probe sets selected from psids corresponding to SEQ ID NOs: 1-111 as depicted in Table 10. The articles can further contain reagents for conducting a microarray analysis, and a medium through which said nucleic acid sequences, their complements, or portions thereof are assayed.

The microarrays provided herein can contain isolated nucleic acid sequences, their complements, or portions thereof of a combination of genes selected from those encoding mRNA: corresponding to SEQ ID NOs: 1-111; or recognized by the probe sets selected from of psids corresponding to SEQ ID NOs: 1-111 as depicted in Table 10 where the combination is sufficient to characterize breast cancer status or risk of relapse in a biological sample.

The microarray measurement or characterization is preferably at least 1.5-fold over- or under-expression. The microarray can detect a pre-determined cut-off levels are at least 1.5-fold over- or under- expression in the sample relative to benign cells or normal tissue. Preferably, the pre-determined cut-off levels have at least a statistically significant p-value over-expression in the sample having metastatic cells relative to benign cells or normal tissue. More preferably, the p-value is less than 0.05.

The present disclosure encompasses a diagnostic/prognostic portfolio containing isolated nucleic acid sequences, their complements, or portions thereof of a combination of genes selected from those encoding mRNA: corresponding to SEQ ID NOs: 1-111; or recognized by the probe sets selected from psids corresponding to SEQ ID NOs: 1-111 as depicted in Table 10 where the combination is sufficient to characterize breast cancer status or risk of relapse in a biological sample. The portfolio can detect a pre-determined cut-off levels are at least 1.5-fold over- or under- expression in the sample relative to benign cells or normal tissue. Preferably, the pre-determined cut-off levels have at least a statistically significant p-value over-expression in the sample having metastatic cells relative to benign cells or normal tissue. More preferably, the p-value is less than 0.05.

SEQ ID NOs: 1-650 are summarized in Table 10. In each SEQ ID NO: 1-650, the marker is identified by a psid or Affymetrix GeneChip Designation and represents the gene encoding any variant, allele etc. corresponding to the given SEQ ID NO. The marker is also defined as the gene encoding mRNA recognized by the probe corresponding to the given psid. Certain markers are described in more detail below.

M83, SEQ ID NO: 45, is mentioned by US patent application publication numbers 20020110547; 20040009491; and 20030236392, and PCT publication numbers WO0149716; WO0170979; WO03025138; WO03042661;

WO03016475; WO2004063355; WO2004047728; and WO2004030615.

ABLIM-s, SEQ ID NO: 43, is mentioned by US patent application publication numbers 20020131971; 20030087818; 20030166064; 20030236392; and 20040005560 and PCT publication numbers WO0036107; WO0159063; WO0160860; WO0174405; WO0177288; WO02059271; WO0212328; WO0224956; WO0229086; WO03025138; WO2004024097; WO2004037996; WO2004039956; WO2004043361; and WO2004047728.

ATAD2, SEQ ID NO: 54 is mentioned by US patent application publication numbers 20020064872; 20020085998; 20020150581; 20020156011; 20020192678; 20030069180; 20030073623; 20030104366; 20040005560; 20040013663; and 20040058340 and PCT publication numbers WO0060076; WO0142467; WO0155322; WO0160860; WO0170979; WO0179286; WO196388; WO02057414; WO02059377; WO02060317; WO02086443; WO0231198; WO0250279; WO03004989; WO03025138; WO03042661; WO03062379; WO2004047728; WO2004048938; WO2004048938; WO2004063355; and WO9938972.

C11ORF9, SEQ ID NO: 48, is mentioned by US patent application publication numbers 20030065157; and 20030073623 and PCT publication numbers WO0056880; WO0058473; WO0159063; WO0174405; WO02059260; WO0229086; WO03101283; WO2004031413; and WO2004060270.

C3, SEQ ID NO: 46, is mentioned by US patent application publication numbers 20040005560; and 20040058340 and PCT publication numbers WO9101999; WO0055350; WO0160860; WO0175067; WO0188088; WO0224956; WO0294629; WO03078572; WO2004028479; WO2004030615; WO2004041170; and WO2004060270.

CGI-41, SEQ ID NO: 65, is mentioned by US patent application publication numbers 20030073623; and 20030096982 and PCT publication numbers WO9514772; WO9940100; WO0154472; WO0157188; WO03046152; WO2004060270.

CLN8, SEQ ID NO: 36, is mentioned by EP 1 104 808 B1, US patent application numbers 20030073623; and 20040009491 and PCT publication numbers WO9845437; WO9935158; WO0142451; WO0190304; WO02090526; WO02095010; WO02102993; WO02102994; WO03004622; WO04024892; and WO04041170.

CNK1, SEQ ID NO: 60, is mentioned by US patent application publication number 20040058340 and PCT publication numbers WO9938972; and WO2004037996.

DUSP4, SEQ ID NO: 57, is mentioned by US patent application publication numbers 20030073623; 20030194704; 20040058340; and 20040110194 and PCT publication numbers WO0100828; WO0204514; WO02059377; WO02103320; WO0224956; WO03004989; WO2004018641; WO2004023973; WO2004037996; WO2004039956; WO2004043361; WO2004047728; WO2004048938; and WO9423039.

FEN1, SEQ ID NO: 62, is mentioned by US patent number 5874283, US patent application publication numbers 20020156011; 20030069180; 20030073623; and 20030194704 and PCT publication numbers WO0196388; WO03016475; WO03016500; WO03072035; WO03101283; WO2004022059; WO2004030615; WO2004039956; WO2004043361; WO2004047728; WO2004048938; WO2004060270; and WO2004079014.

FKBP2, SEQ ID NO: 63, is mentioned by US patent application publication number 20030073623 and PCT publication numbers WO02059377; WO02070737; WO02081731; WO02083898; WO0224956; WO03016475; WO03054152; WO2004039956; WO2004043361; and WO9219745.

GP73, SEQ ID NO: 37, is mentioned by US patent application publication numbers 20030022239; 20030073623; 20030236392; 20040009478; 20040034196; and 20040058340 and PCT publication numbers WO0012708; WO0061610; WO0078961; WO0140466; WO0160860; WO0177168; WO0194629; WO02059260; WO02083876; WO0224956; WO03004989; WO2004024892; WO2004031414; and WO2004053081.

H4FH, SEQ ID NO: 61, is mentioned by US patent application publication number 20020151681 and PCT publication numbers WO0055174; WO0175067; WO0224956; WO03016476; WO2004039943.

IL-18, SEQ ID NO: 39, is mentioned by US patent number 6060283, US patent application publication number 20040115636 and PCT publication numbers WO0194629; WO02057414; WO0224956; WO0229086; WO03025138; WO03042661; and WO9724441.

KIAA0748, SEQ ID NO: 56, is mentioned by PCT publication numbers WO0021991; WO02094988; and WO2004003162.

KPNA2, SEQ ID NO: 64, is mentioned by US patent application publication numbers 20020151681; 20020156011; 20030069180; and 20040058340 and PCT publication numbers WO9712967; WO9964576; WO0055174; WO0146697; WO0170979; WO0196388; WO02057414; WO02059377; WO02086443; WO02102235; WO0224956; WO03010336; WO03025138; WO03027285; WO03042661; WO2004024097; WO2004024892; WO2004028479; WO2004037996; WO2004039956; WO2004043361; WO2004047728; and WO2004063355.

MGC11335, SEQ ID NO: 66, is mentioned by US patent application publication numbers 20030073623; 20030219744; and 20030236392 and PCT publication numbers WO0144448; WO0153312; WO0157182; WO0188088; and WO2004030615.

OR12D2, SEQ ID NO: 52, is mentioned by PCT publication numbers WO0127158; WO02068652; WO02081498; WO02081517; WO030000735; and WO03091388.

ORC3, SEQ ID NO: 53, is mentioned by US patent application publication number 20040013663 and PCT publication numbers WO9810067; WO9958642; WO0060078; WO0175067; WO02059377; WO02070737; WO02102235; and WO03042661.

PLK1, SEQ ID NO: 59, is mentioned by DE4329177, US patent application publication numbers 20020081659; and 20020151681 and PCT publication numbers WO0055174; WO0055320; WO02070737; WO02086443; WO0224956; WO03003906; WO03016476; WO03018807; WO03025138; WO03042661; WO2004030615; WO2004037996; WO2004042022; WO2004047728; WO2004048938; WO2004063355; and WO2004070062.

PPP1CC, SEQ ID NO: 42, is mentioned by US patent application publication numbers 20030073623; and 20040013663 and PCT publication numbers WO03016476; WO0063438; WO0170979; WO0202623; WO02036766; WO02074237; WO0228999; WO03016475; and WO2004030615.

SMC4, SEQ ID NO: 55, is mentioned by US patent application publication numbers 20020123619; 20020168637; and 20040058340 and PCT publication numbers WO9938972; WO9964576; WO0175067; WO0190154; WO0204514; WO02059377; WO02074237; WO02086443; WO02102235; WO03003906; WO03016475; WO03025138; WO03042661; WO2003094848; WO2004024892; WO2004047728; WO2004048938; WO2004063355; and WO2004074301.

TRDL-1, SEQ ID NO: 44, is mentioned by US patent numbers 6171787; and 6440694, US patent application publication numbers 20020055474; 20020072089; 20020081659; 20030065157; 20030073623; and 20030219767 and PCT publication numbers WO9733902; WO9912965; WO9926976; WO9928462; WO9933980; WO9935170; WO9950416; WO9954460; WO0026244; WO0032776; WO0055320; WO012525 WO0177291; WO2004020593; WO2004024892; WO2004033637; and 04037996.

Yiflp, SEQ ID NO: 38, is mentioned by US patent application publication numbers 20020131971; 20030166064; and 20040037842 and PCT publication numbers WO9933981; WO9947540; WO0173027; WO02060317; WO02070737; WO02076488; WO02095010; WO0224956; WO03004622; WO03038063; WO03042661; WO03054152; WO03064589; WO2004024892; WO2004037996; WO2004047728; and WO2004048938.

Using Affymetrix Human U133a GeneChips the expression of 22,000 transcripts was analyzed using total RNA of frozen tumor samples from 286 lymph node negative (LNN) breast cancer patients who did not receive adjuvant systemic treatment. We found that genome-wide measures of gene expression can identify patterns of gene activity that subclassify tumors and provide an improved means for individual risk assessment in patients with lymph node-negative breast cancer.

In a training set of 115 tumors we identified a 76-gene signature composed of 60 genes for ER-positive and 16 genes for ER-negative patients. This signature showed 93% sensitivity and 48% specificity in a subsequent independent testing set of 171 LNN patients. The gene profile was highly informative in identifying patients who develop distant metastasis within 5 years (hazard ratio, HR: 5.67, 95% confidence interval, CI: 2.59-12.4), even when corrected for traditional prognostic factors in multivariable analysis (HR: 5.55, CI: 2.46-12.5). The 76-gene profile also represented a strong prognostic factor for the development of metastasis in the subgroups of 84 premenopausal patients (HR: 9.60, $p=1.6 \times 10^{-4}$), 87 postmenopausal patients (HR: 4.04, $p=1.7 \times 10^{-3}$) and 79 patients with a tumor size ranging from 10 to 20 mm (HR: 14.1, $p=2.3 \times 10^{-6}$) , a group of patients for which disease prognosis is particularly difficult.

In this study we provide results of an analysis of primary tumors from 286 LNN breast cancer patients of all age and tumor size groups. The patients did not receive adjuvant systemic therapy making this the first multigene assessment of prognosis without the potentially confounding contribution by predictive factors related to systemic treatment, properly validated by an independent test set, and unrestricted for tumor size and age. The gene expression based algorithm described can predict, with high confidence in LNN patients, the probability of developing distant recurrence.

The mere presence or absence of particular nucleic acid sequences in a tissue sample has only rarely been found to have diagnostic or prognostic value. Information about the expression of various proteins, peptides or mRNA, on the other hand, is increasingly viewed as important. The mere presence of nucleic acid sequences having the potential to express proteins, peptides, or mRNA (such sequences referred to as "genes") within the genome by itself is not determinative of whether a protein, peptide, or mRNA is expressed in a given cell. Whether or not a given gene capable of expressing proteins, peptides, or mRNA does so and to what extent such expression occurs, if at all, is determined by a variety of complex factors. Irrespective of difficulties in understanding and assessing these factors, assaying gene expression can provide useful information about the occurrence of important events such as tumorogenesis, metastasis, apoptosis, and other clinically relevant phenomena. Relative indications of the degree to which genes are active or inactive can be found in gene expression profiles. The gene expression profiles of this invention are used to provide a prognosis and treat patients for breast cancer.

Sample preparation requires the collection of patient samples. Patient samples used in the inventive method are those that are suspected of containing diseased cells such as epithelial cells taken from the primary tumor in a breast sample. Samples taken from surgical margins are also preferred. Most preferably, however, the sample is taken from a lymph node obtained from a breast cancer surgery. Laser Capture Microdissection (LCM) technology is one way to select the cells to be studied, minimizing variability caused by cell type heterogeneity. Consequently, moderate or small changes in gene expression between normal and cancerous cells can be readily detected. Samples can also comprise circulating epithelial cells extracted from peripheral blood. These can be obtained according to a number of methods but the most preferred method is the magnetic separation technique described in U.S. Patent 6,136,182. Once the sample containing the cells of interest has been obtained, RNA is extracted and amplified and a gene expression profile is obtained, preferably via micro-array, for genes in the appropriate portfolios.

Preferred methods for establishing gene expression profiles include determining the amount of RNA that is produced by a gene that can code for a protein or peptide. This is accomplished by reverse transcriptase PCR (RT-PCR), competitive RT-PCR, real time RT-PCR, differential display RT-PCR, Northern Blot analysis and other related tests. While it is possible to conduct these techniques using individual PCR reactions, it is best to amplify complementary DNA (cDNA) or complementary RNA (cRNA) produced from mRNA and analyze it via microarray. A number of different array configurations and methods for their production are known to those of skill in the art and are described in U.S. Patents such as: 5,445,934; 5,532,128; 5,556,752; 5,242,974; 5,384,261; 5,405,783; 5,412,087; 5,424,186; 5,429,807; 5,436,327; 5,472,672; 5,527,681; 5,529,756; 5,545,531; 5,554,501; 5,561,071; 5,571,639; 5,593,839; 5,599,695; 5,624,711; 5,658,734; and 5,700,637.

Microarray technology allows for the measurement of the steady-state mRNA level of thousands of genes simultaneously thereby presenting a powerful tool for identifying effects such as the onset, arrest, or modulation of uncontrolled cell proliferation. Two microarray technologies are currently in wide use. The first are cDNA arrays and the second are oligonucleotide arrays. Although differences exist in the construction of these chips, essentially all downstream data analysis and output are the same. The product of these analyses are typically measurements of the intensity of the signal received from a labeled probe used to detect a cDNA sequence from the sample that hybridizes to a nucleic acid sequence at a known location on the microarray. Typically, the intensity of the signal is proportional to the quantity of cDNA, and thus mRNA, expressed in the sample cells. A large number of such techniques are available and useful. Preferred methods for determining gene expression can be found in US Patents 6,271,002; 6,218,122; 6,218,114; and 6,004,755.

Analysis of expression levels is conducted by comparing signal intensities. This is best done by generating a ratio matrix of the expression intensities of genes in a test sample versus those in a control sample. For instance, the gene expression intensities from a diseased tissue can be compared with the expression intensities generated from normal tissue of the same type (e.g., diseased breast tissue sample vs. normal breast tissue sample). A ratio of these expression intensities indicates the fold-change in gene expression between the test and control samples.

Gene expression profiles can also be displayed in a number of ways. The most common method is to arrange raw fluorescence intensities or ratio matrix into a graphical dendogram where columns indicate test samples and rows indicate genes. The data are arranged so genes that have similar expression profiles are proximal to each other. The expression ratio for each gene is visualized as a color. For example, a ratio less than one (indicating down-regulation) may appear in the blue portion of the spectrum while a ratio greater than one (indicating up-regulation) may appear as a color in the red portion of the spectrum. Commercially available computer software programs are available to display such data including GeneSpring from Agilent Technologies and Partek Discover™ and Partek Infer™ software from Partek®.

Modulated genes used in the methods of the invention are described in the Examples. Differentially expressed genes are either up- or down-regulated in patients with a relapse of breast cancer relative to those without a relapse. Up regulation and down regulation are relative terms meaning that a detectable difference (beyond the contribution of noise in the system used to measure it) is found in the amount of expression of the genes relative to some baseline. In this case, the baseline is the measured gene expression of a non-relapsing patient. The genes of interest in the diseased cells (from the relapsing patients) are then either up- or down-regulated relative to the baseline level using the same measurement method. Diseased, in this context, refers to an alteration of the state of a body that interrupts or disturbs, or has the potential to disturb, proper performance of bodily functions as occurs with the uncontrolled proliferation of cells. Someone is diagnosed with a disease when some aspect of that person's genotype or phenotype is consistent with the presence of the disease. However, the act of conducting a diagnosis or prognosis includes the determination of disease/status issues such as determining the likelihood of relapse and therapy monitoring. In therapy monitoring, clinical judgments are made regarding the effect of a given course of therapy by comparing the expression of genes over time to determine whether the gene expression profiles have changed or are changing to patterns more consistent with normal tissue.

Preferably, levels of up- and down-regulation are distinguished based on fold changes of the intensity measurements of hybridized microarray probes. A 2.0 fold difference is preferred for making such distinctions (or a p-value less than 0.05). That is, before a gene is said to be differentially expressed in diseased/relapsing versus normal/non-relapsing cells, the diseased cell is found to yield at least 2 times more, or 2 times less intensity than the normal cells. The greater the fold difference, the more preferred is use of the gene as a diagnostic or prognostic tool. Genes selected for the gene expression profiles of the instant invention have expression levels that result in the generation of a signal that is distinguishable from those of the normal or non-modulated genes by an amount that exceeds background using clinical laboratory instrumentation.

Statistical values can be used to confidently distinguish modulated from non-modulated genes and noise. Statistical tests find the genes most significantly different between diverse groups of samples. The Student's T-test is an example of a robust statistical test that can be used to find significant differences between two groups. The lower the p-value, the more compelling the evidence that the gene is showing a difference between the different groups. Nevertheless, since microarrays measure more than one gene at a time, tens of thousands of statistical tests may be performed at one time. Because of this, one is unlikely to see small p-values just by chance and adjustments for this using a Sidak correction as well as a randomization/permutation experiment can be made. A p-value less than 0.05 by the T-test is evidence that the gene is significantly different. More compelling evidence is a p-value less then 0.05 after the Sidak correction is factored in. For a large number of samples in each group, a p-value less than 0.05 after the randomization/permutation test is the most compelling evidence of a significant difference.

Another parameter that can be used to select genes that generate a signal that is greater than that of the non-modulated gene or noise is the use of a measurement of absolute signal difference. Preferably, the signal generated by the modulated gene expression is at least 20% different than those of the normal or non-modulated gene (on an absolute basis). It is even more preferred that such genes produce expression patterns that are at least 30% different than those of normal or non-modulated genes.

Genes can be grouped so that information obtained about the set of genes in the group provides a sound basis for making a clinically relevant judgment such as a diagnosis, prognosis, or treatment choice. These sets of genes make up the portfolios of the invention. In this case, the judgments supported by the portfolios involve breast cancer and its chance of recurrence. As with most diagnostic markers, it is often desirable to use the fewest number of markers sufficient to make a correct medical judgment. This prevents a delay in treatment pending further analysis as well inappropriate use of time and resources.

Preferably, portfolios are established such that the combination of genes in the portfolio exhibit improved sensitivity and specificity relative to individual genes or randomly selected combinations of genes. In the context of the instant invention, the sensitivity of the portfolio can be reflected in the fold differences exhibited by a gene's expression in the diseased state relative to the normal state. Specificity can be reflected in statistical measurements of the correlation of the signaling of gene expression with the condition of interest. For example, standard deviation can be a used as such a measurement. In considering a group of genes for inclusion in a portfolio, a small standard deviation in expression measurements correlates with greater specificity. Other measurements of variation such as correlation coefficients can also be used.

One method of establishing gene expression portfolios is through the use of optimization algorithms such as the mean variance algorithm widely used in establishing stock portfolios. This method is described in detail in US patent publication number 20030194734. Essentially, the method calls for the establishment of a set of inputs (stocks in financial applications, expression as measured by intensity here) that will optimize the return (e.g., signal that is generated) one receives for using it while minimizing the variability of the return. Many commercial software programs are available to conduct such operations. "Wagner Associates Mean-Variance Optimization Application," referred to as "Wagner Software" throughout this specification, is preferred. This software uses functions from the "Wagner Associates Mean-Variance Optimization Library" to determine an efficient frontier and optimal portfolios in the Markowitz sense is preferred. Use of this type of software requires that microarray data be transformed so that it can be treated as an input in the way stock return and risk measurements are used when the software is used for its intended financial analysis purposes.

The process of selecting a portfolio can also include the application of heuristic rules. Preferably, such rules are formulated based on biology and an understanding of the technology used to produce clinical results. More preferably, they are applied to output from the optimization method. For example, the mean variance method of portfolio selection can be applied to microarray data for a number of genes differentially expressed in subjects with breast cancer. Output from the method would be an optimized set of genes that could include some genes that are expressed in peripheral blood as well as in diseased tissue. If samples used in the testing method are obtained from peripheral blood and certain genes differentially expressed in instances of breast cancer are differentially expressed in peripheral blood, then a heuristic rule can be applied in which a portfolio is selected from the efficient frontier excluding those that are differentially expressed in peripheral blood. Of course, the rule can be applied prior to the formation of the efficient frontier by, for example, applying the rule during data pre-selection.

Other heuristic rules can be applied that are not necessarily related to the biology in question. For example, one can apply a rule that only a prescribed percentage of the portfolio can be represented by a particular gene or group of genes. Commercially available software such as the Wagner Software readily accommodates these types of heuristics. This can be useful, for example, when factors other than accuracy and precision (e.g., anticipated licensing fees) have an impact on the desirability of including one or more genes.

One method of the invention involves comparing gene expression profiles for various genes (or portfolios) to ascribe prognoses. The gene expression profiles of each of the genes comprising the portfolio are fixed in a medium such as a computer readable medium. This can take a number of forms. For example, a table can be established into which the range of signals (e.g., intensity measurements) indicative of disease is input. Actual patient data can then be compared to the values in the table to determine whether the patient samples are normal or diseased. In a more sophisticated embodiment, patterns of the expression signals (e.g., fluorescent intensity) are recorded digitally or graphically.

The gene expression patterns from the gene portfolios used in conjunction with patient samples are then compared to the expression patterns. Pattern comparison software can then be used to determine whether the patient samples have a pattern indicative of recurrence of the disease. Of course, these comparisons can also be used to determine whether the patient is not likely to experience disease recurrence. The expression profiles of the samples are then compared to the portfolio of a control cell. If the sample expression patterns are consistent with the expression pattern for recurrence of a breast cancer then (in the absence of countervailing medical considerations) the patient is treated as one would treat a relapse patient. If the sample expression patterns are consistent with the expression pattern from the normal/control cell then the patient is diagnosed negative for breast cancer.

The preferred profiles of this disclosure are the 35-gene portfolio made up of the genes of SEQ ID NOs: 1-35, the 60-gene portfolio made up of the genes of SEQ ID NOs: 36-95 which is best used to prognosticate ER-positive patients, and the 16-gene portfolio made up of genes of SEQ ID NOs: 96-111 which is best used to prognosticate ER-negative patients. Most preferably, the portfolio is made up of genes of SEQ ID NOs: 36-111. This most preferred portfolio best segregates breast cancer patients irrespective of ER status at high risk of relapse from those who are not. Once the high-risk patients are identified they can then be treated with adjuvant therapy.

In this invention, the most preferred method for analyzing the gene expression pattern of a patient to determine prognosis of breast cancer is through the use of a Cox's hazard analysis program. Most preferably, the analysis is conducted using S-Plus software (commercially available from Insightful Corporation). Using such methods, a gene expression profile is compared to that of a profile that confidently represents relapse (i.e., expression levels for the combination of genes in the profile is indicative of relapse). The Cox's hazard model with the established threshold is used to compare the similarity of the two profiles (known relapse versus patient) and then determines whether the patient profile exceeds the threshold. If it does, then the patient is classified as one who will relapse and is accorded treatment such as adjuvant therapy. If the patient profile does not exceed the threshold then they are classified as a non-relapsing patient. Other analytical tools can also be used to answer the same question such as, linear discriminate analysis, logistic regression and neural network approaches.

Numerous other well-known methods of pattern recognition are available. The following references provide some examples:

Weighted Voting: Golub et al. (1999).
Support Vector Machines: Su et al. (2001); and Ramaswamy et al. (2001).
K-nearest Neighbors: Ramaswamy (2001).
Correlation Coefficients: van't Veer et al. (2002).

The gene expression profiles of this invention can also be used in conjunction with other non-genetic diagnostic methods useful in cancer diagnosis, prognosis, or treatment monitoring. For example, in some circumstances it is beneficial to combine the diagnostic power of the gene expression based methods described above with data from conventional markers such as serum protein markers (e.g., Cancer Antigen 27.29 ("CA 27.29")). A range of such markers exists including such analytes as CA 27.29. In one such method, blood is periodically taken from a treated patient and then subjected to an enzyme immunoassay for one of the serum markers described above. When the concentration of the marker suggests the return of tumors or failure of therapy, a sample source amenable to gene expression analysis is taken. Where a suspicious mass exists, a fine needle aspirate (FNA) is taken and gene expression profiles of cells taken from the mass are then analyzed as described above. Alternatively, tissue samples may be taken from areas adjacent to the tissue from which a tumor was previously removed. This approach can be particularly useful when other testing produces ambiguous results.

Articles include representations of the gene expression profiles useful for treating, diagnosing, prognosticating, and otherwise assessing diseases. These profile representations are reduced to a medium that can be automatically read by a machine such as computer readable media (magnetic, optical, and the like). The articles can also include instructions for assessing the gene expression profiles in such media. For example, the articles may comprise a CD ROM having computer instructions for comparing gene expression profiles of the portfolios of genes described above. The articles may also have gene expression profiles digitally recorded therein so that they may be compared with gene expression data from patient samples. Alternatively, the profiles can be recorded in different representational format. A graphical recordation is one such format. Clustering algorithms such as those incorporated in Partek Discover™ and Partek Infer™ software from Partek® mentioned above can best assist in the visualization of such data.

Different types of articles of manufacture according to the disclosure are media or formatted assays used to reveal gene expression profiles. These can comprise, for example, microarrays in which sequence complements or probes are affixed to a matrix to which the sequences indicative of the genes of interest combine creating a readable determinant of their presence. Alternatively, articles according to the disclosure can be fashioned into reagent kits for conducting hybridization, amplification, and signal generation indicative of the level of expression of the genes of interest for detecting breast cancer.

Kits made according to the disclosure include formatted assays for determining the gene expression profiles. These can include all or some of the materials needed to conduct the assays such as reagents and instructions.

The invention is further illustrated by the following non-limiting examples.

**Examples:** Genes analyzed according to this invention are typically related to full-length nucleic acid sequences that code for the production of a protein or peptide. One skilled in the art will recognize that identification of full-length sequences is not necessary from an analytical point of view. That is, portions of the sequences or ESTs can be selected according to well-known principles for which probes can be designed to assess gene expression for the corresponding gene.

## Example 1

### Sample Handling and Microarray Work.

Frozen tumor specimens from LNN patients treated during 1980-1995, but untreated with systemic neoadjuvant therapy, were selected from our tumor bank at the Erasmus Medical Center (Rotterdam, Netherlands). All tumor samples were submitted to our reference laboratory from 25 regional hospitals for steroid hormone receptor measurements. The guidelines for primary treatment were similar for all hospitals. Tumors were selected in a manner to avoid bias. On the assumption of a 25-30% in 5 years, and a substantial loss of tumors because of quality control reasons, 436 invasive tumor samples were processed. Patients with a poor, intermediate, and good clinical outcome were included. Samples were rejected based on insufficient tumor content (53), poor RNA quality (77) or poor chip quality (20) leaving 286 samples eligible for further analysis.

The study protocol was approved by institutional medical ethics committee (MEC no. 02.953). Median age of patients at the time of surgery (breast conserving surgery: 219 patients; modified radical mastectomy: 67 patients) was 52 years (range, 26-83 years). Radiotherapy was given to 248 patients (87%) according to our institutional protocol. The proportions of patients who underwent breast conserving therapy and radiotherapy are normal for LNN disease. Patients were included regardless of radiotherapy status, as this study was not aimed to investigate the potential effects of a specific type of surgery or adjuvant radiotherapy. Furthermore, studies have shown that radiotherapy has no clear effect on distant disease recurrence. Early Breast Cancer Trialists (1995). Lymph node negativity was based on pathological examination by regional pathologists. Foekens et al. (1989a).

Prior to inclusion, all 286 tumor samples were confirmed to have sufficient (>70%) tumor and uniform involvement of tumor in H&E stained 5$\mu$m frozen sections. ER (and PgR) levels were measured by ligand binding assay or enzyme immunoassay (EIA) (Foekens et al. (1989b)) or by immunohistochemistry (in 9 tumors). The cutoff values used to classify patients as positive or negative for ER and PR was 10fmol/mg protein or 10% positive tumor cells. Postoperative follow-up involved examination every 3 months during the first 2 years, every 6 months for years 3 to 5, and every 12 months from year 5. The date of diagnosis of metastasis was defined as the date of confirmation of metastasis after symptoms reported by the patient, detection of clinical signs, or at regular follow-up. The median follow-up period of surviving patients (n=198) was 101 months (range, 20-171). Of the 286 patients included, 93 (33%) showed evidence of distant metastasis within 5 years and were counted as failures in the analysis of distant metastasis-free survival (DMFS). Five patients (2%) died without evidence of disease and were censored at last follow-up. Eighty-three patients (29%) died after a previous relapse. Therefore, a total of 88 patients (31 %) were failures in the analysis of overall survival (OS).

## Example 2

### Gene Expression Analysis of data obtained in Example 1

Total RNA was isolated from 20 to 40 cryostat sections of 30 $\mu$m thickness (50-100 mg) with RNAzol B (Campro Scientific, Veenendaal, Netherlands). Biotinylated targets were prepared using published methods (Affymetrix, CA, Lipshutz et al. (1999)) and hybridized to the Affymetrix oligonucleotide microarray U133a GeneChip. Arrays were scanned using standard Affymetrix protocols. Each probe set was treated as a separate gene. Expression values were calculated using Affymetrix GeneChip analysis software MAS 5.0. Chips were rejected if average intensity was <40 or if the background signal >100. To normalize the chip signals, probe sets were scaled to a target intensity of 600, and scale mask files were not selected.

## Example 3

### Statistical Analysis of genes identified in Example 2

Gene expression data was filtered to include genes called "present" in two or more samples. 17,819 genes passed this filter and were used for hierarchical clustering. Before clustering, the expression level of each gene was divided by its median expression level in the patients. This standardization step limited the effect of the magnitude of expression of genes, and grouped together genes with similar patterns of expression in the clustering analysis. To identify patient subgroups, we carried out average linkage hierarchical clustering on both the genes and the samples using GeneSpring 6·0.

To identify genes that discriminate patients who developed distant metastases from those who remained metastasis-free for 5 years, we used two supervised class prediction approaches. In the first approach, 286 patients were randomly assigned to training and testing sets of 80 and 206 patients, respectively. Kaplan-Meier survival curves (Kaplan et al. (1958)) for the two sets were examined to ensure that there was no significant difference and no bias was introduced by the random selection of the training and testing sets. In the second approach, the patients were allocated to one of two subgroups stratified by ER status (Fig. 1).

Each patient subgroup was analyzed separately in order to select markers. The patients in the ER-positive subgroup were randomly allocated into training and testing sets of 80 and 129 patients, respectively. The patients in the ER-negative subgroup were randomly divided into training and testing sets of 35 and 42 patients, respectively. The markers selected from each subgroup training set were combined to form a single signature to predict tumor metastasis for both ER-positive and ER-negative patients in a subsequent independent validation.

The sample size of the training set was determined by a resampling method to ensure its statistical confidence level. Briefly, the number of patients in the training set started at 15 patients and was increased by steps of 5. For a given sample size, 10 training sets with randomly selected patients were made. A gene signature was constructed from each of training sets and then tested in a designated testing set of patients by analysis of receiver operating characteristic (ROC) curve with distant metastasis within 5 years as the defining point. The mean and the coefficient of variation (CV) of the area under the curve (AUC) for a given sample size were calculated. A minimum number of patients required for the training set were chosen at the point that the average AUC reached a plateau and the CV of the 10 AUC was below 5%.

Genes were selected as follows. First, univariate Cox's proportional hazards regression was used to identify genes for which expression (on $\log_2$ scale) was correlated with the length of DMFS. To reduce the effect of multiple testing and to test the robustness of the selected genes, the Cox's model was constructed with bootstrapping of the patients in the training set. Efron et al. (1981). Briefly, 400 bootstrap samples of the training set were constructed, each with 80 patients randomly chosen with replacement. A Cox's model was run on each of the bootstrap samples. A bootstrap score was created for each gene by removing the top and bottom 5% p-values and then averaging the inverses of the remaining bootstrap p-values. This score was used to rank the genes. To construct a multiple gene signature, combinations of gene markers were tested by adding one gene at a time according to the rank order. ROC analysis using distant metastasis within 5 years as the defining point was performed to calculate the area under AUC for each signature with increasing number of genes until a maximum AUC value was reached.

The Relapse Score (RS) was used to calculate each patient's risk of distant metastasis. The score was defined as the linear combination of weighted expression signals with the standardized Cox's regression coefficient as the weight.

$$\text{Relapse Score} = A \cdot I + \sum_{i=1}^{60} I \cdot w_i x_i + B \cdot (1-I) + \sum_{j=1}^{16} (1-I) \cdot w_j x_j$$

where

$$I = \begin{cases} 1 & \text{if ER level} > 10 \text{ fmol per mg protein} \\ 0 & \text{if ER level} \leq 10 \text{ fmol per mg protein} \end{cases}$$

A and B are constants
$w_i$ is the standardized Cox's regression coefficient for ER + marker
$x_i$ is the expression value of ER + marker on a log2 scale
$w_j$ is the standardized Cox's regression coefficient for ER - marker
$x_j$ is the expression value of ER - marker on a log2 scale

The threshold was determined from the ROC curve of the training set to ensure 100% sensitivity and the highest specificity. The values of constants A of 313.5 and B of 280 were chosen to center the threshold of RS to zero for both ER-positive and ER-negative patients. Patients with positive RS scores were classified into the poor prognosis group and patients with negative RS scores were classified into the good prognosis group. The gene signature and the cutoff were validated in the testing set. Kaplan-Meier survival plots and log-rank tests were used to assess the differences in time to distant metastasis of the predicted high and low risk groups. Odds ratios (OR) were calculated as the ratio of the odds of distant metastasis between the patients predicted to relapse and those predicted to remain relapse-free.

Univariate and multivariable analyses with Cox's proportional hazards regression were done on the individual clinical variables with and without the gene signature. The HR and its 95% confidence interval (CI) were derived from these results. All statistical analyses were performed using S-Plus 6.1 software (Insightful, VA).

## Example 4

### Pathway Analysis of genes identified in Example 3

A functional class was assigned to each of the genes in the prognostic signature gene. Pathway analysis was done with Ingenuity 1.0 software (Ingenuity Systems, CA). Affymetrix probes were used as input to search for biological networks built by the software. Biological networks identified by the program were assessed in the context of general functional classes by GO ontology classification. Pathways with two or more genes in the prognostic signature were selected and evaluated.

## Example 5

### Results for Examples 1-4

Patient and Tumor Characteristics

Clinical and pathological features of the 286 patients are summarized in Table 1.

Table 1. Clinical and Pathological Characteristics of Patients and Their Tumors

| Characteristics | All patients (%) | ER-positive training set (%) | ER-negative training set (%) | Validation set (%) |
|---|---|---|---|---|
| Number | 286 | 80 | 35 | 171 |
| Age (mean±SD) | 54±12 | 54±13 | 54±13 | 54±12 |
| ≤40 yr | 36 (13) | 12 (15) | 3 (9) | 21 (12) |
| 41-55 yr | 129 (45) | 30 (38) | 17 (49) | 82 (48) |
| 56-70 yr | 89 (31) | 28 (35) | 11 (31) | 50 (29) |
| >70 yr | 32 (11) | 10 (13) | 4 (11) | 18 (11) |
| Menopausal status | | | | |
| Premenopausal | 139(49) | 39 (49) | 16 (46) | 84 (49) |
| Postmenopausal | 147 (51) | 41 (51) | 19 (54) | 87 (51) |
| T stage | | | | |
| T1 | 146 (51) | 38 (48) | 14 (40) | 94 (55) |
| T2 | 132 (46) | 41 (51) | 19 (54) | 72 (42) |
| T3/4 | 8 (3) | 1 (1) | 2 (6) | 5 (3) |
| Grade | | | | |
| Poor | 148 (52) | 37 (46) | 24 (69) | 87 (51) |
| Moderate | 42 (15) | 12 (15) | 3 (9) | 27 (16) |
| Good | 7 (2) | 2 (3) | 2 (6) | 3 (2) |
| Unknown | 89 (31) | 29 (36) | 6 (17) | 54 (32) |
| ER* | | | | |
| Positive | 209 (73) | 80 (100) | 0 (0) | 129 (75) |
| Negative | 77 (27) | 0 (0) | 35 (100) | 42 (25) |
| PgR* | | | | |
| Positive | 165 (58) | 59 (74) | 5 (14) | 101 (59) |

(continued)

| Characteristics | All patients (%) | ER-positive training set (%) | ER-negative training set (%) | Validation set (%) |
|---|---|---|---|---|
| Negative | 111 (39) | 19 (24) | 29 (83) | 63 (37) |
| Unknown | 10 (3) | 2 (2) | 1 (3) | 7 (4) |
| Metastasis <5 years | | | | |
| Yes | 93 (33) | 24 (30) | 13 (37) | 56 (33) |
| No | 183 (64) | 51 (64) | 17 (49) | 115 (67) |
| Censored if <5 yr | 10 (3) | 5 (6) | 5 (14) | 0 (0) |

*ER-positive and PgR positive: >10 fmol/mg protein or >10% positive tumor cells.

There were no differences in age or menopausal status. The ER-negative training group had a slightly higher proportion of larger tumors and, as expected, more poor grade tumors than the ER-positive training group. The validation group of 171 patients (129 ER-positive, 42 ER-negative) did not differ from the total group of 286 patients with respect to any of the patients or tumor characteristics.

Two approaches were used to identify markers predictive of disease relapse. First, we randomly divided all the 286 patients (ER-positive and ER-negative combined) into a training set and a testing set. Thirty-five genes were selected from 80 patients in the training set and a Cox's model to predict the occurrence of distant metastasis was built. A moderate prognostic value was observed. Table 2. Unsupervised clustering analysis showed two distinct subgroups highly correlated with the tumor ER status (chi square test p <0.0001). Figure 1B, which supported the second approach in which patients were first placed into subgroups based on ER status.

Table 2

| SEQ ID NO: | Cox's coefficient | p-value |
|---|---|---|
| 1 | 4.008 | 0.00006 |
| 2 | -3.649 | 0.00026 |
| 3 | 4.005 | 0.00006 |
| 4 | -3.885 | 0.00010 |
| 5 | -3.508 | 0.00045 |
| 6 | -3.176 | 0.00150 |
| 7 | 3.781 | 0.00016 |
| 8 | 3.727 | 0.00019 |
| 9 | -3.570 | 0.00036 |
| 10 | -3.477 | 0.00051 |
| 11 | 3.555 | 0.00038 |
| 12 | -3.238 | 0.00120 |
| 13 | -3.238 | 0.00120 |
| 14 | 3.405 | 0.00066 |
| 15 | 3.590 | 0.00033 |
| 16 | -3.157 | 0.00160 |
| 17 | -3.622 | 0.00029 |
| 18 | -3.698 | 0.00022 |
| 19 | 3.323 | 0.00089 |
| 20 | -3.556 | 0.00038 |
| 21 | -3.317 | 0.00091 |
| 22 | -2.903 | 0.00370 |
| 23 | -3.338 | 0.00085 |
| 24 | -3.339 | 0.00084 |
| 25 | -3.355 | 0.00079 |
| 26 | 3.713 | 0.00021 |
| 27 | -3.325 | 0.00088 |
| 28 | -2.984 | 0.00284 |
| 29 | 3.527 | 0.00042 |

(continued)

| SEQ ID NO: | Cox's coefficient | p-value |
|---|---|---|
| 30 | -3.249 | 0.00116 |
| 31 | -2.912 | 0.00360 |
| 32 | 3.118 | 0.00182 |
| 33 | 3.435 | 0.00059 |
| 34 | -2.971 | 0.00297 |
| 35 | 3.282 | 0.00103 |

Each subgroup was analyzed in order to select markers. Seventy-six genes were selected from patients in the training sets (60 for the ER-positive group, 16 for the ER-negative group) (Fig. 2A, *left*). With the selected genes and ER status taken together, a Cox's model to predict recurrence of cancer was built for all LNN patients. Validation of the 76-gene predictor in the 171 patient testing set produced an ROC with an AUC value of 0.694, sensitivity of 93% (52/56), and specificity of 48% (55/115) (Fig. 2A, right). Patients with a relapse score above the threshold of the prognostic signature have an 11-9-fold OR (95% CI: 4.04-35.1; p<0.0001) to develop distant metastasis within 5 years. As a control, randomly selected 76-gene sets were generated. These produced ROC with an average AUC value of 0.515, sensitivity of 91%, and specificity of 12% in the testing group. Patients stratified by such a gene set would have an odds ratio of 1.3 (0.50-3.90; p=0.8) for development of metastases, indicating a random classification. In addition, the Kaplan-Meier analyses for distant metastasis free survival (DMFS) and overall survival (OS) as a function of the 76-gene signature showed highly significant differences in time to metastasis between the groups predicted to have good and poor prognosis (Fig. 2). At 60 and 80 months, the respective absolute differences in DMFS between the groups with predicted good and poor prognosis were 40% (93% vs. 53%) and 39% (88% vs. 49%) and those in OS were 27% (97% vs. 70%) and 32% (95% vs. 63%), respectively.

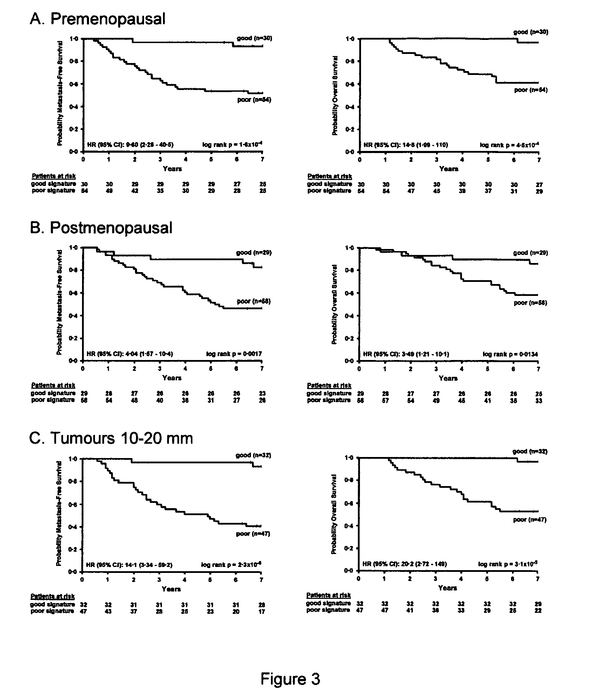

The 76-gene profile also represented a strong prognostic factor for the development of distant metastasis in the subgroups of 84 premenopausal patients (HR: 9.60), 87 postmenopausal patients (HR: 4.04) and 79 patients with tumor sizes of 10 to 20 mm (HR: 14.1) (Fig. 3).

Univariate and multivariable Cox's regression analyses are summarized in Table 3.

Table 3: Uni- and multivariable analyses for DMFS in the testing set of 171 LNN patients

| | Univariate analysis | | | Multivariable analysis* | | |
|---|---|---|---|---|---|---|
| | HR† | (95% CI) † | p-value | HR† | (95% CI) † | p-value |
| Age ‡ | | | | | | |
| Age2 vs. Age 1 | 1.16 | (0.51 - 2.65) | 0.7180 | 1.14 | (0.45 - 2.91) | 0.7809 |
| Age3 vs. Age 1 | 1.32 | (0.56 - 3.10) | 0.5280 | 0.87 | (0.26 - 2.93) | 0.8232 |
| Age4 vs. Age 1 | 0.95 | (0.32 - 2.82) | 0.9225 | 0.61 | (0.15 - 2.60) | 0.5072 |
| Menopausal status§ | 1.24 | (0.76 - 2.03) | 0.3909 | 1.53 | (0.68 - 3.44) | 0.3056 |
| Stage ‖ | 1.08 | (0.66 - 1.77) | 0.7619 | 2.57 | (0.23 - 29.4) | 0.4468 |
| Differentiation¶ | 0.38 | (0.16 - 0.90) | 0.0281 | 0.60 | (0.24 - 1.46) | 0.2590 |
| Tumor size** | 1.06 | (0.65 - 1.74) | 0.8158 | 0.34 | (0.03 - 3.90) | 0.3849 |
| ER†† | 1.09 | (0.61-1.98) | 0.7649 | 1.05 | (0.54 - 2.04) | 0.8935 |
| PR†† | 0.83 | (0.51 - 1.38) | 0.4777 | 0.85 | (0.47 - 1.53) | 0.5882 |
| 76-gene signature | 5.67 | (2.59 - 12.4) | $1.5 \times 10^{-5}$ | 5.55 | (2.46 - 12.5) | $3.6 \times 10^{-5}$ |

*The multivariable model included 162 patients, due to missing values in 9 patients
†Hazard ratio and 95% confidence interval
‡Age1 is ≤40 yr, Age2 is 41 to 55 yr, Age3 is 56 to 70 yr, Age4 is >70 yr
§Post-menopausal vs. pre-menopausal
‖ Stage: II & III vs. I
¶Grade: moderate/good vs. poor, unknown grade was included as a separate group
**Tumor size: >20 mm vs. ≤20 mm
††Positive vs. negative

Other than the 76-gene signature, only grade was significant in univariate analysis and moderate/good differentiation was associated with favorable DMFS. Multivariable regression estimation of HR for the occurrence of tumor metastasis within 5 years was 5.55 (p<0.0001), indicating that the 76-gene set represents an independent prognostic signature strongly associated with a higher risk of tumor metastasis. Univariate and multivariable analyses were also done separately for ER-positive and ER-negative patients the 76-gene signature was also an independent prognostic variable in the subgroups stratified by ER status.

The function of the 76 genes (Table 4) in the prognostic signature was analyzed to relate the genes to biological pathways.

Table 4

| ER Status | SEQ ID NO. | Std. Cox's coefficient | Cox's p-value |
|---|---|---|---|
| + | 36 | -3.83 | 0.00005 |
| + | 37 | -3.865 | 0.00001 |
| + | 38 | 3.63 | 0.00002 |
| + | 39 | -3.471 | 0.00016 |
| + | 40 | 3.506 | 0.00008 |
| + | 41 | -3.476 | 0.00001 |
| + | 42 | 3.392 | 0.00006 |
| + | 43 | -3.353 | 0.00080 |
| + | 44 | -3.301 | 0.00038 |
| + | 45 | 3.101 | 0.00033 |
| + | 46 | -3.174 | 0.00128 |
| + | 47 | 3.083 | 0.00020 |
| + | 48 | 3.336 | 0.00005 |
| + | 49 | -3.054 | 0.00063 |
| + | 50 | -3.025 | 0.00332 |
| + | 51 | 3.095 | 0.00044 |
| + | 52 | -3.175 | 0.00031 |
| + | 53 | -3.082 | 0.00086 |
| + | 54 | 3.058 | 0.00016 |
| + | 55 | 3.085 | 0.00009 |
| + | 56 | -2.992 | 0.00040 |
| + | 57 | -2.791 | 0.00020 |
| + | 58 | -2.948 | 0.00039 |
| + | 59 | 2.931 | 0.00020 |
| + | 60 | -2.896 | 0.00052 |
| + | 61 | 2.924 | 0.00050 |
| + | 62 | 2.915 | 0.00055 |
| + | 63 | -2.968 | 0.00099 |
| + | 64 | 2.824 | 0.00086 |
| + | 65 | -2.777 | 0.00398 |
| + | 66 | -2.635 | 0.00160 |
| + | 67 | -2.854 | 0.00053 |
| + | 68 | 2.842 | 0.00051 |
| + | 69 | -2.835 | 0.00033 |
| + | 70 | 2.777 | 0.00164 |
| + | 71 | -2.759 | 0.00222 |
| + | 72 | -2.745 | 0.00086 |
| + | 73 | 2.79 | 0.00049 |
| + | 74 | 2.883 | 0.00031 |
| + | 75 | -2.794 | 0.00139 |
| + | 76 | -2.743 | 0.00088 |
| + | 77 | -2.761 | 0.00164 |
| + | 78 | -2.831 | 0.00535 |
| + | 79 | 2.659 | 0.00073 |

(continued)

| ER Status | SEQ ID NO. | Std. Cox's coefficient | Cox's p-value |
|:---:|:---:|:---:|:---:|
| + | 80 | -2.715 | 0.00376 |
| + | 81 | 2.836 | 0.00029 |
| + | 82 | -2.687 | 0.00438 |
| + | 83 | -2.631 | 0.00226 |
| + | 84 | -2.716 | 0.00089 |
| + | 85 | 2.703 | 0.00232 |
| + | 86 | -2.641 | 0.00537 |
| + | 87 | -2.686 | 0.00479 |
| + | 88 | -2.654 | 0.00363 |
| + | 89 | 2.695 | 0.00095 |
| + | 90 | -2.758 | 0.00222 |
| + | 91 | 2.702 | 0.00084 |
| + | 92 | -2.694 | 0.00518 |
| + | 93 | 2.711 | 0.00049 |
| + | 94 | -2.771 | 0.00156 |
| + | 95 | 2.604 | 0.00285 |
| - | 96 | -3.495 | 0.00011 |
| - | 97 | 3.224 | 0.00036 |
| - | 98 | -3.225 | 0.00041 |
| - | 99 | -3.145 | 0.00057 |
| - | 100 | -3.055 | 0.00075 |
| - | 101 | -3.037 | 0.00091 |
| - | 102 | -3.066 | 0.00072 |
| - | 103 | 3.06 | 0.00077 |
| - | 104 | -2.985 | 0.00081 |
| - | 105 | -2.983 | 0.00104 |
| - | 106 | -3.022 | 0.00095 |
| - | 107 | -3.054 | 0.00082 |
| - | 108 | -3.006 | 0.00098 |
| - | 109 | -2.917 | 0.00134 |
| - | 110 | -2.924 | 0.00149 |
| - | 111 | -2.882 | 0.0017 |

Although 18 of the 76 genes have unknown function, several pathways or biochemical activities were identified that were well represented such as cell death, cell cycle and proliferation, DNA replication and repair and immune response (Table 5).

Table 5. Pathway analysis of the 76 genes from the prognostic signature

| Functional Class | 76-gene signature |
|---|---|
| Cell death | TNFSF10, TNFSF13, MAP4, CD44, IL18, GAS2, NEFL, EEF1A2, BCLG, C3 |
| Cell cycle | CCNE2, CD44, MAP4, SMC4L1, TNFSF10, AP2A2, FEN1, KPNA2, ORC3L, PLK1 |
| Proliferation | CD44, IL18, TNFSF10, TNFSF13, PPP1CC, CAPN2, PLK1, SAT |
| DNA replication, recombination / repair | TNFSF10, SMC4L1, FEN1, ORC3L, KPNA2, SUPT16H, POLQ, ADPRTL1 |
| Immune response | TNFSF10, CD44, IL18, TNFSF13, ARHGDIB, C3 |
| Growth | PPP1CC, CD44, IL18, TNFSF10, SAT, HDGFRP3 |
| Cellular assembly and organization | MAP4, NEFL, TNFSF10, PLK1, AP2A2, SMC4L1 |
| Transcription | KPNA2, DUSP4, SUPT16H, DKFZP434E2220, PHF11, ETV2 |

(continued)

| Functional Class | 76-gene signature |
|---|---|
| Cell-to-cell signaling and interaction | CD44, IL18, TNFSF10, TNFSF13, C3 |
| Survival | TNFSF10, TNFSF13, CD44, NEFL |
| Development | IL18, TNFSF10, COL2A1 |
| Cell morphology | CAPN2, CD44, TACC2 |
| Protein synthesis | IL18, TNFSF10, EEF1A2 |
| ATP binding | PRO2000, URKL1, ACACB |
| DNA binding | HIST1H4H, DKFZP434E2220, PHF11 |
| Colony formation | CD44, TNFSF10 |
| Adhesion | CD44, TMEM8 |
| Neurogenesis | CLN8, NEURL |
| Golgi apparatus | GOLPH2, BICD1 |
| Kinase activity | CNK1, URKL1 |
| Transferase activity | FUT3, ADPRTL1 |

Genes implicated in disease progression were found including calpain2, origin recognition protein, dual specificity phosphatases, Rho-GDP dissociation inhibitor, TNF superfamily protein, complement component 3, microtubule-associated protein, protein phosphatase 1 and apoptosis regulator BCL-G. Furthermore, previously characterized prognostic genes such as cyclin E2 (Keyomarsi et al. (2002)) and CD44 (Herrera-Gayol et al. (1999)) were in the gene signature.

**Example 6**

**Discussion for Examples 1-5**

We provide results of an analysis of primary tumors from 286 patients with lymph-node negative breast cancer of all age-groups and tumor sizes. The patients had not received adjuvant systemic therapy, so the multigene assessment of prognosis was not subject to potentially confounding contributions by predictive factors related to systemic treatment.

This study revealed a 76-gene signature that accurately predicts distant tumor recurrence. This signature is applicable to all LNN breast cancer patients independently of age, tumor size and grade and ER status. In Cox's multivariable analysis for DMFS the 76-gene signature was the only significant variable, superseding the clinical variables, including grade. After 5 years, absolute differences in DMFS and OS between the patients with the good and poor 76-gene signatures were 40% and 27%, respectively. Of the patients with a good prognosis signatures, 7% developed distant metastases and 3% died within 5 years. If further validated, this prognostic signature will yield a positive predictive value of 37% and a negative predictive value of 95%, on the assumption of a 25% rate of disease recurrence in LNN patients. In particular, this signature can be valuable for defining the risk of recurrence for the increasing proportion of T1 tumors (<2 cm). Comparison with the St Gallen and NIH guidelines was instructive. Although ensuring the same number of the high-risk patients would receive the necessary treatment, our 76-gene signature would recommend systemic adjuvant chemotherapy to only 52% of the low-risk patients, as compared to 90% and 89% by the St. Gallen and NIH guidelines, respectively (Table 6).

Table 6. Comparison of the 76-gene signature and the current conventional consensus on treatment of breast cancer

| Method | Patients guided to receive adjuvant chemotherapy in the testing set | |
|---|---|---|
| | Metastatic disease at 5 years (%) | Metastatic disease free at 5 years (%) |
| St Gallen | 52/55 (95) | 104/115 (90) |
| NIH | 52/55 (95) | 101/114 (89) |
| 76-gene signature | 52/56 (93) | 60/115 (52) |
| The conventional consensus criteria. St. Gallen: tumor ≥ 2cm, ER-negative, grade 2-3, patient <35 yr (either one of these criteria); NIH: tumor >1 cm. | | |

The 76-gene signature can thus result in a reduction of the number of low-risk LNN patients who would be recommended to have unnecessary adjuvant systemic therapy.

The 76-genes in the prognostic signature belong to many functional classes, suggesting that different paths could lead to disease progression. The signature included well-characterized genes and 18 unknown genes. This finding could explain the superior performance of this signature as compared to other prognostic factors. Although genes involved in cell death, cell proliferation, and transcriptional regulation were found in both patient groups stratified by ER status, the 60 genes selected for the ER-positive group and the 16 genes selected for the ER-negative group had no overlap. This result supports the idea that the extent of heterogeneity and the underlying mechanisms for disease progression could differ for the two ER-based subgroups of breast cancer patients.

Comparison of our results with those of the study by van de Vijver et al. (2002) is difficult because of differences in patients, techniques and materials used. van de Vijver et al. included both node-negative and node-positive patients, who had or had not received adjuvant systemic therapy, and only women younger than 53 years. Furthermore, the microarray platforms used in the studies are different, Affymetrix vs. Agilent. Of the 70 genes of the van't Veer (2002) study, only 48 are present on the Affymetrix U133a array, while of our 76 genes only 38 are present on the Agilent array. There is a 3-gene overlap between the two signatures (cyclin E2, origin recognition complex, and TNF superfamily protein). Despite the apparent difference, both signatures included genes that identified several common pathways that might be involved in tumor recurrence. This finding supports the idea that while there might be redundancy in gene members, effective signatures could be required to include representation of specific pathways.

The strengths of our study compared with the study of van de Vijver et al. (2002) are the larger number of untreated LNN patients (286 vs. 141), and the independence of our 76-gene signature with respect to age, menopausal status, and tumor size. The validation set of our patients is completely without overlap with the training set in contrast to 90% of other reports. Ransohoff (2004). In conclusion, as only approximately 30-40% of the untreated LNN patients develop tumor recurrence, the prognostic signature could provide a powerful tool to identify those patients at low risk preventing over treatment in substantial numbers of patients. The recommendation of adjuvant systemic therapy in patients with primary breast cancer could be guided in the future by this prognostic signature. The predictive value of our gene signature with respect to the efficacy of different modes of systemic therapy could be tested in the adjuvant setting or in patients with metastatic disease.

**Example 7**

**Comparison of Breast Tumor Gene Profile Generated From Laser Capture Microdissection and Bulk Tissue In Stage I/II Breast Cancer**

Gene-expression profiling has been shown to be a powerful diagnostic and prognostic tool for a variety of cancer types. Almost exclusively in all cases bulk tumor RNA was used for hybridization on the chip. Estrogens play important roles in the development and growth of hormone-dependent tumors.

About 75% of breast cancers express estrogen receptor (ER), which is an indicator for (adjuvant) tamoxifen treatment and is associated with patient outcomes.

To gain insights into the mechanisms trigged by estrogen in breast epithelia cells and their association with tumorigenesis, laser capture microdissection (LCM) was used to procure histologically homogenous population of tumor cells from 29 early stage primary breast tumors, in combination with GeneChip expression analysis. Of these 29 patients, 11 were ER-negative and 17 were ER-positive based on quantitative ligand binding or enzyme immunoassays on tumor cytosols. For comparison, gene expression profiling was also obtained using bulk tissue RNA isolated from the same group of 29 patients.

Fresh frozen tissue samples were collected from 29 lymph-node-negative breast cancer patients (for tumor characteristics, Table 2) who had been surgically treated for a breast tumor and had not received neoadjuvant systemic therapy. For each patient tissue sample, we first used the H&E slide to evaluate the cell morphology. RNA was isolated from both tumor cells obtained by LCM (PALM) performed on cryostat sections and from whole cryostat sections, i.e., bulk tissue of the same tumor. RNA sample quality was analyzed by an Agilent BioAnalyzer. The RNA samples were hybridized to Affymetrix human U133A chip that contains approximately 22,000 probe sets. The fluorescence was quantified and the intensities were normalized. Clustering Analysis and Principal Component Analysis were used to group patients with similar gene expression profiles. Genes that are differentially expressed between ER-positive and ER-negative samples were selected.

Total RNA isolated from LCM procured breast cancer cells was subjected to two-round T7 based amplification in target preparation, versus one round amplification with bulk tissue RNA. Expression levels of 21 control genes (Table 7) were compared between LCM data set and bulk tissue set to demonstrate the fidelity of linear amplification.

Table 7: Control gene list

| SEQ ID NO: | Name |
|---|---|
| 112 | protein phosphatase 2, regulatory subunit B (B56), delta isoform |
| 113 | CCCTC-binding factor (zinc finger protein) |
| 114 | solute carrier family 4 (anion exchanger), member 1, adaptor protein |
| 115 | ribonuclease P |
| 116 | hypothetical protein FLJ20188 |
| 117 | KIAA0323 protein |
| 118 | cDNA FLJ12469 |
| 119 | translation initiation factor eIF-2b delta subunit |
| 120 | heterogeneous nuclear ribonucleoprotein K |
| 121 | hydroxymethylbilane synthase |
| 122 | cDNA DKFZp58600222 |
| 123 | chromosome 20 open reading frame 4 |
| 124 | thyroid hormone receptor interactor 4 |
| 125 | hypoxanthine phosphoribosyltransferase 1 (Lesch-Nyhan syndrome) |
| 126 | DnaJ (Hsp40) homolog, subfamily C, member 8 |
| 127 | dual specificity phosphatase 11 (RNA/RNP complex 1-interacting) |
| 128 | calcium binding atopy-related autoantigen 1 |
| 129 | stromal cell-derived factor 2 |
| 130 | Ewing sarcoma breakpoint region 1 |
| 131 | CCR4-NOT transcription complex, subunit 2 |
| 132 | F-box only protein 7 |

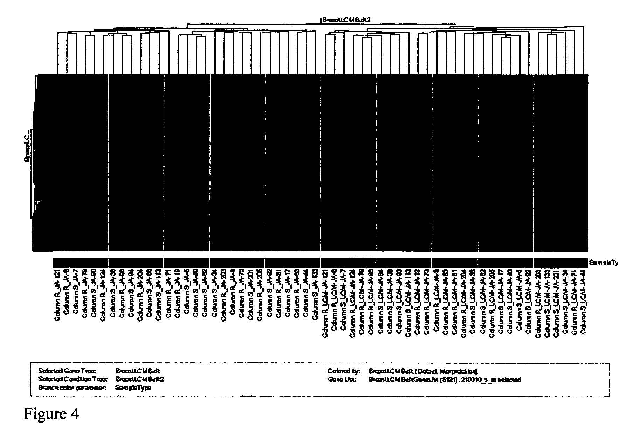

The results obtained are depicted in Table 8 and Figures 4-9.

Table 8 Clinical characteristics of patients

| Characteristic | | No. of patients (%) |
|---|---|---|
| Age in years | | |
| | < 40 | 1 (3) |
| | 40-44 | 5 (17) |
| | 45-49 | 8 (28) |
| | ≥ 50 | 15 (52) |
| Tumor diameter in mm | | |
| | ≤ 20 | 11 (40) |
| | > 20 | 17(59) |
| Histologic grade | | |
| | II (intermediate) | 5 (17) |
| | III (poor) | 12 (41) |
| Estrogen-receptor status | | |
| | Negative | 11 (40) |

(continued)

| Characteristic | | No. of patients (%) |
|---|---|---|
| | Positive | 17 (59) |
| Surgery | | |
| | Breast-conserving therapy | 26 (90) |
| | Mastectomy | 3 (10) |
| Chemotherapy | | |
| | No | 29 (100) |
| Hormonal therapy | | |
| | No | 29 (100) |
| Disease-free survival in months | | |
| | ≤48 | 13 (45) |
| | >48 | 16 (55) |

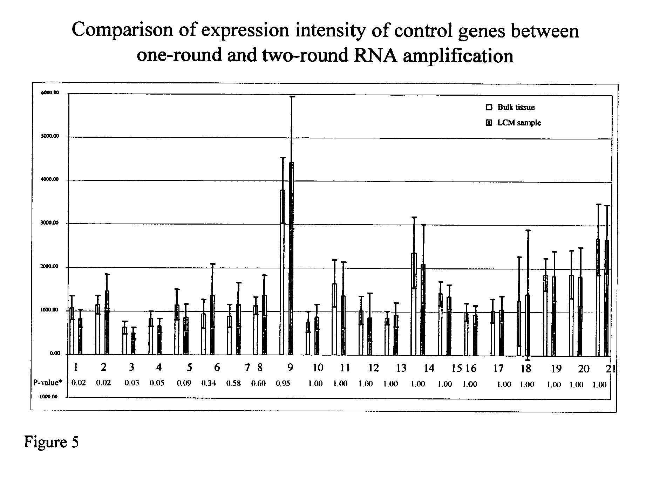

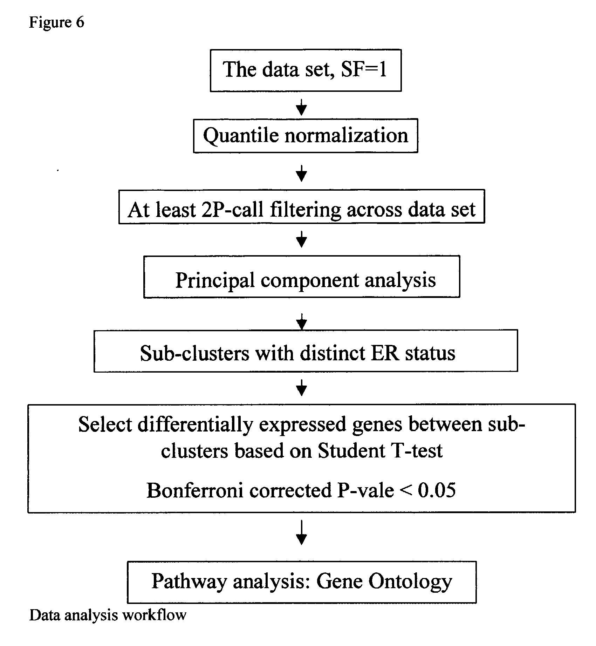

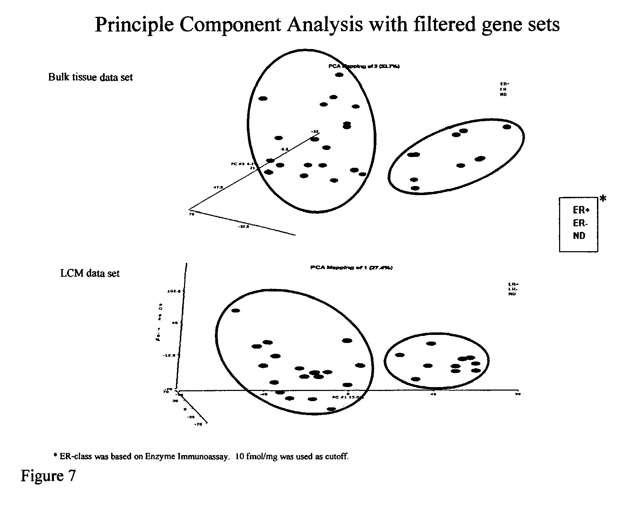

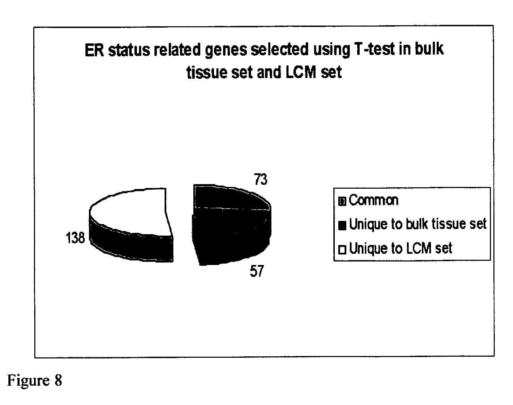

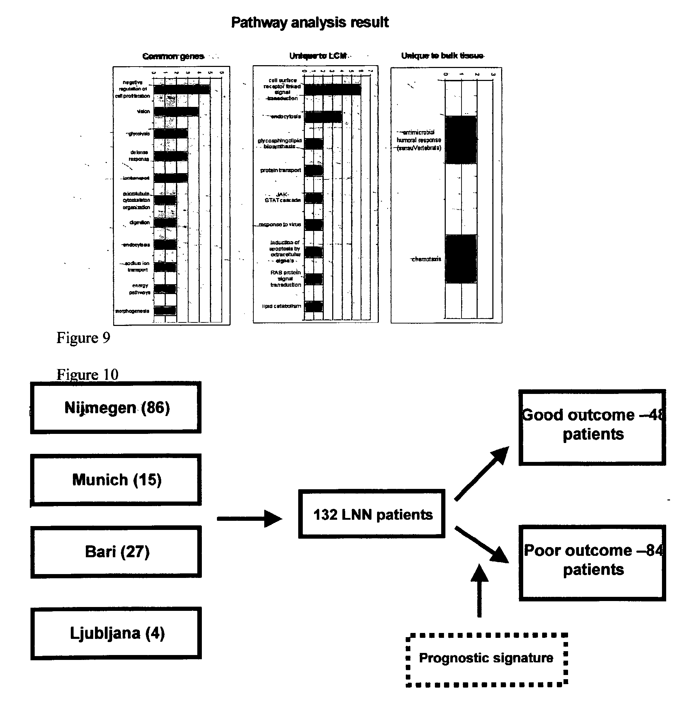

Figure 4 is a hierarchical clustering based on 5121 genes and shows that LCM and bulk tissue samples are completely separated based on global RNA expression profiles. Figure 5 is a bar graph depicting the expression levels of 21 control genes (Table 6) in RNA isolates from LCM samples and bulk tissues and shows that an additional round of linear amplification used for RNA obtained by LCM did not cause differential expression of the control genes. Figure 6 is a data analysis pathway and Figure 7 shows the PCA analysis with filtered gene sets. Figure 8 is a pie chart where ER status was used to assign patient subgroups. Differentially expressed genes between ER-positive and ER-negative sub-clusters in both LCM and bulk tissue samples were defined by Student T-test. Figure 9 is a series of bar graphs showing the results of pathway analysis by Gene Ontology for genes exclusively associated with ER in LCM samples, exclusively in bulk tissues, and for those that are common in both LCM and bulk tissue.

In summary, the results obtained show several important conclusions. First, genes related to cell proliferation and energy metabolism were seen differentially expressed in ER-/ER+ patients both in bulk tissue data set and LCM data set. Second, due to the enrichment of breast cancer cells via LCM, genes involved in cell surface receptor linked signal transduction, RAS signal transduction, JAK-STAT signal transduction and apoptosis were found associate to ER status. These genes were not identified in bulk data set. Third, microdissection provides a sensitive approach to studying epithelial tumor cells and an insight into signaling pathway associated with estrogen receptors. Therefore, it is clear that the application of the gene expression profile described herein to LCM isolated tumor cells is commensurate with results obtained in heterogeneous bulk tissue.

**Example 8**

**Validation and pathway analysis of the 76-gene prognostic signature in breast cancer**

This Example reports the results of a validation study in which the 76-gene signature was used to predict outcomes of 132 patients obtained from 4 independent sources.

In addition, in order to evaluate the robustness of this gene signature, this Example further provides identification of substitutable components of the signature and describes how the substitutions lead to the identification of key pathways in an effective signature.

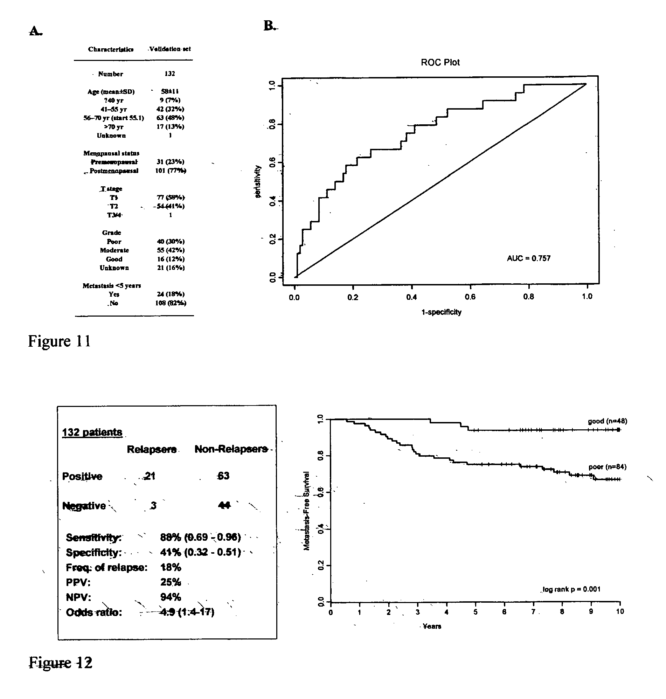

Fresh frozen tissue samples were collected from 132 patients who had been surgically treated for a breast tumor and had not received adjuvant systemic therapy. The patient samples used were collected between 1980 and 1996 (Fig. 10). For each patient tissue sample, an H&E slide was used to evaluate the cell morphology. Then total RNA samples were prepared and the sample quality was analyzed by Agilent BioAnalyzer. The RNA samples were analyzed by microarray analysis. The fluorescence was quantified and the intensities were normalized. A relapse hazard score was calculated for each patient based on the expression levels of the 76-gene signature. The patients were classified into good and poor outcome groups. Figures 11 and 12.

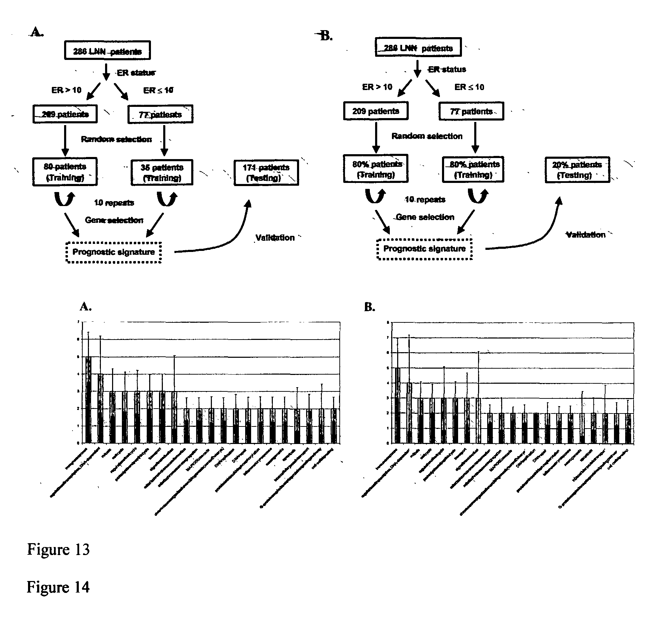

In order to evaluate the robustness of this gene signature, two statistical analyses were designed and used. Figure 13. First, gene selection and signature construction procedures that were used to discover the 76-gene signature were repeated. As shown in Table 8, ten training sets of 115 patients each were randomly selected from the total of 286 patients. The remaining patients were served as the testing set.

Second, the number of patients in a training set was increased to 80% of the 286 patients and used the remaining 20% of the patients as the testing set. This selection procedure was also repeated 10 times. In both procedures, Kaplan-Meier survival curves were used to ensure no significant difference in disease free survival between the training and the testing pair. Genes were selected and a signature was built from each of the training sets using Cox's proportional-hazards regression. Each signature was validated in the corresponding testing set. Furthermore, the 76-gene prognostic signature was assigned into functional groups using GO ontology classification. Pathways that cover significant numbers of genes in the signature were selected (p-value <0.05 and >2 hits). The selected pathways were also evaluated in all the prognostic signatures derived from different training sets.

In Table 9, A. contains the results from 10 signatures using training sets of 115 patients and B. contains the results from 10 signatures using training sets of 80% of the patients.

| A | | B | |
|---|---|---|---|
| AUC of ROC | 0.62 (0.55-0.70) | AUC of ROC | 0.62 (0.53-0.72) |
| Sensitivity | 86% (0.84 - 0.88) | Sensitivity | 83% (0.81 - 0.85) |
| Specificity | 34% (0.21 - 0.56) | Specificity | 46% (0.28 - 0.62) |
| Freq. of relapse | 33% | Freq. of relapse | 33% |
| PPV | 40% (0.35 - 0.49) | PPV | 47% (0.32 - 0.58) |
| NPV | 81% (0.75 - 0.89) | NPV | 82% (0.78 - 0.89) |
| Odds Ratio | 3.5 (1.7-7.9) | Odds Ratio | 5.6 (1.7-15) |

The results obtained in this Example show that:

- The 76-gene signature is successfully validated in 132 independent patients, giving an AUC value of 0.757 in the 132 LNN breast cancer patients from 4 independent sources. The signature shows 88% sensitivity and 41% specificity.
- The average AUC for the substitute signatures is 0.64 (95% CI: 0.53 -0.72). This result is consistent with that of the 76-gene predictor (AUC of 0.69). Twenty-one pathways over-represented in the 76-gene signature were also found in all the other prognostic signatures, suggesting that common biological pathways are involved in tumor recurrence.
- These results suggest that gene expression profiles provide a powerful approach to perform risk assessment of patient outcome. The data highlight the feasibility of a molecular prognostic assay that provides patients with a quantitative measurement of tumor relapse.

Although the foregoing invention has been described in some detail by way of illustration and example for purposes of clarity of understanding, the descriptions and examples should not be construed as limiting the scope of the invention.

Table 10

| Sequence identification | | | | |
|---|---|---|---|---|
| SEQ ID NO: | psid | Gene Name | Accession # | Gene description |
| 1 | 213165_at | CDABP0086 | AI041204 | |
| 2 | 217432_s_at | | AF179281 | iduronate 2-sulfatase (Hunter syndrome) |
| 3 | 221500_s_at | | BE782754 | syntaxin 16 / |
| 4 | 208452_x_at | MYO9B | NM_004145 | myosin IXB |
| 5 | 220234_at | CA8 | NM_004056 | carbonic anhydrase VIII |
| 6 | 207865_s_at | BMP8 | NM_001720 | bone morphogenetic protein 8 (osteogenic protein 2) |
| 7 | 201769_at | KIAA0171 | NM_014666 | KIAA0171 gene product |

(continued)

| Sequence identification | | | | |
|---|---|---|---|---|
| SEQ ID NO: | psid | Gene Name | Accession # | Gene description |
| 8 | 218940_at | FLJ13920 | NM_024558 | hypothetical protein FLJ13920 |
| 9 | 209018_s_at | BRPK | BF432478 | protein kinase BRPK |
| 10 | 216647_at | DKFZp586L1 824 | AL117663 | from clone DKFZp586L1824 |
| 11 | 213405_at | DKFZp564E1 22 | N95443 | from clone DKFZp564E122 |
| 12 | 202921_s_at | ANK2 | N_001148 | ankyrin 2, neuronal, transcript variant 1 |
| 13 | 208401_s_at | | U01157 | glucagon-like peptide-1 receptor with CA dinucleotide repeat |
| 14 | 218090_s_at | WDR11 | NM_018117 | WD40 repeat domain 11 protein |
| 15 | 218139_s_at | FLJ10813 | N_018229 | hypothetical protein FLJ10813 |
| 16 | 202485_s_at | MBD2 | NM_003927 | methyl-CpG binding domain protein 2, transcript variant 1 |
| 17 | 201357_s_at | SF3A1 | NM_005877 | splicing factor 3a, subunit 1, 120kD |
| 18 | 214616_at | H3FD | NM_003532 | H3 histone family, member D |
| 19 | 207719_x_at | KIAA0470 | NM_014812 | KIAA0470 gene product |
| 20 | 202734_at | TRIP10 | NM_004240 | thyroid hormone receptor interactor 10 |
| 21 | 202175_at | FLJ22678 | NM_024536 | hypothetical protein FLJ22678 |
| 22 | 213870_at | | AL031228 | clone 1033B10 on chromosome 6p21.2-21.31 |
| 23 | 208967_s_at | adk2 | U39945 | adenylate kinase 2 |
| 24 | 204312_x_at | | AI655737 | cAMP responsive element binding protein 1 |
| 25 | 203815_at | GSTT1 | NM_000853 | glutathione S-transferase $\zeta$ 1 |
| 26 | 207996_s_at | C18ORF1 | NM_004338 | chromosome 18 open reading frame 1 |
| 27 | 221435_x_at | HT036 | NM_031207 | hypothetical protein HT036 |
| 28 | 219987_at | FLJ12684 | NM_024534 | hypothetical protein FLJ12684 |
| 29 | 221559_s_at | MGC:2488 | BC000229 | clone MGC:2488 |
| 30 | 207007_at | NR1I3 | NM_005122 | nuclear receptor subfamily 1, group I, mem 3 |
| 31 | 219265_at | FLJ13204 | NM_024761 | hypothetical protein FLJ13204 |
| 32 | 40420_at | | AB015718 | lok mRNA for protein kinase |
| 33 | 202266_at | AD022 | NM_016614 | TRAF and TNF receptor-associated protein |
| 34 | 219522_at | FJX1 | NM_014344 | putative secreted ligand homologous to fjx1 |
| 35 | 212334_at | AKAP350C | BE880245 | AKAP350C, alternatively spliced |
| 36 | 219340_s_at | CLN8 | AF123759 | Putative transmembrane protein |
| 37 | 217771_at | GP73 | NM_016548 | Golgi membrane protein (LOC51280) |
| 38 | 202418_at | Yif1p | NM_020470 | Putative transmembrane protein; homolog of veast Golgi membrane protein |
| 39 | 206295_at | IL-18 | NM_001562 | Interleukin 18 |

(continued)

| Sequence identification | | | | |
|---|---|---|---|---|
| SEQ ID NO: | psid | Gene Name | Accession # | Gene description |
| 40 | 201091_s_at | | BE748755 | Heterochromatin-like protein |
| 41 | 204015_s_at | DUSP4 | BC002671 | Dual specificity phosphatase 4 |
| 42 | 200726_at | PPP1CC | NM_002710 | Protein phosphatase 1, catalytic subunit, $\gamma$ isoform |
| 43 | 200965_s_at | ABLIM-s | NM_006720 | Actin binding LIM protein 1, transcript variant |
| 44 | 210314_x_at | TRDL-1 | AF114013 | Tumor necrosis factor-related death ligand 1 $\gamma$ |
| 45 | 221882_s_at | M83 | AI636233 | Five-span transmembrane protein |
| 46 | 217767_at | C3 | NM_000064 | Complement component 3 |
| 47 | 219588_s_at | FLJ20311 | NM_017760 | hypothetical protein |
| 48 | 204073_s_at | C11ORF9 | NM 013279 | chromosome 11 open reading frame 9 |
| 49 | 212567_s_at | | AL523310 | Putative translation initiation factor |
| 50 | 211382_s_at | TACC2 | AF220152 | |
| 51 | 201663_s_at | CAP-C | NM_005496 | chromosome-associated polypeptide C |
| 52 | 221344_at | OR12D2 | NM_013936 | Olfactory receptor, family 12, subfamily D, member 2 |
| 53 | 210028_s_at | ORC3 | AF125507 | Origin recognition complex subunit 3 |
| 54 | 218782_s_at | PRO2000 | NM_014109 | PRO2000 protein |
| 55 | 201664_at | SMC4 | AL136877 | (Structural maintenance of chromosome 4, yeast)-like |
| 56 | 219724_s_at | KIAA0748 | NM_014796 | KIAA0748 gene product |
| 57 | 204014_at | DUSP4 | NM_001394 | Dual specificity phosphatase 4 |
| 58 | 212014_x_at | CD44 | AI493245 | CD44 |
| 59 | 202240_at | PLK1 | NM_005030 | Polo (Drosophila)-like kinase 1 |
| 60 | 204740_at | CNK1 | NM_006314 | connector enhancer of KSR-like (Drosophila kinase suppressor of ras) |
| 61 | 208180_s_at | H4FH | N_003543 | H4 histone family, member H |
| 62 | 204768_s_at | FEN1 | NM_004111 | Flap structure-specific endonuclease |
| 63 | 203391_at | FKBP2 | NM_004470 | FK506-binding protein 2 |
| 64 | 211762_s_at | KPNA2 | BC005978 | Karyopherin $\alpha$ 2 (RAG cohort 1, importin $\alpha$ 1) |
| 65 | 218914_at | CGI-41 | NM_015997 | CGI-41 protein |
| 66 | 221028_s_at | MGC11335 | NM_030819 | hypothetical protein MGC11335 |
| 67 | 211779_x_at | MGC13188 | BC006155 | Clone MGC:13188 |
| 68 | 218883_s_at | FLJ23468 | NM_024629 | hypothetical protein FLJ23468 |
| 69 | 204888_s_at | | AA772093 | Neuralized (Drosophila)-like |
| 70 | 217815_at | FACTP140 | NM_007192 | Chromatin-specific transcription elongation factor, 140 kD subunit |
| 71 | 201368_at | Tis11d | U07802 | |
| 72 | 201288_at | ARHGDIB | NM_001175 | Rho GDP dissociation inhibitor (GDI) $\beta$ |

(continued)

| SEQ ID NO: | psid | Gene Name | Accession # | Gene description |
|---|---|---|---|---|
| | Sequence identification | | | |
| 73 | 201068_s_at | PSMC2 | NM_002803 | Proteasome (prosome, macropain) 26S subunit, ATPase, 2 |
| 74 | 218478_s_at | DKFZP434E2220 | NM_017612 | hypothetical protein DKFZP434E2220 |
| 75 | 214919_s_at | KIAA1085 | R39094 | |
| 76 | 209835_x_at | | BC004372 | Similar to CD44 |
| 77 | 217471_at | | AL117652 | |
| 78 | 203306_s_at | SLC35A1 | NM_006416 | Solute carrier family 35 (CMP-sialic acid transporter), member 1 |
| 79 | 205034_at | CCNE2 | NM_004702 | Cyclin E2 |
| 80 | 221816_s_at | | BF055474 | Putative zinc finger protein NY-REN-34 antigen |
| 81 | 219510_at | POLQ | NM_006596 | Polymerase (DNA directed) $\zeta$ |
| 82 | 217102_at | | AF041410 | Malignancy-associated protein |
| 83 | 208683_at | CANP | M23254 | Ca2-activated neutral protease large subunit |
| 84 | 215510 at | | AV693985 | ets variant gene 2 |
| 85 | 218533_s_at | FLJ20517 | NM_017859 | hypothetical protein FLJ20517 |
| 86 | 215633_x_at | LST-1 N | AV713720 | mRNA for LST-1N protein |
| 87 | 221928_at | | AI057637 | Hs234898 ESTs, weakly similar to 2109260A B-cell growth factor |
| 88 | 214806_at | BICD | U90030 | Bicaudal-D |
| 89 | 204540_at | EEF1A2 | NM_001958 | eukaryotic translation elongation factor 1 $\alpha$ 2 |
| 90 | 221916_at | | BF055311 | hypothetical protein |
| 91 | 216693_x_at | DKFZp434C1722 | AL133102 | |
| 92 | 209500_x_at | | AF114012 | tumor necrosis factor-related death liqand-1$\beta$ |
| 93 | 209534_at | FLJ10418 | AK001280 | moderately similar to Hepatoma-derived growth factor |
| 94 | 207118_s_at | MMP23A | NM_004659 | matrix metalloproteinase 23A |
| 95 | 211040_x_at | | BC006325 | G-2 and S-phase expressed 1 |
| 96 | 218430_s_at | FLJ12994 | NM 022841 | hypothetical protein FLJ 12994 |
| 97 | 217404_s_at | | X16468 | $\alpha$-1 type II collagen. |
| 98 | 205848_at | GAS2 | NM_005256 | qrowth arrest-specific 2 |
| 99 | 214915_at | FLJ11780 | AK021842 | clone HEMBA1005931, weakly similar to zinc finger protein 83 |
| 100 | 216010_x_at | | D89324 | $\alpha$ (1,31,4) fucosyltransferase |
| 101 | 204631_at | MYH2 | NM_017534 | myosin heavy polypep 2 skeletal muscle adult |
| 102 | 202687_s_at | | U57059 | Apo-2 ligand mRNA |

(continued)

| | SEQ ID NO: | psid | Gene Name | Accession # | Gene description |
|---|---|---|---|---|---|
| | | | Sequence identification | | |
| | 103 | 221634_at | | BC000596 | Similar to ribosomal protein L23a, clone MGC:2597 |
| | 104 | 220886_at | GABRQ | NM_018558 | γ-aminobutyric acid (GABA) receptor, ζ |
| | 105 | 202237_at | ADPRTL1 | NM_006437 | ADP-ribosyltransferase (NAD+; poly (ADP-ribose) polymerase)-like 1 |
| | 106 | 204218_at | DKFZP564M 082 | NM_014042 | protein DKFZP564M082 |
| | 107 | 221241_s_at | BCLG | NM_030766 | apoptosis regulator BCL-G |
| | 108 | 209862_s_at | | BC001233 | Similar to KIAA0092 gene product, clone MGC:4896 |
| | 109 | 217019_at | RPS4X | AL137162 | Contains novel gene and 5 part of gene for novel protein similar to X-linked ribosomal protein 4 |
| | 110 | 210593 at | | M55580 | spermidinespermine N1-acetyltransferase |
| | 111 | 216103_at | KIAA0707 | AB014607 | KIAA0707 |
| | 112 | 202513_s_at | PPP2R5D | NM_006245 | protein phosphatase 2, regulatory subunit B (B56), delta isoform |
| | 113 | 202521_at | CTCF | NM_006565 | CCCTC-binding factor (zinc finger protein) |
| | 114 | 218682_s_at | SLC4A1AP | NM_018158 | solute carrier family 4 (anion exchanger member 1, adaptor protein |
| | 115 | 203436_at | RPP30 | NM_006413 | ribonuclease P |
| | 116 | 220127_s_at | FLJ20188 | NM_017703 | hypothetical protein FLJ20188 |
| | 117 | 212355_at | KIAA0323 | AI075450 | KIAA0323 protein |
| | 118 | 215158_s_at | FLJ12469 | AK022531 | cDNA FLJ12469 |
| | 119 | 209429_x_at | | AF112207 | translation initiation factor elF-2b delta subunit |
| | 120 | 200097_s_at | | AI701949 | heterogeneous nuclear ribonucleoprotein K |
| | 121 | 203040_s_at | HMBS | NM_000190 | hydroxymethylbilane synthase |
| | 122 | 221647_s_at | DKFZp586O 0222 | AL136935 | clone DKFZp586O0222 |
| | 123 | 218089_at | C20orf4 | NM_015511 | chromosome 20 open reading frame 4 DKFZP564N1363 |
| | 124 | 203732_at | TRIP4 | NM_016213 | thyroid hormone receptor interactor 4 |
| | 125 | 202854_at | HPRT1 | NM_000194 | hypoxanthine phosphoribosyltransferase 1 (Lesch-Nyhan syndrome) |
| | 126 | 205545_x_at | DNAJC8 | NM_014280 | DnaJ (Hsp40) homolog, subfamily C, mem 8 |
| | 127 | 202703_at | DUSP11 | NM_003584 | dual specificity phosphatase 11 (RNA/RNP complex 1-interacting) |
| | 128 | 216903_s_at | CBARA1 | AK022697 | calcium binding atopy-related autoantigen 1 |
| | 129 | 203090_at | SDF2 | NM_006923 | stromal cell-derived factor 2 |
| | 130 | 209214_s_at | EWSR1 | BC004817 | Ewing sarcoma breakpoint region 1 |
| | 131 | 217798_at | CNOT2 | AI123426 | CCR4-NOT transcription complex, subunit 2 |
| | 132 | 201178_at | FBXO7 | NM_012179 | F-box only protein 7 |

(continued)

| | Sequence identification | | | |
|---|---|---|---|---|
| SEQ ID NO: | psid | Gene Name | Accession # | Gene description |
| 133 | 212160_at | | AI984005 | exportin, tRNA (nuclear export receptor for tRNAs) |
| 134 | 201111_at | CSE1 | AF053641 | brain cellular apoptosis susceptibility protein |
| 135 | 201112_s_at | CSE1L | NM_001316 | chromosome segregation 1 (yeast homolog)-like |
| 136 | 204817_at | KIAA0165 | NM_012291 | extra spindle poles, S. cerevisiae, homolog of (KIAA0165) |
| 137 | 215623_x_at | FLJ11338 | AK002200 | highly similar to chromosome-associated polypeptide-C mRNA |
| 138 | 38158_at | KIAA0165 | D79987 | |
| 139 | 201076_at | NHP2L1 | NM_005008 | non-histone chromosome protein 2 (S. cerevisiae)-like 1 |
| 140 | 201947_s_at | CCT2 | NM_006431 | chaperonin containing TCP1, subunit 2 (beta) |
| 141 | 202647_s_at | NRAS | NM_002524 | neuroblastoma RAS viral (v-ras) oncogene homolog |
| 142 | 202705_at | CCNB2 | NM_004701 | cyclin B2 |
| 143 | 204009_s_at | | NM_004985 | v-Ki-ras2 Kirsten rat sarcoma 2 viral oncogene homolog |
| 144 | 204566_at | PPM1D | NM_003620 | protein phosphatase 1 D magnesium-dependent, delta isoform |
| 145 | 214710_s_at | | BE407516 | |
| 146 | 202095_s_at | BIRC5 | NM_001168 | baculoviral IAP repeat-containing 5 (survivin) |
| 147 | 204900_x_at | SAP30 | NM_003864 | sin3-associated polypeptide, 30kD |
| 148 | 201986_at | KIAA0593 | AB011165 | KIAA0593 protein mRNA, partial cds |
| 149 | 201987_at | | AI984051 | thyroid hormone receptor-associated protein, 240 kDa subunit |
| 150 | 203605_at | SRP54 | NM_003136 | signal recognition particle 54kD |
| 151 | 213226_at | | AI346350 | polymyositisscleroderma autoantigen 1 (75kD) |
| 152 | 205757_at | ENTPD5 | NM_001249 | ectonucleoside triphosphate diphosphohydrolase 5 |
| 153 | 212062_at | KIAA0611 | AB014511 | mRNA for KIAA0611 protein |
| 154 | 213007_at | | W74442 | polymerase (DNA directed), $\gamma$ |
| 155 | 203362_s_at | MAD2L1 | NM_002358 | MAD2 (mitotic arrest deficient, yeast, homolog)-like 1 |
| 156 | 204641_at | NEK2 | NM_002497 | NIMA (never in mitosis gene a)-rel. kinase 2 |
| 157 | 206983 at | CCR6 | NM_004367 | chemokine (C-C motif) receptor 6 |
| 158 | 210375_at | EP3a2 | X83858 | prostaglandin E receptor |
| 159 | 213933_at | DKFZp586M0723 | AW242315 | from clone DKFZp586M0723 |
| 160 | 201756_at | RPA2 | NM_002946 | replication protein A2 (32kD) |
| 161 | 208688_x_at | eIF3 | U78525 | eukaryotic translation initiation factor |
| 162 | 212655_at | KIAA0579 | AB011151 | KIAA0579 protein |

(continued)

| Sequence identification | | | | |
|---|---|---|---|---|
| SEQ ID NO: | psid | Gene Name | Accession # | Gene description |
| 163 | 213124_at | | BG538800 | DKFZP434N043 protein |
| 164 | 213520_at | RECQL4 | NM_004260 | RecQ protein-like 4 |
| 165 | 218277_s_at | FLJ22060 | NM_024612 | hypothetical protein FLJ22060 |
| 166 | 201938_at | DOC1 | NM_004642 | deleted in oral cancer (mouse, homolog) 1 |
| 167 | 202692_s_at | UBTF | NM_014233 | upstream binding transcription factor, RNA polymerase I |
| 168 | 203616_at | POLB | NM_002690 | polymerase (DNA directed), $\beta$ |
| 169 | 204407_at | | AF080255 | lodestar protein |
| 170 | 206188_at | KIAA0628 | NM 014789 | KIAA0628 gene product |
| 171 | 209831_x_at | | AB004574 | deoxyribonuclease II, lysosomal |
| 172 | 211980_at | | AI922605 | collagen, type IV, alpha 1 |
| 173 | 214853_s_at | | AI091079 | (Src homology 2 domain-containing) transforming protein 1 |
| 174 | 215888_at | FLJ23236 | AK026889 | clone COL00725 |
| 175 | 216037_x_at | | AA664011 | transcription factor 7-like 2 (T-cell specific, HMG-box) |
| 176 | 202666_s_at | BAF53A | NM_004301 | BAF53 |
| 177 | 204146_at | | BE966146 | RAD51-interacting protein |
| 178 | 203920_at | NR1H3 | NM_005693 | nuclear receptor subfamily 1, group H, mem 3 |
| 179 | 205322_s_at | | AW182367 | metal-regulatory transcription factor 1 |
| 180 | 206644_at | NR0B1 | NM_000475 | nuclear receptor subfamily 0, group B, mem 1 |
| 181 | 201558_at | RAE1 | NM_003610 | (RNA export 1, S. pombe) homolog |
| 182 | 209448_at | | BC002439 | Tat-interacting protein (30kD) |
| 183 | 220960_x_at | RPL22 | NM_000983 | ribosomal protein L22 |
| 184 | 207320_x_at | STAU | NM_004602 | staufen (Drosophila, RNA-binding protein) transcript variant T4 |
| 185 | 208948_s_at | MGC:4921 | BC000830 | MGC:4921 |
| 186 | 213037_x_at | | AJ132258 | staufen protein, partial Drosophila, RNA-binding protein) |
| 187 | 200725_x_at | RPL10 | NM_006013 | ribosomal protein L10 |
| 188 | 200937_s_at | RPL5 | NM_000969 | ribosomal protein L5 |
| 189 | 208696_at | PNAS-102 | AF275798 | PNAS-102 mRNA chaperonin containing TCP1, subunit 5 (epsilon) |
| 190 | 209593_s_at | FKSG18 | AF317129 | FKSG18 |
| 191 | 209619_at | | K01144 | MHC II antigen $\gamma$ chain |
| 192 | 218336_at | PFDN2 | NM_012394 | prefoldin 2 |
| 193 | 219390_at | FLJ20731 | NM_017946 | hypothetical protein FLJ20731 |
| 194 | 206976_s_at | HSP105B | NM_006644 | heat shock 105kD |

(continued)

| | Sequence identification | | | |
|---|---|---|---|---|
| SEQ ID NO: | psid | Gene Name | Accession # | Gene description |
| 195 | 204444_at | KNSL1 | NM_004523 | kinesin-like 1 |
| 196 | 206364_at | KIAA0042 | NM_014875 | KIAA0042 gene product |
| 197 | 209408_at | | U63743 | mitotic centromere-associated kinesin |
| 198 | 202629_at | APPBP2 | AV681579 | amyloid β precursor protein (cytoplasmic tail)-binding protein 2 |
| 199 | 202630_at | APPBP2 | AA046411 | amyloid β precursor protein (cytoplasmic tail)-binding protein 2 |
| 200 | 202631_s_at | APPBP2 | NM_006380 | amyloid β precursor protein (cytoplasmic tail)-binding protein 2 |
| 201 | 210629_x_at | | AF000425 | cLST1A splice variant |
| 202 | 204670_x_at | HLA-DRB5 | NM_002125 | MHC, class II, DR β 5 |
| 203 | 208306_x_at | HLA-DRB4 | NM_021983 | MHC, class II, DR β 4 |
| 204 | 206654_s_at | RPC32 | NM_006467 | polymerase (RNA) III (DNA directed) (32kD) |
| 205 | 218360_at | RAB22A | NM_020673 | RAB22A, member RAS oncogene family |
| 206 | 209380_s_at | CFTRMRP | AF146074 | ABC protein, TP-binding cassette, sub-family C |
| 207 | 201114_x_at | PSMA7 | NM_002792 | proteasome (prosome, macropain) subunit, α type, 7 |
| 208 | 202243_s_at | PSMB4 | NM_002796 | proteasome (prosome, macropain) subunit, β type, 4 |
| 209 | 202244_at | PSMB4 | NM_002796 | proteasome (prosome, macropain) subunit, β type, 4 |
| 210 | 203878_s_at | MMP11 | NM_005940 | matrix metalloproteinase 11 (stromelysin 3) |
| 211 | 216474_x_at | | AF206667 | mast cell β I tryptase, alternatively spliced |
| 212 | 217009_at | | AL121974 | DNA sequence from clone RP3-417L20 on chromosome 6p12-21.3 |
| 213 | 202968_s_at | Dyrk2 | Y09216 | mRNA for protein kinase, Dyrk2 |
| 214 | 202969_at | | AI216690 | dual-specificity tyrosine-(Y)-phosphorylation regulated kinase 2 |
| 215 | 204092_s_at | STK15 | NM_003600 | serinethreonine kinase 15 |
| 216 | 204171_at | RPS6KB1 | NM_003161 | ribosomal protein S6 kinase, 70kD, polypep 1 |
| 217 | 204825_at | KIAA0175 | NM_014791 | KIAA0175 gene product |
| 218 | 208079_s_at | STK6 | NM_003158 | serinethreonine kinase 6 |
| 219 | 219148_at | TOPK | NM_018492 | PDZ-binding kinase; T-cell originated protein kinase |
| 220 | 219813_at | LATS1 | NM_004690 | LATS (large tumor suppressor, Drosophila) homolog 1 |
| 221 | 202779_s_at | E2-EPF | NM_014501 | ubiquitin carrier protein |
| 222 | 217978_s_at | HSA243666 | NM_017582 | NICE-5 protein |
| 223 | 210413_x_at | SCCA2 | U19557 | squamous cell carcinoma antigen 2 |
| 224 | 219478_at | WFDC1 | NM_021197 | WAP four-disulfide core domain 1 |
| 225 | 204319_s_at | RGS10 | NM 002925 | regulator of G-protein signalling 10 |

(continued)

| Sequence identification | | | | |
| --- | --- | --- | --- | --- |
| SEQ ID NO: | psid | Gene Name | Accession # | Gene description |
| 226 | 204017_at | KDELR3 | NM_006855 | KDEL (Lys-Asp-Glu-Leu) endoplasmic reticulum protein retention receptor 3 |
| 227 | 206150_at | TNFRSF7 | NM_001242 | tumor necrosis factor receptor superfamily, member 7 |
| 228 | 205926_at | WSX-1 | NM_004843 | class I cytokine receptor |
| 229 | 205400_at | WAS | NM_000377 | Wiskott-Aldrich syndrome (eczema-thrombocytopenia) |
| 230 | 209539_at | KIAA0006 | D25304 | mRNA for KIAA0006 gene |
| 231 | 221922_at | | AW195581 | KIAA0761 protein |
| 232 | 200614_at | CLTC | NM_004859 | clathrin, heavy polypeptide (Hc) |
| 233 | 202550_s_at | VAPB | NM_004738 | VAMP (vesicle-associated membrane protein)-associated protein B and C |
| 234 | 212159_x_at | | AI125280 | adaptor-related protein complex 2, $\alpha$ 2 subunit |
| 235 | 202733_at | P4HA2 | NM_004199 | procollagen-proline 2-oxoglutarate 4-dioxygenase (proline 4-hydroxylase) $\alpha$ polypep II |
| 236 | 208905_at | | BC005299 | cytochrome C, clone MGC:12367 |
| 237 | 32137_at | JAG2 | AF029778 | Jagged2 |
| 238 | 201088_at | KPNA2 | NM_002266 | karyopherin $\alpha$ 2 (RAG cohort 1, importin $\alpha$ 1) |
| 239 | 202824_s_at | TCEB1 | NM_005648 | transcription elongation factor B (SIII), polypeptide 1 (15kD, elongin C) |
| 240 | 201584_s_at | DDXL | NM_005804 | nuclear RNA helicase, DECD variant of DEAD box family |
| 241 | 218461_at | LOC51184 | NM_016301 | protein x 0004 (LOC51184) |
| 242 | 204489_s_at | CD44 | NM_000610 | CD44 |
| 243 | 204490_s_at | CDw44 | M24915 | CDw44 antigen |
| 244 | 207165_at | HMMR | NM_012485 | hyaluronan-mediated motility receptor (RHAMM) |
| 245 | 210916_s_at | CD44 | AF098641 | CD44 isoform RC |
| 246 | 212063_at | CMPX1 | BE903880 | zinc finger protein 6 |
| 247 | 204470_at | GRO1 | NM_001511 | GRO1 oncogene (melanoma growth stimulating activity, alpha) |
| 248 | 207430_s_at | MSMB | NM_002443 | microseminoprotein, $\beta$ |
| 249 | 210297_s_at | | U22178 | prostatic secretory protein 57 |
| 250 | 213009_s_at | FLJ12639 | AK022701 | cDNA FLJ12639 |
| 251 | 203536_s_at | CIAO1 | NM_004804 | WD40 protein Ciao1 |
| 252 | 204026_s_at | ZWINT | NM_007057 | ZW10 interactor |
| 253 | 204435_at | KIAA0410 | NM_014778 | KIAA0410 qene product |
| 254 | 209271_at | | AB032251 | BPTF mRNA for bromodomain PHD finger transcription factor |
| 255 | 212074 at | KIAA0810 | BE972774 | KIAA0810 protein |

(continued)

| Sequence identification | | | | |
|---|---|---|---|---|
| SEQ ID NO: | psid | Gene Name | Accession # | Gene description |
| 256 | 218009_s_at | PRC1 | NM_003981 | protein regulator of cytokinesis 1 |
| 257 | 218768_at | NUP107 | NM_020401 | nuclear pore complex protein |
| 258 | M33197_3_at | GAPDH | M33197 | glyceraldehyde-3-phosphate dehydrogenase |
| 259 | 203524_s_at | MPST | NM_021126 | mercaptopyruvate sulfurtransferase |
| 260 | 206335_at | GALNS | NM_000512 | galactosamine (N-acetyl)-6-sulfate sulfatase (Morquio syndrome, mucopolysaccharidosis type IVA) |
| 261 | 203503_s_at | PEX14 | NM_004565 | peroxisomal biogenesis factor 14 |
| 262 | 202673_at | DPM1 | NM_003859 | dolichyl-phosphate mannosyltransferase potypeptide 1, catalytic subunit 1 |
| 263 | 207543_s_at | P4HA1 | NM_000917 | procollagen-proline, 2-oxoglutarate 4-dioxygenase (proline 4-hydroxylase), $\alpha$ polypeptide I |
| 264 | 204192_at | CD37 | NM_001774 | CD37 antigen |
| 265 | 204960 at | PTPRCAP | NM_005608 | protein tyrosine phosphatase, receptor type, C-associated protein |
| 266 | 211991_s_at | | M27487 | MHC class II DPw3-alpha-1 chain |
| 267 | 200822_x_at | TPI1 | NM_000365 | triosephosphate isomerase 1 |
| 268 | 219502_at | FLJ10858 | NM_018248 | hypothetical protein FLJ10858 |
| 269 | 219499_at | FLJ10578 | NM_018144 | hypothetical protein FLJ10578 |
| 270 | 209238_at | | BE966922 | syntaxin 3A |
| 271 | 212593_s_at | | N92498 | |
| 272 | 201598_s_at | INPPL1 | NM_001567 | inositol polyphosphate phosphatase-like 1 |
| 273 | 201760_s_at | LOC55884 | NM_018639 | CS box-containing WD protein |
| 274 | 222077_s_at | | AU153848 | GTPase activating protein |
| 275 | 203764_at | KIAA0008 | NM_014750 | KIAA0008 gene product |
| 276 | 59705_at | | AA911739 | |
| 277 | 204070_at | RARRES3 | NM_004585 | retinoic acid receptor responder (tazarotene induced) 3 |
| 278 | 212149_at | | AW470003 | |
| 279 | 214039_s_at | DKFZp586E1124 | T15777 | DKFZp586E1124 |
| 280 | 217956_s_at | MASA | NM_021204 | E-1 enzyme |
| 281 | 200994_at | | BG291787 | RAN binding protein 7 |
| 282 | 200995_at | | AI741392 | |
| 283 | 205106_at | MTCP1 | NM_014221 | mature T-cell proliferation 1 |
| 284 | 209035_at | hMK-1 | M69148 | Midkine (neurite growth-promoting factor 2) |
| 285 | 210311_at | FGF5 | AF171928 | fibroblast growth factor 5 short variant |

(continued)

| | Sequence identification | | | |
|---|---|---|---|---|
| SEQ ID NO: | psid | Gene Name | Accession # | Gene description |
| 286 | 211251_x_at | NFY-C | U78774 | NFY-C mRNA |
| 287 | 220406_at | TGFB2 | NM_003238 | transforming growth factor.β 2 |
| 288 | 206967 at | CCNT1 | NM_001240 | cyclin T1 |
| 289 | 204252_at | | M68520 | cdc2-related protein kinase mRNA |
| 290 | 205955_at | FLJ11136 | NM 018336 | hypothetical protein FLJ11136 |
| 291 | 203258_at | DRAP1 | NM_006442 | DR1-assoc. protein 1 (negative cofactor 2 a) |
| 292 | 204022_at | | AI668780 | |
| 293 | 207660_at | | NM_004019 | dystrophin (muscular dystrophy, Duchenne and Becker types), |
| 294 | 215050_x_at | | BG325734 | mitogen-activated protein kinase-activated protein kinase 2 |
| 295 | 204337_at | | AL514445 | regulator of G-protein signaling 4 |
| 296 | 217687_at | | AA224446 | |
| 297 | 221546_at | | BC000794 | pre-mRNA splicing factor similar to S. cerevisiae Prp18 |
| 298 | 206546_at | SYCP2 | NM_014258 | synaptonemal complex protein 2 |
| 299 | 206278_at | | D10202 | mRNA for platelet-activating factor receptor |
| 300 | 206429_at | F2RL1 | NM_005242 | coagulation factor II (thrombin) receptor-like 1 |
| 301 | 216408_at | | AJ302584 | gene for olfactory receptor, cell line BM28.7 |
| 302 | 221306_at | GPR27 | NM_018971 | G protein-coupled receptor 27 |
| 303 | 221442_at | MC3R | NM_019888 | melanocortin 3 receptor |
| 304 | 201446_s_at | | BF692742 | |
| 305 | 205018_s_at | | NM_005757 | C3H-type zinc finger protein; similar to D. melanogaster muscleblind B protein (MBLL) |
| 306 | 214379_at | | AI954458 | |
| 307 | 214698_at | | AW190873 | |
| 308 | 219336_s_at | | NM_015947 | CGI-18 protein (LOC51008) |
| 309 | 220760_x_at | FLJ14345 | NM_024733 | hypothetical protein FLJ14345 |
| 310 | 221480_at | | BG180941 | |
| 311 | 221615_at | PPIE | AF104013 | peptidyl-prolyl cis-trans isomerase E |
| 312 | 221923_s_at | | AA191576 | nucleophosmin (nucleolar phosphoprotein B23, numatrin) |
| 313 | 201211_s_at | DDX3 | AF061337 | DEAD box RNA helicase DDX3 |
| 314 | 205638_at | BAI3 | NM_001704 | brain-specific angiogenesis inhibitor 3 |
| 315 | 205881_at | ZNF74 | NM_003426 | zinc finger protein 74 (Cos52) |
| 316 | 206179_s_at | | NM_007030 | brain-specific protein p25 alpha (p25) |
| 317 | 206308_at | | AJ223333 | mRNA for putative DNA methyltransferase |

(continued)

| | SEQ ID NO: | psid | Gene Name | Accession # | Gene description |
|---|---|---|---|---|---|
| Sequence identification | | | | | |
| | 318 | 207361_at | HBP1 | NM_012257 | HMG-box containing protein 1 |
| | 319 | 208902_s_at | | BF431363 | |
| | 320 | 209603_at | | AI796169 | |
| | 321 | 214174_s_at | | BE043700 | |
| | 322 | 215747_s_at | | X06130 | mRNA for cell cycle gene RCC1 |
| | 323 | 216480_x_at | AF10CALM | AF060927 | type I AF10CALM fusion protein |
| | 324 | 216711_s_at | | M73444 | CCG1p mRNA |
| | 325 | 222115_x_at | | BC003693 | Similar to RIKEN cDNA 3930401 K13 qene |
| | 326 | 221686_s_at | DKFZp434J0 450 | AL136869 | DKFZp434J0450 |
| | 327 | 210533_at | MSH4 | AF104243 | meiosis-specific MutS homolog |
| | 328 | 217485_x_at | hPMS3 | D38435 | hPMS3 mRNA |
| | 329 | 202162_s_at | | AI769416 | CCR4-NOT transcription complex, subunit 8 |
| | 330 | 202401_s_at | SRF | NM_003131 | serum response factor (c-fos serum response element-binding transcription factor) |
| | 331 | 206067_s_at | | NM_024426 | Wilms tumor 1 (WT1), transcript variant D |
| | 332 | 206127_at | ELK3 | NM_005230 | ETS-domain protein (SRF accessory protein 2) |
| | 333 | 207402_at | ZNF132 | NM_003433 | zinc finger protein 132 |
| | 334 | 207768_at | EGR4 | NM_001965 | early growth response 4 |
| | 335 | 208414_s_at | HOXB3 | N_002146 | homeo box B3 |
| | 336 | 214879_x_at | | AY007087 | clone TCCCIA00046, upstream transcription factor 2, c-fos interacting |
| | 337 | 219314_s_at | ZNF219 | NM_016423 | zinc finger protein 219 |
| | 338 | 219779_at | FLJ20980 | NM_024721 | hypothetical protein FLJ20980 |
| | 339 | 220653_at | ZIM2 | NM_015363 | zinc finger, imprinted 2 |
| | 340 | 219778_at | FOG2 | NM_012082 | Friend of GATA2 |
| | 341 | 203947_at | CSTF3 | NM_001326 | cleavage stimulation factor, 3 pre-RNA, subunit 3,77kD |
| | 342 | 220096_at | FLJ20378 | NM_017795 | hypothetical protein FLJ20378 |
| | 343 | 201326_at | | BE737030 | chaperonin cont'ing TCP1, subunit 6A (zeta 1) |
| | 344 | 206769_at | TMSB4Y | N_004202 | thymosin, beta 4, Y chromosome |
| | 345 | 211197_s_at | KIAA0653 | AL355690 | EST from clone 34465, full insert |
| | 346 | 204994_at | MX2 | NM_002463 | myxovirus (influenza) resistance 2, murine homolog |
| | 347 | 201662_s_at | | D89053 | Acyl-CoA synthetase 3 |
| | 348 | 206141_at | MOCS3 | NM_014484 | molybdopterin synthase sulfurylase |
| | 349 | 209992_at | | AB044805 | 6-phosphofructo-2-kinase heart isoform |

(continued)

| Sequence identification | | | | |
|---|---|---|---|---|
| SEQ ID NO: | psid | Gene Name | Accession # | Gene description |
| 350 | 210160_at | | BC000398 | platelet-activating factor acetylhydrolase, isoform lb, beta subunit (30kD), |
| 351 | 218016_s_at | FLJ10509 | NM_018119 | hypothetical protein FLJ10509 |
| 352 | 220582_at | FLJ12190 | NM_025071 | hypothetical protein FLJ12190 |
| 353 | 222294_s_at | | AW971415 | |
| 354 | 202239_at | ADPRTL1 | NM_006437 | ADP-ribosyltransferase (NAD+; poly(ADP-ribose) polymerase)-like 1 |
| 355 | 205342_s_at | | AF026303 | sulfotransferase family, cytosolic, 1C, member 1 |
| 356 | 202294_at | | AI126490 | |
| 357 | 201597_at | COX7A2 | NM 001865 | Cyt C oxidase subunit VIIa polypeptide 2 (liver) |
| 358 | 206353_at | COX6A2 | NM_005205 | Cyt C oxidase subunit VIa polypeptide 2 |
| 359 | 218739_at | LOC51099 | NM 016006 | CGI-58 protein |
| 360 | 217557_s_at | | AV710357 | |
| 361 | 202413_s_at | USP1 | NM_003368 | ubiquitin specific protease 1 |
| 362 | 213661_at | | AI671186 | DKFZP586H2123 protein |
| 363 | 212729_at | | AI916274 | KIAA1232 protein |
| 364 | 202951_at | | BE048506 | serine threonine protein kinase |
| 365 | 207667_s_at | MAP2K3 | NM_002756 | mitogen-activated protein kinase kinase 3 |
| 366 | 212565_at | | BE302191 | KIAA0965 protein |
| 367 | 212740_at | | BF740111 | phosphoinositide-3-kinase, regulatory subunit 4, p150 |
| 368 | 213490_s_at | | AI762811 | mitogen-activated protein kinase kinase 2 |
| 369 | 213595_s_at | KIAA0451 | AA127643 | KIAA0451 qene product |
| 370 | 220640_at | CSNK1G1 | NM_022048 | casein kinase 1, gamma 1 |
| 371 | 207569_at | ROS1 | NM_002944 | v-ros avian UR2 sarcoma virus oncogene homolog 1 |
| 372 | 209041_s_at | | BG395660 | ubiquitin-conjugating enzyme E2G 2 |
| 373 | 207214_at | PEC-60 | NM_014471 | gastrointestinal peptide |
| 374 | 214425_at | | AV645756 | alpha-1-microglobulinbikunin precursor |
| 375 | 203650_at | EPCR | NM_006404 | protein C receptor, endothelial |
| 376 | 210733_at | PRO1292 | AF130055 | FLB4941 PRO1292, translocating chain-associating membrane protein |
| 377 | 220056_at | IL22R | NM_021258 | interleukin 22 receptor |
| 378 | 205049_s_at | CD79A | NM_001783 | CD79A antigen (immunoglobulin-associated alpha) transcript variant 1 |
| 379 | 211245_x_at | | AF002256 | killer cell inhibitory receptor homolog cl-9 mRNA, FEAn-like receptor, two domains, long cytoplasmic tail, 4 |

(continued)

| Sequence identification | | | | |
|---|---|---|---|---|
| SEQ ID NO: | psid | Gene Name | Accession # | Gene description |
| 380 | 212128_s_at | | AW411370 | dystroglycan 1 (dystrophin-associated glycoprotein 1) |
| 381 | 205019_s_at | VIPR1 | NM_004624 | vasoactive intestinal peptide receptor 1 |
| 382 | 206001_at | NPY | NM_000905 | neuropeptide Y |
| 383 | 216289_at | MAMMA1002427 | AU148039 | MAMMA1002427 |
| 384 | 208250_s_at | DMBT1 | NM_004406 | deleted in malignant brain tumors 1 |
| 385 | 202091_at | | BC003087 | binder of Arl Two, clone MGC:1121 |
| 386 | 205068_s_at | | BE671084 | GTPase regulator associated with the focal adhesion kinase pp125(FAK) |
| 387 | 221136_at | GDF2 | NM_016204 | growth differentiation factor 2 |
| 388 | 202688_at | TNFSF10 | NM_003810 | tumor necrosis factor (ligand) superfamily, member 10 |
| 389 | 214336_s_at | TNFSF10 | AI621079 | coatomer protein complex, subunit α, TNF (ligand) superfamily, member 10 |
| 390 | 213055_at | | BF693956 | CD47 antigen (Rh-related antigen, integrin-associated signal transducer) |
| 391 | 201719_s_at | EPB41 L2 | NM_001431 | erythrocyte membrane protein band 4.1-like 2 |
| 392 | 208353_x_at | ANK1 | NM_020480 | ankyrin 1, erythrocytic transcript variant 7 |
| 393 | 215717_s_at | | X62009 | partial mRNA for fibrillin 5 |
| 394 | 206826_at | PMP2 | NM_002677 | peripheral myelin protein 2 |
| 395 | 207542_s_at | AQP1 | NM_000385 | aquaporin 1 (channel-forming integral protein, 28kD) |
| 396 | 207596_at | PRO2176 | NM_018515 | hypothetical protein PRO2176 |
| 397 | 208297_s_at | EVI5 | NM_005665 | ecotropic viral integration site5 |
| 398 | 217289_s_at | G6PT | AF097831 | glucose-6-phosphate transporter |
| 399 | 205972_at | | NM_006841 | transporter protein; system N1 Na+ and H+-coupled glutamine transporter (G17) |
| 400 | 218835_at | SFTPA2 | NM_006926 | surfactant, pulmonary-associated protein A2 |
| 401 | 219716_at | APOL6 | NM_030641 | apolipoprotein L, 6 |
| 402 | 218928_s_at | SLC37A1 | NM_018964 | solute carrier family 37 (glycerol-3-phosphate transporter), member 1 |
| 403 | 214205_x_at | FLJ12069 | AK022131 | FLJ12069 |
| 404 | 205008_s_at | KIP2 | NM_006383 | DNA-dependent protein kinase catalytic subunit-interacting protein 2 |
| 405 | 211272_s_at | DAGK1 | AF064771 | clone 24 diacylglycerol kinase α |
| 406 | 206316_s_at | KIAA0166 | NM_014708 | KIAA0166 gene product |
| 407 | 209737_at | KIAA0705 | AB014605 | KIAA0705 protein |
| 408 | 213117_at | FLJ10262 | AW138594 | hypothetical protein FLJ10262 |
| 409 | 217161_x_at | | X17406 | cartilage specific proteoglycan |

(continued)

| Sequence identification | | | | |
|---|---|---|---|---|
| SEQ ID NO: | psid | Gene Name | Accession # | Gene description |
| 410 | 209436_at | KIAA0762 | AB018305 | KIAA0762 protein |
| 411 | 213993_at | | AI885290 | spondin 1, (f-spondin) extracellular matrix protein |
| 412 | 214354_x_at | | T91506 | N-acylsphingosine amidohydrolase (acid ceramidase)-like |
| 413 | 205799_s_at | | M95548 | amino acid transport protein |
| 414 | 213522_s_at | | AA527578 | solute carrier family 16 (monocarboxylic acid transporters), member 3 |
| 415 | 212393_at | | AL096767 | DNA sequence from clone 579N16 on chromosome 22 |
| 416 | 219179_at | LOC51339 | NM_016651 | heptacellular carcinoma novel gene-3 protein |
| 417 | 201502_s_at | | AI078167 | nuclear factor of kappa light polypeptide gene enhancer in B-cells inhibitor, $\alpha$ |
| 418 | 201512_s_at | | BC003633 | translocase of outer mitochondrial membrane 70 (yeast) homolog A |
| 419 | 201576_s_at | GLB1 | NM_000404 | galactosidase, beta 1 |
| 420 | 201765_s_at | | AL523158 | |
| 421 | 215155_at | HEXA | J04178 | abnormal $\beta$-hexosaminidase $\alpha$ chain |
| 422 | 203518_at | CHS1 | NM_000081 | Chediak-Higashi syndrome 1 |
| 423 | 220801_s_at | HAO2 | NM_016527 | hydroxyacid oxidase 2 (long chain) |
| 424 | 204690_at | STX8 | NM_004853 | syntaxin 8 |
| 425 | 201126_s_at | MGAT1 | NM_002406 | mannosyl ($\alpha$-,3-)-glycoprotein $\beta$-,2-N-acetylglucosaminyltransferase |
| 426 | 206925_at | SIAT8D | NM_005668 | sialyltransferase 8($\alpha$-2 8-polysialytransferase)D |
| 427 | 205319_at | PSCA | NM_005672 | prostate stem cell antigen |
| 428 | 206199_at | CEACAM7 | NM_006890 | carcinoembryonic antigen-rel. cell adhesion molecule 7 |
| 429 | 207695_s_at | IGSF1 | NM_001555 | immunoglobulin superfamily, member 1 |
| 430 | 219249_s_at | FLJ22041 | NM_021939 | hypothetical protein FLJ22041 |
| 431 | 213413_at | FLJ13555 | BG434174 | FLJ13555 |
| 432 | 219793_at | SNX16 | NM_022133 | sorting nexin 16 |
| 433 | 212035_s_at | KIAA1067 | AI817079 | KIAA1067 protein |
| 434 | 202730_s_at | PDCD4 | NM_014456 | programmed cell death 4 |
| 435 | 202130_at | sudD | AA725102 | suppressor of bimD6, Aspergillus nidulans homolog |
| 436 | 206623_at | PDE6A | NM_000440 | phosphodiesterase 6A, cGMP-specific, rod, $\alpha$ |
| 437 | 222201_s_at | KIAA1315 | AB037736 | KIAA1315 protein |
| 438 | 221752_at | KIAA1298 | AL041728 | KIAA1298 protein |
| 439 | 205003_at | KIAA0716 | NM_014705 | KIAA0716 gene product |

| Sequence identification | | | | |
|---|---|---|---|---|
| SEQ ID NO: | psid | Gene Name | Accession # | Gene description |
| 441 | 205006_s_at | NMT2 | NM_004808 | N-myristoyltransferase 2 |
| 442 | 205677_s_at | DLEU1 | NM_005887 | deleted in lymphocytic leukemia, 1 |
| 443 | 204947_at | E2F1 | NM_005225 | E2F transcription factor 1 |
| 444 | 207505_at | PRKG2 | NM_006259 | protein kinase, cGMP-dependent, type II |
| 445 | 211036_x_at | | BC006301 | anaphase-promoting complex subunit 5, clone MGC:13295, |
| 446 | 211269_s_at | | K03122 | interleukin-2 receptor mRNA (short form), |
| 447 | 202610_s_at | TRAP170 | AF135802 | thyroid hormone receptor-associated protein complex component |
| 448 | 204762_s_at | | BE670563 | guanine nucleotide binding protein (G protein), alpha activating activity polypeptide O |
| 449 | 201313_at | ENO2 | NM_001975 | enolase 2, (y, neuronal) |
| 450 | 203225_s_at | FLJ11149 | NM_018339 | hypothetical protein FLJ11149 |
| 451 | 221567_at | NOP | AF064599 | nucleolar protein Nop30 |
| 452 | 201518_at | CBX1 | NM_006807 | chromobox homolog 1 (Drosophila HP1 beta) |
| 453 | 204970_s_at | | NM_002359 | v-maf musculoaponeurotic fibrosarcoma (avian) oncogene family, protein G (MAFG), |
| 454 | 214615_at | P2Y10 | NM_014499 | putative purinergic receptor |
| 455 | 221386_at | OR3A2 | NM_002551 | olfactory receptor family 3 subfamily A mem 2 |
| 456 | 200014_s_at | HNRPC | NM_004500 | heterogeneous nuclear ribonucleoprotein C (C1C2) |
| 457 | 200053_at | SPAG7 | NM_004890 | sperm associated antigen 7 |
| 458 | 203462_x_at | EIF3S9 | NM_003751 | translation initiation factor 3 sub 9 (eta 116kD) |
| 459 | 205917_at | ZNF264 | NM_003417 | zinc finger protein 264 |
| 460 | 207753_at | ZNF304 | NM_020657 | zinc finger protein 304 |
| 461 | 209751_s_at | | AF291676 | MBP-1 interacting protein-2A |
| 462 | 217403_s_at | BC228680 | AC074331 | chromosome 19, BAC CIT-HSPC_204F22 |
| 463 | 219571_s_at | GIOT-3 | NM_016265 | GIOT-3 for gonadotropin inducible transcription repressor-3 |
| 464 | 203119_at | MGC2574 | NM_024098 | hypothetical protein MGC2574 |
| 465 | 203721_s_at | LOC51096 | NM_016001 | CGI-48 protein |
| 466 | 204880_at | MGMT | NM_002412 | O-6-methylguanine-DNA methyltransferase |
| 467 | 205664_at | KIN | NM_012311 | antigenic determinant of recA protein (mouse) homolog |
| 468 | 212597_s_at | | AL079310 | Novel human gene mapping to chromosome 22 |
| 469 | 212759_s_at | | AI703074 | transcription factor 7-like 2 (T-cell specific, HMG-box) |
| 470 | 213824_at | | AF221520 | protein kinase C binding protein 2 |
| 471 | 218137_s_at | FLJ13159 | NM_021940 | hypothetical protein FLJ13159 |

(continued)

| | Sequence identification | | | |
|---|---|---|---|---|
| SEQ ID NO: | psid | Gene Name | Accession # | Gene description |
| 472 | 220445_s_at | TRAG3 | NM_004909 | taxol resistance associated gene 3 |
| 473 | 203720_s_at | | NM_001983 | excision repair cross-complementing rodent repair deficiency, complementation group 1 |
| 474 | 201046_s_at | RAD23A | NM_005053 | RAD23 (S. cerevisiae) homolog A |
| 475 | 202344_at | HSF1 | NM_005526 | heat shock transcription factor 1 |
| 476 | 202580_x_at | FOXM1 | NM_021953 | forkhead box M1 |
| 477 | 205690_s_at | G10 | NM_003910 | maternal G10 transcript |
| 478 | 206307_s_at | FOXD1 | NM_004472 | forkhead box D1 |
| 479 | 213090_s_at | | AI744029 | TATA box binding protein (TBP)-associated factor |
| 480 | 218215_s_at | NR1 H2 | NM_007121 | nuclear receptor subfamily 1, group H, mem 2 |
| 481 | 207469_s_at | PIR | NM_003662 | Pirin |
| 482 | 209062_x_at | RAC3 | AF010227 | receptor-associated coactivator 3 |
| 483 | 200823_x_at | RPL29 | NM_000992 | ribosomal protein L29 |
| 484 | 202868_s_at | POP4 | NM_006627 | POP4 (processing of precursor, S. cerevisiae) homolog |
| 485 | 217747_s_at | RPS9 | NM_001013 | ribosomal protein S9 |
| 486 | 32723_at | | L02547 | (clone pZ50-19) cleavage stimulation factor 50kDa subunit |
| 487 | 212105_s_at | | BE910323 | DEADH (Asp-Glu-Ala-AspHis) box polypep 9 |
| 488 | 207320_x_at | | NM_004602 | staufen (Drosophila, RNA-binding protein) (STAU), transcript v. T4, |
| 489 | 200081_s_at | | BE741754 | ribosomal protein S6 |
| 490 | 217559_at | | A1001784 | Highly similar to A42735 ribosomal protein L10, cytosolic |
| 491 | 217907_at | HSPC071 | NM_014161 | HSPC071 protein |
| 492 | 213504_at | | W63732 | COP9 subunit 6 (MOV34 homolog, 34 kD) |
| 493 | 204102 s at | EEF2 | NM_001961 | eukaryotic translation elongation factor 2 |
| 494 | 208696_at | | AF275798 | PNAS-102 |
| 495 | 209275_s_at | CLN3 | AF015593 | CLN3 protein |
| 496 | 214591_at | | BF215673 | Drosophila Kelch like protein |
| 497 | 201642_at | IFNGR2 | NM_005534 | interferon gamma receptor 2 (interferon qamma transducer 1) |
| 498 | 219080_s_at | CTPS2 | NM_019857 | CTP synthase II |
| 499 | 202282_at | HADH2 | NM_004493 | hydroxyacyl-CoenzymeA dehydrogenase typell |
| 500 | 201453_x_at | RHEB2 | NM_005614 | Ras homolog enriched in brain 2 |
| 501 | 208733_at | | NM_002865 | RAB2, member RAS oncogene family |
| 502 | 211004_s_at | | BC002553 | Similar to aldehyde dehydrogenase 7 |

(continued)

| | | | Sequence identification | |
|---|---|---|---|---|
| SEQ ID NO: | psid | Gene Name | Accession # | Gene description |
| 503 | 201400_at | PSMB3 | NM_002795 | proteasome (prosome, macropain) sub β type3 |
| 504 | 208799_at | | BC004146 | proteasome (prosome, macropain) sub β type5 |
| 505 | 216088_s_at | | AL078633 | DNA sequence from clone RP5-1005F21 on chromosome 20 |
| 506 | 213912_at | KIAA0984 | AW134976 | KIAA0984 protein |
| 507 | 213913_s_at | KIAA0984 | AW134976 | KIAA0984 protein |
| 508 | 205356_at | USP13 | NM_003940 | ubiquitin spec protease 13 (isopeptidase T-3) |
| 509 | 208166_at | MMP16 | NM_022564 | matrix metalloproteinase 16 (membrane-inserted) transcript v. 2, |
| 510 | 202018_s_at | LTF | NM_002343 | lactotransferrin |
| 511 | 213829_x_at | FLJ20478 | AK000485 | FLJ20478 |
| 512 | 212766_s_at | | AW294587 | Similar to hypothetical protein FLJ12484, |
| 513 | 202969_at | | Y09216 | dual-specificity tyrosine-(Y)-phosphorylation regulated kinase 2 |
| 514 | 203218_at | | W37431 | mitogen-activated protein kinase 9 |
| 515 | 204171_at | RPS6KB1 | NM_003161 | ribosomal protein S6 kinase, |
| 516 | 204906_at | | BC002363 | ribosomal protein S6 kinase, 90kD, polypep 2, |
| 517 | 205126_at | VRK2 | NM_006296 | vaccinia related kinase 2 |
| 518 | 214716_at | DKFZp434P0116 | AW504018 | hypothetical protein DKFZp434P0116 |
| 519 | 37170_at | HRIHFB2017 | AB015331 | HRIHFB2017 |
| 520 | 211474_s_at | | BC004948 | MGC:10846, |
| 521 | 204237_at | CED-6 | NM_016315 | CED-6 protein |
| 522 | 203005_at | LTBR | NM_002342 | lymphotoxin β receptor (TNFR superfamily, member 3 |
| 523 | 211000_s_at | gp130-RAPS | AB015706 | gp130 of the rheumatoid arthritis antigenic peptide-bearing soluble form |
| 524 | 202679_at | NPC1 | NM_000271 | Niemann-Pick disease, type C1 |
| 525 | 205282_at | LRP8 | NM_004631 | low density lipoprotein receptor-related protein 8, apolipoprotein e receptor |
| 526 | 207270_x_at | CMRF35 | NM_006678 | CMRF35 leukocyte immunoglobulin-like receptor |
| 527 | 211846_s_at | | AF110314 | herpesvirus immunoglobulin-like receptor HIgR |
| 528 | 201810_s_at | | AL562152 | SH3-domain binding protein 5 (BTK-assoc) |
| 529 | 213684_s_at | | BF671400 | LIM protein (similar to rat protein kinase C-binding enigma) |
| 530 | 206636_at | RASA2 | NM_006506 | RAS p21 protein activator 2 |
| 531 | 219907_at | SNT-2 | NM_006653 | suc1-associated neurotrophic factor target 2 (FGFR signalling adaptor) |

(continued)

| Sequence identification | | | | |
|---|---|---|---|---|
| SEQ ID NO: | psid | Gene Name | Accession # | Gene description |
| 532 | 201281_at | GP110 | NM_007002 | cell membrane glycoprotein, 110000M(r) (surface antigen) |
| 533 | 209462_at | | U48437 | amyloid precursor-like protein 1 |
| 534 | 210880_s_at | | AB001467 | Efs2, |
| 535 | 212567_s_at | | AL523310 | CS0DC001YN06 (3 prime) |
| 536 | 202222_s_at | DES | NM_001927 | desmin |
| 537 | 219615_s_at | KCNK5 | NM_003740 | potassium channel, subfamily K, mem 5 (TASK-2) |
| 538 | 214210_at | | AL049764 | clone RP3-362J20 on chrom 22q13.1-13.31 |
| 539 | 52078_at | | AI828080 | |
| 540 | 203765_at | GCL | NM_012198 | grancalcin |
| 541 | 213308_at | | AB028945 | cortactin SH3 domain-binding protein |
| 542 | 203082_at | KIAA0187 | NM_014753 | KIAA0187 gene product |
| 543 | 212543_at | AIM1 | U83115 | non-lens $\beta$ gamma-crystallin like protein |
| 544 | 209709_at | RHAMM | U29343 | hyaluronan receptor |
| 545 | 205807_s_at | TUFT1 | NM_020127 | tuftelin 1 |
| 546 | 213895_at | | BF445047 | epithelial membrane protein 1 |
| 547 | 201565_s_at | ID2 | NM_002166 | inhibitor of DNA binding 2, dominant negative helix-loop-helix protein |
| 548 | 202639_s_at | | AI689052 | RAN binding protein 3 |
| 549 | 209230_s_at | COM1 | AF135266 | p8 protein homolog |
| 550 | 218626_at | 4E-T | NM_019843 | eIF4E-transporter |
| 551 | 221506_s_at | | BG258639 | karyopherin beta 2b, transportin |
| 552 | 221521_s_at | | BC003186 | HSPC037 protein, clone MGC:673, |
| 553 | 202069_s_at | | AI826060 | isocitrate dehydrogenase 3 (NAD+) $\alpha$ |
| 554 | 203524_s_at | MPST | NM_021126 | mercaptopyruvate sulfurtransferase |
| 555 | 201916_s_at | SEC63L | NM_007214 | SEC63, endoplasmic reticulum translocon component (S. cerevisiae) like |
| 556 | 217776_at | ARSDR1 | AF167438 | androgen-regulated short-chain dehydrogenasereductase 1 |
| 557 | 218623_at | LOC51617 | NM_015980 | HMP19 protein |
| 558 | 208777_s_at | | AF001212 | 26S proteasome subunit 9 |
| 559 | 203971_at | SLC31A1 | NM_001859 | solute carrier family 31 (copper transporters), member 1 |
| 560 | 215243_s_at | | AF099730 | connexin 31 (GJB3) gene, |
| 561 | 201591_s_at | | NM_007184 | imidazoline receptor candidate (I-1), |
| 562 | 205466_s_at | HS3ST1 | NM_005114 | heparan sulfate (glucosamine) 3-O-sulfotransferase 1 |

(continued)

| Sequence identification | | | | |
|---|---|---|---|---|
| SEQ ID NO: | psid | Gene Name | Accession # | Gene description |
| 563 | 207829_s_at | BNIP1 | NM_013978 | Bcl-2 adenovirus E1B 19kD-interacting protein 1 transcript variant BNIP1-a, |
| 564 | 205742_at | TNNI3 | NM_000363 | troponin I, cardiac |
| 565 | 211178_s_at | | AF038602 | CD2 binding protein 1 short form |
| 566 | 219113_x_at | LOC51171 | NM_016246 | retinal short-chain dehydrogenasereductase retSDR3 |
| 567 | 217807_s_at | GLTSCR2 | NM_015710 | glioma tumor suppressor candidate region gene 2 |
| 568 | 206133_at | HSXIAPAF1 | NM_017523 | XIAP associated factor-1 |
| 569 | 201379_s_at | TPD52L2 | N_003288 | tumor protein D52-like 2 |
| 570 | 209373_at | | BC003179 | MGC:4419, |
| 571 | 210142_x_at | | AF117234 | flotillin |
| 572 | 218942_at | FLJ22055 | NM_024779 | hypothetical protein FLJ22055 |
| 573 | 216716_at | | U15197 | histo-blood group ABO protein, partial 3 UTR sequence. |
| 574 | 218185_s_at | FLJ10511 | NM_018120 | hypothetical protein FLJ10511 |
| 575 | 219215_s_at | FLJ20327 | NM_017767 | hypothetical protein FLJ20327 |
| 576 | 214683_s_at | | AI251890 | CDC-like kinase1 |
| 577 | 202612_s_at | CRSP2 | NM_004229 | cofactor required for Sp1 transcriptional activation, subunit 2 (150kD) |
| 578 | 208932_at | | BC001416 | protein phosphatase 4 (formerly X), catalytic subunit, |
| 579 | 206766_at | ITGA10 | AF112345 | integrin $\alpha$ 10 subunit |
| 580 | 216213_at | | AF155113 | NY-REN-55 antigen |
| 581 | 205609_at | ANGPT1 | NM_001146 | angiopoietin 1 |
| 582 | 204644_at | tNOX | AF207881 | tumor-assoc hydroquinone (NADH) oxidase |
| 583 | 206812_at | ADRB3 | NM_000025 | adrenergic, $\beta$-3-, receptor |
| 584 | 202176_at | ERCC3 | NM_000122 | excision repair cross-complementing rodent repair deficiency, complementation group 3 (xeroderma pigmentosum group B complementing) |
| 585 | 219816_s_at | FLJ10482 | NM_018107 | hypothetical protein FLJ10482 |
| 586 | 200934_at | DEK | NM_003472 | DEK oncogene (DNA binding) |
| 587 | 206098_at | ZID | NM_006626 | zinc finger protein with interaction domain |
| 588 | 206987_x_at | FGF18 | NM_003862 | fibroblast growth factor 18 |
| 589 | 208392_x_at | IFI75 | NM_004510 | interferon-induced protein 75, 52kD |
| 590 | 213048_s_at | | W26593 | SET translocation (myeloid leukemia-assoc) |
| 591 | 220861_at | | AF118067 | PRO1578 |
| 592 | 222115_x_at | | BC003693 | Similar to RIKEN cDNA 3930401 K13 gene, |
| 593 | 222236_s_at | FLJ20246 | AK000253 | FLJ20246 |

(continued)

| Sequence identification | | | | |
|---|---|---|---|---|
| SEQ ID NO: | psid | Gene Name | Accession # | Gene description |
| 594 | 41644_at | KIAA0790 | AB018333 | KIAA0790 protein, |
| 595 | 204107_at | | NM_002505 | nuclear transcription factor Y, α |
| 596 | 209706_at | | AF247704 | homeobox protein NKX3.1 |
| 597 | 210174_at | | AF228413 | hepatocyte transcription factor |
| 598 | 213844_at | HOXA5 | NM_019102 | homeo box A5 |
| 599 | 214108_at | | AI346181 | MAX protein |
| 600 | 212064_x_at | | AI471665 | MYC-associated zinc finger protein (purine-binding transcription factor) |
| 601 | 214316_x_at | | AI348935 | calreticulin |
| 602 | 201514_s_at | G3BP | NM_005754 | Ras-GTPase-activating protein SH3-domain-binding protein |
| 603 | 203092_at | hTIM44 | AF026030 | putative mitochondrial inner membrane protein import receptor |
| 604 | 208725_at | | AL031668 | DNA sequence from clone RP1-64K7 on chromosome 20q11.21-11.23. |
| 605 | 219819_s_at | HSPC007 | NM_014018 | HSPC007 protein |
| 606 | 200724 at | | BC003358 | ribosomal protein L10, |
| 607 | 214350_at | | AI762021 | Syntrophin, beta 2 (dystrophin-associated protein A1, 59kD, basic component 2) |
| 608 | 210197_at | | BC003622 | Similar to inositol 1,3,4-triphosphate 56 kinase, |
| 609 | 218150_at | ARL5 | NM_012097 | ADP-ribosylation factor-like 5 |
| 610 | 214241_at | | AA723057 | NADH dehydrogenase (ubiquinone) 1 beta subcomplex, 8 (19kD, ASHI) |
| 611 | 206253_at | DLG2 | NM_001364 | discs large (Dros) homolog 2 (chapsyn-110) |
| 612 | 205435_s_at | KIAA1048 | NM_014911 | KIAA1048 protein |
| 613 | 215154_at | DKFZp434G043 | AL080134 | DKFZp434G043 |
| 614 | 215771_x_at | | X15786 | ret-II gene. |
| 615 | 213795_s_at | | BF740139 | clone RP4-534B8 chromosome 20 |
| 616 | 209042_s_at | | BC001738 | Similar to ubiquitin-conjugating enzyme E2G 2 (homologous to yeast UBC7), |
| 617 | 201602_s_at | | NM_002480 | myosin phosphatase, target subunit 1 |
| 618 | 211133_x_at | | AF009643 | clone 6 immunoglobulin-like transcript 5 protein |
| 619 | 211245_x_at | | AF002256 | killer cell inhibitory receptor homolog cl-9 |
| 620 | 210713_at | | U61166 | SH3 domain-containing protein SH3P17 |
| 621 | 210840_s_at | KIAA0051 | D29640 | KIAA0051 |
| 622 | 218157_x_at | SPEC1 | NM_020239 | small protein effector 1 of Cdc42 |
| 623 | 208292_at | BMP10 | NM_014482 | bone morphogenetic protein 10 |

(continued)

| Sequence identification | | | | |
|---|---|---|---|---|
| SEQ ID NO: | psid | Gene Name | Accession # | Gene description |
| 624 | 210518 at | | AB035305 | CDH8 mRNA for cadherin-8, |
| 625 | 205064_at | SPRR1B | NM_003125 | small proline-rich protein 1 B (cornifin) |
| 626 | 206990_at | TNR | NM_003285 | tenascin R (restrictin, janusin) |
| 627 | 211304 x_at | | D50134 | inward rectifying K channel, |
| 628 | 203950_s_at | CIC-6a | NM_001286 | chloride channel 6 (CLCN6), transcript variant |
| 629 | 214211_at | | AA083483 | ferritin, heavy polypeptide 1 |
| 630 | 211056_s_at | | BC006373 | clone MGC: 12762 |
| 631 | 212185_x_at | MT2A | NM_005953 | metallothionein 2A |
| 632 | 203415_at | PDCD6 | NM_013232 | programmed cell death 6 |
| 633 | 205388_at | TNNC2 | NM_003279 | troponin C2, fast |
| 634 | 208871 at | | D31840 | DRPLA |
| 635 | 218840_s_at | FLJ10631 | NM_018161 | hypothetical protein FLJ10631 |
| 636 | 206400_at | LGALS7 | NM_002307 | lectin galactoside-binding soluble 7 (galectin 7) |
| 637 | 207733_x_at | PSG9 | NM_002784 | pregnancy specific beta-1-glycoprotein 9 |
| 638 | 207850_at | GRO3 | NM 002090 | GRO3 oncogene |
| 639 | 210884_s_at | | AF168619 | HE2 gamma1 |
| 640 | 220162_s_at | LOC64170 | NM_022352 | caspase recruitment domain protein 9 |
| 641 | 209749_s_at | | AI623989 | angiotensin I converting enzyme (peptidyl-dipeptidase A) 1 |
| 642 | 216010_x_at | | D89324 | DNA for alpha (1,31,4) fucosyltransferase, |
| 643 | 203970_s_at | PEX3 | NM_003630 | peroxisomal biogenesis factor 3 |
| 644 | 203972_s_at | | AB035307 | mRNA for Pex3p, |
| 645 | 217749 at | LOC51137 | NM_016128 | coat protein gamma-cop |
| 646 | 207022_s_at | LDHC1 | NM_002301 | lactate dehydrogenase C, transcript variant 1 |
| 647 | 209905_at | | AI246769 | homeo box A9 |
| 648 | 209099_x_at | HJ1 | U73936 | Jagged 1 |
| 649 | 222201_s_at | KIAA1315 | AB037736 | KIAA1315 protein, |
| 650 | 201335_s_at | KIAA0382 | NM_015313 | KIAA0382 protein; |
| 651 | 221748_s_at | tensin | AL046979 | tensin |
| 652 | 205139_s_at | UST | NM_005715 | uronyl 2-sulfotransferase |
| 653 | 210678_s_at | | U56418 | lysophosphatidic acid acyltransferase-beta |
| 654 | 215293_s_at | DKFZp564E053 | AL049261 | DKFZp564E053 |
| 655 | 41858_at | DKFZp564E053 | AL049261 | DKFZp564E053 |
| 656 | 218047_at | FLJ12492 | NM_024586 | hypothetical protein FLJ12492 |
| 657 | 215069_at | FLJ21412 | AK025065 | FLJ21412 |
| 658 | 201095_at | DAP | NM_004394 | death-associated protein |

(continued)

| Sequence identification | | | | |
|---|---|---|---|---|
| SEQ ID NO: | psid | Gene Name | Accession # | Gene description |
| 659 | 220197_at | ATP6N1B | NM_020632 | ATPase, H(+)-transporting, lysosomal, noncatalytic accessory protein 1 B |
| 660 | 213351_s_at | KIAA0779 | AI934469 | KIAA0779 |
| 661 | 203285_s_at | HS2ST1 | NM_012262 | heparan sulfate 2-O-sulfotransferase |
| 662 | 204326_x_at | MT1L | NM_002450 | metallothionein 1L |
| 663 | 208581_x_at | MT1X | NM_005952 | metallothionein 1X |
| 650 | 212690_at | KIAA0725 | AB018268 | KIAA0725 protein, |

References cited

| | | | | |
|---|---|---|---|---|
| 5,242,974 | 20030069180 | WO01053312 | WO02094988 | WO04030615 |
| 5,384,261 | 20030073623 | WO01054472 | WO02095010 | WO04031413 |
| 5,405,783 | 20030087818 | WO01055322 | WO02102235 | WO04031414 |
| 5,412,087 | 20030096982 | WO01057182 | WO02102993 | WO04033637 |
| 5,424,186 | 20030104366 | WO01057188 | WO02102994 | WO04037996 |
| 5,429,807 | 20030124128 | WO01059063 | WO03000735 | WO04039943 |
| 5,436,327 | 20030124148 | WO01060860 | WO03003906 | WO04039956 |
| 5,445,934 | 20030128884 | WO01070979 | WO03004622 | WO04041170 |
| 5,472,672 | 20030166064 | WO01073027 | WO03004989 | WO04042022 |
| 5,527,681 | 20030194704 | WO01075067 | WO03010336 | WO04043361 |
| 5,529,756 | 20030219744 | WO01077168 | WO03016475 | WO04047728 |
| 5,532,128 | 20030219767 | WO01077288 | WO03016476 | WO04048938 |
| 5,545,531 | 20030236392 | WO01077291 | WO03016500 | WO04053081 |
| 5,554,501 | 20040005560 | WO01088088 | WO03018807 | WO04060270 |
| 5,556,752 | 20040009478 | WO01090154 | WO03025138 | WO04063355 |
| 5,561,071 | 20040009491 | WO01090304 | WO03027258 | WO04070062 |
| 5,571,639 | 20040013663 | WO01094629 | WO03038063 | WO04072265 |
| 5,593,839 | 20040034196 | WO02002623 | WO03042661 | WO04074301 |
| 5,599,695 | 20040037842 | WO02004514 | WO03042661 | WO04076613 |
| 5,624,711 | 20040058340 | WO02012235 | WO03042661 | WO04079014 |
| 5,658,734 | 20040110194 | WO02012328 | WO03046152 | WO91001999 |
| 5,700,637 | 20040115636 | WO02024956 | WO03054152 | WO92019745 |
| 5874283 | DE 432977 | WO02028999 | WO03062379 | WO94023039 |
| 6,004,755 | EP 1104808 | WO02029086 | WO03064589 | WO95014772 |
| 6060283 | WO00021991 | WO02031198 | WO03072035 | WO97012967 |
| 6,136,182 | WO00026244 | WO02036766 | WO03078572 | WO97024441 |
| 6171787 | WO00032776 | WO02050279 | WO03081201 | WO97033902 |
| 6,218,114 | WO00036107 | WO02057414 | WO03091388 | WO98010067 |
| 6,218,122 | WO00055320 | WO02059260 | WO03094848 | WO98045437 |
| 6,271,002 | WO00055350 | WO02059271 | WO03101283 | WO99026976 |
| 6440694 | WO00056880 | WO02059377 | WO04003162 | W990033980 |
| 200200131971 | WO00058473 | WO02060317 | WO04018641 | WO99033981 |
| 20020055474 | WO00060078 | WO02068652 | WO04020497 | WO99035158 |
| 20020064872 | WO00061610 | WO02070737 | WO04020593 | WO99035170 |

(continued)

| | | | | |
|---|---|---|---|---|
| 20030065157 | WO00063438 | WO02074237 | WO04022059 | WO99038972 |
| 20020072089 | WO00078961 | WO02076488 | WO04023973 | WO99047540 |
| 20020081659 | WO01000828 | WO02081498 | WO04024097 | WO99050416 |
| 20020110547 | WO01025256 | WO02081517 | WO04024892 | WO99054460 |
| 20020123619 | WO01027158 | WO02081731 | WO04028479 | WO99058642 |
| 20020150581 | WO01040466 | WO02083876 | | WO99064576 |
| 20020151681 | WO01042451 | WO02083898 | | |
| 20020168637 | WO01042467 | WO02086443 | | |
| 20020192678 | WO01044448 | WO02090526 | | |
| 20030194734 | WO01046697 | WO02094629 | | |
| 20030022239 | WO01049716 | | | |

Ahr et al. (2002) "Identification of high risk breast-cancer patients by gene-expression profiling" Lancet 359:131-132

Chang et al. (2003) "Gene expression profiling for the prediction of therapeutic response to docetaxel in patients with breast cancer" Lancet 362:362-9

Early Breast Cancer Trialists' Collaborative Group (1995) "Effects of radiotherapy and surgery in early breast cancer. An overview of the randomized trials" N Engl J Med 333:1444-1455

Early Breast Cancer Trialists' Collaborative Group (1998a) "Polychemotherapy for early breast cancer: an overview of the randomized trials" Lancet 352:930-942

Early Breast Cancer Trialists' Collaborative Group (1998b) "Tamoxifen for early breast cancer: an overview of randomized trials" Lancet 351:1451-1467

Efron (1981) "Censored data and the bootstrap" J Am Stat Assoc 76:312-319

Eifel et al. (2001) "National Institutes of Health Consensus Development Conference Statement: adjuvant therapy for breast cancer, November 1-3, 2000" J Natl Cancer Inst 93:979-989

Foekens et al. (1989b) "Prognostic value of estrogen and progesterone receptors measured by enzyme immunoassays in human breast tumor cytosols" Cancer Res 49:5823-5828

Foekens et al. (1989a) "Prognostic value of receptors for insulin-like growth factor 1, somatostatin, and epidermal growth factor in human breast cancer" Cancer Res 49:7002-7009

Goldhirsch et al. (2003) "Meeting highlights: Updated International Expert Consensus on the Primary Therapy of Early Breast Cancer" J Clin Oncol 21:3357-3365

Golub et al. (1999) "Molecular classification of cancer: class discovery and class prediction by gene expression monitoring" Science 286:531-537

Gruvberger et al. (2001) "Estrogen receptor status in breast cancer is associated with remarkably distinct gene expression patterns" Cancer Res 61:5979-5984

Hedenfalk et al. (2001) "Gene-expression profiles in hereditary breast cancer" N Engl J Med 344:539-548

Herrera-Gayol et al. (1999) "Adhesion proteins in the biology of breast cancer: contribution of CD44" Exp Mol Pathol 66:149-156

Huang et al. (2003) "Gene expression predictors of breast cancer outcomes" Lancet 361:1590-1596

Kaplan et al. (1958) "Non-parametric estimation of incomplete observations" J Am Stat Assoc 53:457-481

Keyomarsi et al. (2002) "Cyclin E and survival in patients with breast cancer" N Engl J Med 347:1566-1575

Lipshutz et al. (1999) "High density synthetic oligonucleotide arrays" Nat Genet 21:20-24

Ma et al. (2003) "Gene expression profiles of human breast cancer progression" Proc Natl Acad Sci USA 100:5974-5979

Ntzani et al. (2003) "Predictive ability of DNA microarrays for cancer outcomes and correlates: an empirical assessment" Lancet 362:1439-1444

Perou et al. (2000) "Molecular portraits of human breast tumors" Nature 406:747-752

Ramaswamy et al. (2001) "Multiclass cancer diagnosis using tumor gene expression signatures" Proc Natl Acad Sci USA 98:15149-15154

Ramaswamy et al. (2003) "A molecular signature of metastasis in primary solid tumors" Nat Genet 33:1-6

Ransohoff (2004) "Rules of evidence for cancer molecular-marker discovery and validation" Nat Rev Cancer 4:309-314

Sørlie et al. (2001) "Gene expression patterns of breast carcinomas distinguish tumor subclasses with clinical implications" Proc Natl Acad Sci USA 98:10869-10874

Sørlie et al. (2003) "Repeated observation of breast tumor subtypes in independent gene expression data sets" Proc Natl Acad Sci USA 100:8418-8423

Sotiriou et al. (2003) "Gene expression profiles derived from fine needle aspiration correlate with response to systemic chemotherapy in breast cancer" Breast Cancer Res 4:R3

Sotiriou et al. (2003) "Breast cancer classification and prognosis based on gene expression profiles from a population-based study" Proc Natl Acad Sci USA 100:10393-10398

Su et al. (2001) "Molecular classification of human carcinomas by use of gene expression signatures" Cancer Res 61:7388-7393

van de Vijver et al. (2002) "A gene expression signature as a predictor of survival in breast cancer" N Engl J Med 347:1999-2009

van't Veer et al. (2002) "Gene expression profiling predicts clinical outcome of breast cancer" Nature 415:530-536

Wang et al. (2004) "Gene expression profiles and molecular markers to predict recurrence of Dukes' B colon cancer" J Clin Oncol 22:1564-1571

Woelfle et al. (2003) "Molecular signature associated with bone marrow micrometastasis in human breast cancer" Cancer Res 63:5679-5684

## Claims

1. An *in vitro* method of assessing breast cancer status comprising the steps of measuring in a biological sample from a breast cancer patient the expression levels of genes consisting of those encoding mRNA identified by all of SEQ ID NOs: 1-111 in Table 10; wherein the gene expression levels above or below pre-determined cut-off levels are indicative of breast cancer status.

2. An *in vitro* method of determining breast cancer patient treatment protocol comprising the steps of measuring in a biological sample from a breast cancer patient the expression levels of genes consisting of those encoding mRNA identified by all of SEQ ID NOs : 1-111 in Table 10; wherein the gene expression levels above or below pre-determined cut-off levels are sufficiently indicative of risk of recurrence to enable a physician to determine the degree and type of therapy recommended to prevent recurrence.

3. Adjuvant therapy for use in treating a breast cancer patient at high risk of recurrence of breast cancer, wherein the patient is identified by measuring in a biological sample from a breast cancer patient the expression levels of genes consisting of those encoding mRNA identified by all of SEQ ID NOs: 1-111 in Table 10; wherein the gene expression levels above or below pre-determined cut-off levels indicate a high risk of recurrence.

4. The method of claims 1 or 2, or the adjuvant therapy of claim 3 wherein the sample is prepared by a method selected from the group consisting of bulk tissue preparation and laser capture microdissection.

5. The method or the adjuvant therapy of claim 4 wherein the bulk tissue preparation is obtained from a biopsy or a surgical specimen.

6. The method of claim 1 or 2, or the adjuvant therapy of claim 3 further comprising measuring the expression level of at least one gene encoding mRNA identified by SEQ ID NOs: 112-132 in Table 10.

7. The method of claim 1 or 2, or the adjuvant therapy of claim 3 further comprising measuring the expression level of at least one gene constitutively expressed in the sample.

8. The method of claim 1 or 2, or the adjuvant therapy of claim 3 further comprising determining the estrogen receptor (ER) status of the sample.

9. The method or the adjuvant therapy of claim 8 wherein the ER status is determined by measuring the expression level of at least one gene indicative of ER status.

10. The method or the adjuvant therapy of claim 8 wherein the ER status is determined by measuring the presence of ER in the sample.

11. The method or the adjuvant therapy of claim 10 wherein the presence of ER is measured immunohistochemically.

12. The method of claim 1 or 2, or the adjuvant therapy of claim 3 wherein the sample is obtained from a primary tumor.

**13.** The method of claim 1 or 2, or the adjuvant therapy of claim 3 wherein the specificity is at least about 40%.

**14.** The method of claim 1 or 2, or the adjuvant therapy of claim 3 wherein the sensitivity is at least at least about 90%.

**15.** The method of claim 1 or 2, or the adjuvant therapy of claim 3 wherein the expression pattern of the genes is compared to an expression pattern indicative of a relapse patient.

**16.** The method or the adjuvant therapy of claim 15 wherein the comparison of expression patterns is conducted with pattern recognition methods.

**17.** The method or the adjuvant therapy of claim 16 wherein the pattern recognition methods include the use of a Cox's proportional hazards analysis.

**18.** The method of claim 1 or 2, or the adjuvant therapy of claim 3 wherein the pre-determined cut-off levels are at least 1.5-fold over-or under-expression in the sample relative to benign cells or normal tissue.

**19.** The method of claim 1 or 2, or the adjuvant therapy of claim 3 wherein the pre-determined cut-off levels have at least a statistically significant p-value over-expression in the sample having metastatic cells relative to benign cells or normal tissue.

**20.** The method or the adjuvant therapy of claim 19 wherein the p-value is less than 0.05.

**21.** The method of claim 1 or 2, or the adjuvant therapy of claim 3 wherein gene expression is measured on a microarray or gene chip.

**22.** The method or the adjuvant therapy of claim 21 wherein the microarray is a cDNA array or an oligonucleotide array.

**23.** The method or the adjuvant therapy of claim 21 wherein the microarray or gene chip further comprises one or more internal control reagents.

**24.** The method of claim 1 or 2, or the adjuvant therapy of claim 3 wherein gene expression is determined by nucleic acid amplification conducted by polymerase chain reaction (PCR) of RNA extracted from the sample.

**25.** The method or the adjuvant therapy of claim 24 wherein said PCR is reverse transcription polymerase chain reaction (RT-PCR).

**26.** The method or the adjuvant therapy of claim 25, wherein the RT-PCR further comprises one or more internal control reagents.

**27.** The method of claim 1 or 2, or the adjuvant therapy of claim 3 wherein gene expression is detected by measuring or detecting a protein encoded by the gene.

**28.** The method or the adjuvant therapy of claim 27 wherein the protein is detected by an antibody specific to the protein.

**29.** The method of claim 1 or 2, or the adjuvant therapy of claim 3 wherein gene expression is detected by measuring a characteristic of the gene.

**30.** The method or the adjuvant therapy of claim 29 wherein the characteristic measured is selected from the group consisting of DNA amplification, methylation, mutation and allelic variation.

**31.** A method of generating a Relapse Hazard Score to enable prognosis of breast cancer patients comprising the steps of: a. obtaining gene expression data from a statistically significant number of patient biological samples; b. applying univariate Cox's regression analysis to the data to obtain selected genes; c. applying weighted expression levels to the selected genes with standard Cox's coefficients to obtain a prediction model that can be applied as a relapse Hazard Score, wherein the Relapse Hazard Score is obtained by the formula:

$$\text{Relapse Score} = A \cdot I + \sum_{i=1}^{66} I \cdot w_i x_i + B \cdot (1 - I) + \sum_{j=1}^{10} (1 - I) \cdot w_j x_j$$

where

$$I = \begin{cases} 1 & \text{if X gene level} > 10 \text{ fmol per mg protein} \\ 0 & \text{if X gene level} \leq 10 \text{ fmol per mg protein} \end{cases}$$

A and B are constants
$w_i$ is the standardized Cox's regression coefficient for X gene + marker
$x_i$ is the expression value of ER + marker in log2 scale
$w_j$ is the standardized Cox's regression coefficient for X gene - marker
$x_j$ is the expression value of ER - marker in log2 scale
X gene is selected from the group consisting of those encoding mRNA :

i. corresponding to SEQ ID NOs 1-111

32. A method of generating a breast cancer prognostic patient report comprising the steps of :

a. obtaining a biological sample from the patient;
b. measuring gene expression of the sample;
c. applying a Relapse Hazard Score to the results of step b.; and
d. using the results obtained in step c. to generate the report;

wherein the Relapse Hazard Score is obtained by the formula:

$$\text{Relapse Score} = A \cdot I + \sum_{i=1}^{66} I \cdot w_i x_i + B \cdot (1 - I) + \sum_{j=1}^{10} (1 - I) \cdot w_j x_j$$

where

$$I = \begin{cases} 1 & \text{if X gene level} > 10 \text{ fmol per mg protein} \\ 0 & \text{if X gene level} \leq 10 \text{ fmol per mg protein} \end{cases}$$

A and B are constants
$w_i$ is the standardized Cox's regression coefficient for X gene + marker
$x_i$ is the expression value of ER + marker in log2 scale
$w_j$ is the standardized Cox's regression coefficient for X gene - marker
$x_j$ is the expression value of ER - marker in log2 scale
X gene is selected from the group consisting of those encoding mRNA :

i. corresponding to SEQ ID NOs 1 -111.

33. The method of claim 32 wherein the report contains an assessment of patient outcome and/or probability of risk relative to the patient population.

34. A composition comprising a probe set consisting of all of SEQ ID NOs: 1-111 in Table 10.

35. The use of a kit in a method for conducting an assay to determine breast cancer prognosis in a biological sample comprising: materials for detecting isolated nucleic acid sequences, their complements, or portions thereof of a combination of genes consisting of those encoding mRNA identified by all of SEQ ID NOs: 1-111 in Table 10.

**36.** The use of claim 35 further comprising reagents for conducting a microarray analysis.

**37.** The use of claim 35 further comprising a medium through which said nucleic acid sequences, their complements, or portions thereof are assayed.

**38.** The use of a microarray or gene chip in the method of any one of claims 1 or 2, or the adjuvant therapy of claim 3.

**39.** The use of claim 38 comprising isolated nucleic acid sequences, their complements, or portions thereof of a combination of genes consisting of those encoding mRNA identified by SEQ ID NOs: 1-111 in Table 10; where the combination is sufficient to characterize breast cancer status or risk of relapse in a biological sample.

**40.** The use of claim 38 wherein the measurement or characterization is at least 1.5-fold over-or under-expression.

**41.** The use of claim 38 wherein the measurement provides a statistically significant p-value over-or under-expression.

**42.** The use of claim 38 wherein the p-value is less than 0.05.

**43.** The use of claim 38, wherein the microarray comprises a cDNA array or an oligonucleotide array.

**44.** The use of claim 38, wherein the microarray further comprises or more internal control reagents.


**Patentansprüche**

**1.** In-vitro-Verfahren zur Beurteilung des Brustkrebs-Status, wobei man in den Verfahrensschritten in einer biologischen Probe von einem Brustkrebspatienten die Expressionsniveaus von Genen misst, die aus denen bestehen, die für durch alle der SEQ ID NO: 1-111 in Tabelle 10 identifizierte mRNA codieren; wobei die Genexpressionsniveaus oberhalb bzw. unterhalb vorbestimmter Ausschlussgrenzniveaus den Brustkrebs-Status anzeigen.

**2.** In-vitro-Verfahren zur Feststellung eines Brustkrebspatientenbehandlungsprotokolls, wobei man in den Verfahrensschritten in einer biologischen Probe von einem Brustkrebspatienten die Expressionsniveaus von Genen misst, die aus denen bestehen, die für durch alle der SEQ ID NO: 1-111 in Tabelle 10 identifizierte mRNA codieren; wobei die Genexpressionsniveaus oberhalb bzw. unterhalb vorbestimmter Ausschlussgrenzniveaus das Risiko eines Wiederauftretens hinreichend anzeigen, so dass ein Arzt den zur Vorbeugung eines Wiederauftretens empfohlenen Grad und Typ der Therapie bestimmen kann.

**3.** Adjuvante Therapie zur Anwendung bei der Behandlung eines Brustkrebspatienten mit hohem Risiko eines Wiederauftretens von Brustkrebs, wobei der Patient dadurch identifiziert wird, dass man in einer biologischen Probe von einem Brustkrebspatienten die Expressionsniveaus von Genen misst, die aus denen bestehen, die für durch alle der SEQ ID NO: 1-111 in Tabelle 10 identifizierte mRNA codieren; wobei die Genexpressionsniveaus oberhalb bzw. unterhalb vorbestimmter Ausschlussgrenzniveaus ein hohes Risiko eines Wiederauftretens anzeigen.

**4.** Verfahren nach Anspruch 1 oder 2 oder adjuvante Therapie nach Anspruch 3, wobei die Probe mit einem aus der aus großformatiger Gewebepräparation und Laser-Mikrodissektion bestehenden Gruppe ausgewählten Verfahren hergestellt wird.

**5.** Verfahren oder adjuvante Therapie nach Anspruch 4, wobei die großformatige Gewebepräparation aus einer Biopsie oder einem chirurgischen Präparat erhalten wird.

**6.** Verfahren nach Anspruch 1 oder 2 oder adjuvante Therapie nach Anspruch 3, wobei man ferner das Expressionsniveau wenigstens eines für durch SEQ ID NO: 112-132 in Tabelle 10 identifizierte mRNA codierenden Gens misst.

**7.** Verfahren nach Anspruch 1 oder 2 oder adjuvante Therapie nach Anspruch 3, wobei man ferner das Expressionsniveau wenigstens eines in der Probe konstitutiv exprimierten Gens misst.

**8.** Verfahren nach Anspruch 1 oder 2 oder adjuvante Therapie nach Anspruch 3, wobei man ferner den Östrogenrezeptor(ER)-Status der Probe bestimmt.

9. Verfahren oder adjuvante Therapie nach Anspruch 8, wobei der ER-Status bestimmt wird, indem man das Expressionsniveau wenigstens eines den ER-Status anzeigenden Gens misst.

10. Verfahren oder adjuvante Therapie nach Anspruch 8, wobei der ER-Status bestimmt wird, indem man das Vorliegen von ER in der Probe misst.

11. Verfahren oder adjuvante Therapie nach Anspruch 10, wobei das Vorliegen von ER immunhistochemisch gemessen wird.

12. Verfahren nach Anspruch 1 oder 2 oder adjuvante Therapie nach Anspruch 3, wobei die Probe aus einem Primärtumor gewonnen wird.

13. Verfahren nach Anspruch 1 oder 2 oder adjuvante Therapie nach Anspruch 3, wobei die Spezifität wenigstens etwa 40% beträgt.

14. Verfahren nach Anspruch 1 oder 2 oder adjuvante Therapie nach Anspruch 3, wobei die Empfindlichkeit wenigstens wenigstens etwa 90% beträgt.

15. Verfahren nach Anspruch 1 oder 2 oder adjuvante Therapie nach Anspruch 3, wobei das Expressionsmuster der Gene mit einem einen Rezidivpatienten kennzeichnenden Expressionsmuster verglichen wird.

16. Verfahren oder adjuvante Therapie nach Anspruch 15, wobei der Vergleich von Expressionsmustern mit Mustererkennungsmethoden durchgeführt wird.

17. Verfahren oder adjuvante Therapie nach Anspruch 16, wobei die Mustererkennungsmethoden die Verwendung einer Cox-Regressionsanalyse (Cox's Proportional Hazards Analysis) umfassen.

18. Verfahren nach Anspruch 1 oder 2 oder adjuvante Therapie nach Anspruch 3, wobei es sich bei den vorbestimmten Ausschlussgrenzniveaus um wenigstens 1,5-fache Über- bzw. Unterexpression in der Probe relativ zu gutartigen Zellen oder Normalgewebe handelt.

19. Verfahren nach Anspruch 1 oder 2 oder adjuvante Therapie nach Anspruch 3, wobei die vorbestimmten Ausschlussgrenzniveaus wenigstens eine Überexpression mit statistisch signifikantem p-Wert in der Probe mit metastatischen Zellen relativ zu gutartigen Zellen oder Normalgewebe aufweisen.

20. Verfahren oder adjuvante Therapie nach Anspruch 19, wobei der p-Wert weniger als 0,05 beträgt.

21. Verfahren nach Anspruch 1 oder 2 oder adjuvante Therapie nach Anspruch 3, wobei die Genexpression auf einem Mikroarray oder Gen-Chip gemessen wird.

22. Verfahren oder adjuvante Therapie nach Anspruch 21, wobei es sich bei dem Mikroarray um ein cDNA-Array oder ein Oligonukleotid-Array handelt.

23. Verfahren oder adjuvante Therapie nach Anspruch 21, wobei das Mikroarray bzw. der Gen-Chip ferner ein oder mehrere interne Kontrollreagentien umfasst.

24. Verfahren nach Anspruch 1 oder 2 oder adjuvante Therapie nach Anspruch 3, wobei die Genexpression durch mittels Polymerasekettenreaktion (PCR) von aus der Probe extrahierter RNA ausgeführte Nukleinsäureamplifikation bestimmt wird.

25. Verfahren oder adjuvante Therapie nach Anspruch 24, wobei es sich bei der PCR um Reverse-Transkription-Polymerasekettenreaktion (RT-PCR) handelt.

26. Verfahren oder adjuvante Therapie nach Anspruch 25, wobei die RT-PCR ferner ein oder mehrere interne Kontrollreagentien umfasst.

27. Verfahren nach Anspruch 1 oder 2 oder adjuvante Therapie nach Anspruch 3, wobei die Genexpression dadurch nachgewiesen wird, dass man ein von dem Gen codiertes Protein misst oder nachweist.

**28.** Verfahren oder adjuvante Therapie nach Anspruch 27, wobei das Protein mit einem für das Protein spezifischen Antikörper nachgewiesen wird.

**29.** Verfahren nach Anspruch 1 oder 2 oder adjuvante Therapie nach Anspruch 3, wobei die Genexpression dadurch nachgewiesen wird, dass man eine Charakteristik des Gens misst.

**30.** Verfahren oder adjuvante Therapie nach Anspruch 29, wobei die gemessene Charakteristik aus der aus DNA-Amplifikation, Methylierung, Mutation und allelischer Variation bestehenden Gruppe ausgewählt ist.

**31.** Verfahren zur Erzeugung eines Relapse Hazard Score, um die Prognose von Brustkrebspatienten zu ermöglichen, wobei man in den Verfahrensschritten:

a. Genexpressionsdaten aus einer statistisch signifikanten Anzahl biologischer Patientenproben gewinnt; b. die Daten einer univariaten Cox-Regressionsanalyse unterzieht, so dass ausgewählte Gene erhalten werden; c. gewichtete Expressionsniveaus auf die ausgewählten Gene mit Standard-Cox-Koeffizienten anwendet, so dass ein Vorhersagemodell erhalten wird, das als Relapse Hazard Score angewendet werden kann, wobei das Relapse Hazard Score über die Formel:

[FORMULA 60/20a; COR: please follow line numbers pencilled in]

erhalten wird, wobei

[FORMULA 60/20c; „if X gene level" → „wenn X-Gen-Niveau"; „per mg protein" → „pro mg Protein"]

A und B Konstanten sind,
$w_i$ der standardisierte Cox-Regressionskoeffizient für X-Gen + Marker ist,
$x_i$ der Expressionswert von ER + Marker auf der log2-Skala ist,
$w_j$ der standardisierte Cox-Regressionskoeffizient für X-Gen - Marker ist,
$x_j$ der Expressionswert von ER - Marker auf der log2-Skala ist,
X-Gen aus der aus den für mRNA :

i. entsprechend SEQ ID NO: 1-111

codierenden bestehenden Gruppe ausgewählt ist.

**32.** Verfahren zur Erstellung eines Brustkrebsprognose-Patientenberichts, wobei man in den Verfahrensschritten:

a. eine biologischeProbe von dem Patienten erhält;
b. die Genexpression der Probe misst;
c. ein Relapse Hazard Score auf die Ergebnisse aus Schritt b. anwendet; und
d. die in Schritt c. erhaltenen Ergebnisse zur Erstellung des Berichts verwendet;

wobei das Relapse Hazard Score über die Formel:

[FORMULA 61/8a; COR: please follow line numbers pencilled in]

erhalten wird, wobei

[FORMULA 61/8c; „if X gene level" → „wenn X-Gen-Niveau"; „per mg protein" → „pro mg Protein"]

A und B Konstanten sind,
$w_i$ der standardisierte Cox-Regressionskoeffizient für X-Gen + Marker ist,
$x_i$ der Expressionswert von ER + Marker auf der log2-Skala ist,
$w_j$ der standardisierte Cox-Regressionskoeffizient für X-Gen - Marker ist,
$x_j$ der Expressionswert von ER - Marker auf der log2-Skala ist,
X-Gen aus der aus den für mRNA :

    i. entsprechend SEQ ID NO: 1-111

codierenden bestehenden Gruppe ausgewählt ist.

33. Verfahren nach Anspruch 32, wobei der Bericht eine Beurteilung des Ausgangs und/oder der Risikowahrscheinlichkeit für den Patienten relativ zur Gesamtheit der Patienten enthält.

34. Zusammensetzung, umfassend einen aus allen der SEQ ID NO: 1-111 in Tabelle 10 bestehenden Sondensatz.

35. Verwendung eines Kits bei einem Verfahren zur Durchführung eines Tests zur Bestimmung der Brustkrebsprognose in einer biologischen Probe, umfassend: Materialien zum Nachweisen isolierter Nukleinsäuresequenzen, ihrer Komplemente oder von Teilen davon einer Kombination von Genen, die aus denen bestehen, die für durch alle der SEQ ID NO: 1-111 in Tabelle 10 identifizierte mRNA codieren.

36. Verwendung nach Anspruch 35, ferner umfassend Reagentien zur Durchführung einer Mikroarray-Analyse.

37. Verwendung nach Anspruch 35, ferner umfassend ein Medium, über das die Nukleinsäuresequenzen, ihre Komplemente oder Teile davon getestet werden.

38. Verwendung eines Mikroarrays oder Gen-Chips bei dem Verfahren nach einem der Ansprüche 1 oder 2 oder der adjuvanten Therapie nach Anspruch 3.

39. Verwendung nach Anspruch 38, umfassend isolierte Nukleinsäuresequenzen, ihre Komplemente oder Teile davon einer Kombination von Genen, die aus denen bestehen, die für durch SEQ ID NO: 1-111 in Tabelle 10 identifizierte mRNA codieren; worin die Kombination ausreicht, um den Brustkrebs-Status oder das Rezidivrisiko in einer biologischen Probe zu charakterisieren.

40. Verwendung nach Anspruch 38, wobei es sich bei der Messung oder Charakterisierung um wenigstens 1,5-fache Über- bzw. Unterexpression handelt.

41. Verwendung nach Anspruch 38, wobei die Messung eine Über- bzw. Unterexpression mit statistisch signifikantem p-Wert ergibt.

42. Verwendung nach Anspruch 38, wobei der p-Wert weniger als 0,05 beträgt.

43. Verwendung nach Anspruch 38, wobei das Mikroarray ein cDNA-Array oder ein Oligonukleotid-Array umfasst.

44. Verwendung nach Anspruch 38, wobei das Mikroarray ferner oder mehrere interne Kontrollreagentien umfasst.

**Revendications**

1. Procédé *in vitro* d'évaluation du statut du cancer du sein comprenant les étapes de mesure dans un échantillon biologique d'un patient atteint de cancer du sein des taux d'expression de gènes constitués de ceux codant pour un ARNm identifié par l'ensemble de SEQ ID NO: 1 à 111 dans le tableau 10 ; dans lequel les taux d'expression génique au-dessus ou au-dessous de seuils de coupure prédéterminés sont indicatifs du statut du cancer du sein.

**2.** Procédé *in vitro* de détermination d'un protocole de traitement d'un patient atteint de cancer du sein comprenant les étapes de mesure dans un échantillon biologique d'un patient atteint de cancer du sein des taux d'expressions de gènes constitués de ceux codant pour un ARNm identifié par l'ensemble de SEQ ID NO: 1 à 111 dans le tableau 10 ; dans lequel les taux d'expression génique au-dessus ou au-dessous de seuils de coupure prédéterminés sont suffisamment indicatifs du risque de récidive pour permettre à un médecin de déterminer le degré et le type de traitement recommandé pour prévenir une récidive.

**3.** Traitement adjuvant pour utilisation dans le traitement d'un patient atteint de cancer du sein à risque élevé de récidive de cancer du sein, dans lequel le patient est identifié par mesure dans un échantillon biologique d'un patient atteint de cancer du sein des taux d'expression de gènes constitués de ceux codant pour un ARNm identifié par l'ensemble des SEQ ID NO: 1 à 111 dans le tableau 10 ; les taux d'expression génique au-dessus ou au-dessous de seuils de coupure prédéterminés indiquant un risque élevé de récidive.

**4.** Procédé des revendications 1 ou 2, ou traitement adjuvant de la revendication 3, dans lesquels l'échantillon est préparé par un procédé choisi dans le groupe constitué de préparation de tissu en bloc et de microdissection par capture laser.

**5.** Procédé ou traitement adjuvant de la revendication 4, dans lesquels la préparation de tissu en bloc est obtenue à partir d'une biopsie ou d'un spécimen chirurgical.

**6.** Procédé de la revendication 1 ou 2, ou traitement adjuvant de la revendication 3 comprenant en outre la mesure du taux d'expression d'au moins un gène codant pour un ARNm identifié par SEQ ID NO: 112 à 132 dans le tableau 10.

**7.** Procédé de la revendication 1 ou 2, ou traitement adjuvant de la revendication 3 comprenant en outre la mesure du taux d'expression d'au moins un gène constitutivement exprimé dans l'échantillon.

**8.** Procédé de la revendication 1 ou 2, ou traitement adjuvant de la revendication 3 comprenant en outre la détermination du statut de récepteur d'oestrogène (ER) de l'échantillon.

**9.** Procédé ou traitement adjuvant de la revendication 8 dans lesquelles le statut ER est déterminé par mesure du taux d'expression d'au moins un gène indicatif de statut ER.

**10.** Procédé ou traitement adjuvant de la revendication 8 dans lesquels le statut ER est déterminé par mesure de la présence de ER dans l'échantillon.

**11.** Procédé ou traitement adjuvant de la revendication 10 dans lesquels la présence de ER est mesurée de façon immunohistochimique.

**12.** Procédé de la revendication 1 ou 2, ou traitement adjuvant de la revendication 3 dans lesquels l'échantillon est obtenu à partir d'une tumeur primitive.

**13.** Procédé de la revendication 1 ou 2, ou traitement adjuvant de la revendication 3 dans lesquels la spécificité est d'au moins environ 40 %.

**14.** Procédé de la revendication 1 ou 2, ou traitement adjuvant de la revendication 3 dans lesquels la sensibilité est d'au moins au moins environ 90 %.

**15.** Procédé de la revendication 1 ou 2, ou traitement adjuvant de la revendication 3 dans lesquelles le profil d'expression des gènes et comparer un profil d'expression indicatifs d'un patient en rechute.

**16.** Procédé ou traitement adjuvant de la revendication 15 dans lesquels la comparaison de profils d'expression est conduite par des procédés de reconnaissance de profil.

**17.** Procédé ou traitement adjuvant de la revendication 16 dans lesquels les procédés de reconnaissance de profils comprennent l'utilisation d'une analyse des risques proportionnels de Cox.

**18.** Procédé de la revendication 1 ou 2, ou traitement adjuvant de la revendication 3 dans lesquels les seuils de coupure prédéterminés sont une surexpression ou sous-expression d'au moins environ 1,5 fois dans l'échantillon par rapport à des cellules bénignes ou du tissu normal.

**19.** Procédé de la revendication 1 ou 2, ou traitement adjuvant de la revendication 3 dans lesquels les seuils de coupure prédéterminés ont au moins une surexpression à valeur p statistiquement significative dans l'échantillon ayant des cellules métastasiques par rapport à des cellules bénignes ou du tissu normal.

**20.** Procédé ou traitement adjuvant de la revendication 19 dans lesquels la valeur p est inférieure à 0,05.

**21.** Procédé de la revendication 1 ou 2, ou traitement adjuvant de la revendication 3 dans lesquels l'expression génique est mesurée sur une micromatrice ou une puce à ADN.

**22.** Procédé ou traitement adjuvant de la revendication 21 dans lesquels la micromatrice est une matrice d'ADNc ou une matrice d'oligonucléotides.

**23.** Procédé ou traitement adjuvant de la revendication 21 dans lesquels la micromatrice ou puce à ADN comprend en outre un ou plusieurs réactifs témoins internes.

**24.** Procédé de la revendication 1 ou 2, ou traitement adjuvant de la revendication 3 dans lesquels l'expression génique est déterminée par amplification d'acide nucléique conduite par réaction de polymérase en chaîne (PCR) d'ARN extrait de l'échantillon.

**25.** Procédé ou traitement adjuvant de la revendication 24 dans lesquels ledit PCR est la réaction de polymérase en chaîne par transcription inverse (RT-PCR).

**26.** Procédé ou traitement adjuvant de la revendication 25, dans lesquels la RT-PCR comprend en outre un ou plusieurs réactifs témoins internes.

**27.** Procédé de la revendication 1 ou 2, ou traitement adjuvant de la revendication 3 dans lesquels l'expression génique est détectée par mesure ou détection d'une protéine codée par le gène.

**28.** Procédé ou traitement adjuvant de la revendication 27 dans lesquels la protéine est détectée par un anticorps spécifique pour la protéine.

**29.** Procédé de la revendication 1 ou 2, ou traitement adjuvant de la revendication 3 dans lesquels l'expression génique est détectée par mesure d'une caractéristique du gène.

**30.** Procédé ou traitement adjuvant de la revendication 29 dans lesquels la caractéristique mesurée est choisie dans le groupe constitué de l'amplification, la méthylation, la mutation et la variation allélique d'ADN.

**31.** Procédé de génération d'un score de risque de récidive pour permettre le pronostic de patients atteints de cancer du sein comprenant les étapes de :

a. obtention de données d'expression génique à partir d'un nombre statistiquement significatif d'échantillon biologique de patients ; b. application d'une analyse de régression de Cox univariée aux données pour obtenir des gènes sélectionnés ; c. application de taux d'expression pondérés aux gènes sélectionnés avec des coefficients de Cox standard pour obtenir un modèle de prédiction qui peut être appliquée sous la forme d'un score de risque de récidive, le score de risque de récidive étant obtenu par la formule :

$$\text{Score de récidive} = A \cdot I + \sum_{i=1}^{w} I \cdot w_i x_i + B \cdot (1-I) + \sum_{j=1}^{w} (1-I) \cdot w_j x_j$$

où

$$I = \begin{cases} 1 & \text{si taux de gène X} > 10 \text{ fmol par mg de protéine} \\ 0 & \text{si taux de gène X} \leq 10 \text{ fmol par mg de protéine} \end{cases}$$

A et B sont des constantes

$w_i$ est le coefficient de régression de Cox standardisé pour gène X + marqueur

$x_i$ est la valeur d'expression de ER + marqueur en échelle log2

$w_j$ est le coefficient de régression de Cox standardisé pour gène X - marqueur

$x_j$ est la valeur d'expression de ER - marqueur en échelle log2

le gène X est choisi dans le groupe constitué de ceux codant pour un ARNm :

    i. correspondant aux SEQ ID NO: 1 à 111.

**32.** Procédé de génération d'un rapport de patient de pronostic de cancer du sein comprenant les étapes de :

    a. obtention d'un échantillon biologique du patient ;

    b. mesure de l'expression génique de l'échantillon ;

    c. application d'un score de risque de récidive aux résultats de l'étape b. ; et

    d. utilisation des résultats obtenus dans l'étape c. pour générer le rapport ;

dans lequel le score de risque de récidive est obtenu par la formule :

$$\text{Score de récidive} = A \cdot I + \sum_{i=1}^{w} I \cdot w_i x_i + B \cdot (1 - I) + \sum_{j=1}^{w} (1 - I) \cdot w_j x_j$$

où

$$I = \begin{cases} 1 & \text{si taux de gène X} > 10 \text{ fmol par mg de protéine} \\ 0 & \text{si taux de gène X} \leq 10 \text{ fmol par mg de protéine} \end{cases}$$

A et B sont des constantes

$w_i$ est le coefficient de régression de Cox standardisé pour gène X + marqueur

$x_i$ est la valeur d'expression de ER + marqueur en échelle log2

$w_j$ est le coefficient de régression de Cox standardisé pour gène X - marqueur

$x_j$ est la valeur d'expression de ER - marqueur en échelle log2

le gène X est choisi dans le groupe constitué de ceux codant pour un ARNm :

    i. correspondant aux SEQ ID NO: 1 à 111.

**33.** Procédé de la revendication 32 dans lequel le rapport contient une évaluation de l'évolution du patient et/ou la probabilité de risque par rapport à la population de patients.

**34.** Composition comprenant un ensemble de sondes constitué de l'ensemble de SEQ ID NO: 1 à 111 dans le tableau 10.

**35.** Utilisation d'un kit dans un procédé pour conduire un essai pour déterminer un pronostic de cancer du sein dans un échantillon biologique comprenant : des matériaux pour détecter des séquences d'acide nucléique isolées, leurs compléments, ou des parties de celles-ci d'une combinaison de gènes constituée de ceux codant pour un ARNm identifié par l'ensemble de SEQ ID NO: 1 à 111 dans le tableau 10.

**36.** Utilisation de la revendication 35 comprenant en outre des réactifs pour conduire une analyse sur micromatrice.

**37.** Utilisation de la revendication 35 comprenant en outre un support par l'intermédiaire duquel lesdites séquences d'acide nucléique, leurs compléments, ou des parties de celles-ci sont analysés.

**38.** Utilisation d'une micromatrice ou puce à ADN dans le procédé de l'une quelconque des revendications 1 ou 2, ou traitement adjuvant de la revendication 3.

**39.** Utilisation de la revendication 38 comprenant des séquences d'acide nucléique isolées, leurs compléments, ou des parties de celles-ci d'une combinaison de gènes constitués de ceux codant pour un ARNm identifié par SEQ ID NO: 1 à 111 dans le tableau 10 ; la combinaison étant suffisante pour caractériser le statut d'un cancer du sein ou le risque de récidive dans un échantillon biologique.

**40.** Utilisation de la revendication 38 dans laquelle la mesure ou caractérisation est une surexpression ou sous-expression d'au moins 1,5 fois.

**41.** Utilisation de la revendication 38 dans laquelle la mesure indique une surexpression ou sous-expression à valeur p statistiquement significative.

**42.** Utilisation de la revendication 38 dans laquelle la valeur p est inférieure à 0,05.

**43.** Utilisation de la revendication 38, dans laquelle la micromatrice comprend une matrice d'ADNc ou une matrice d'oligonucléotides.

**44.** Utilisation de la revendication 38, dans laquelle la micromatrice comprend en outre ou plusieurs réactifs témoins internes.

1A

Figure 1B

## A. Selection of genes

## B. Validation set 171 LNN patients

Figure 2

EP 1 721 159 B1

## A. Premenopausal

## B. Postmenopausal

## C. Tumours 10-20 mm

Figure 3

Figure 4

## Comparison of expression intensity of control genes between one-round and two-round RNA amplification

Figure 5

Figure 6

```
          ┌─────────────────────────────┐
          │     The data set, SF=1       │
          └─────────────────────────────┘
                        ↓
          ┌─────────────────────────────┐
          │    Quantile normalization    │
          └─────────────────────────────┘
                        ↓
       ┌────────────────────────────────────┐
       │  At least 2P-call filtering across data set │
       └────────────────────────────────────┘
                        ↓
        ┌──────────────────────────────────┐
        │    Principal component analysis    │
        └──────────────────────────────────┘
                        ↓
      ┌────────────────────────────────────┐
      │   Sub-clusters with distinct ER status │
      └────────────────────────────────────┘
                        ↓
   ┌────────────────────────────────────────────┐
   │  Select differentially expressed genes between sub- │
   │        clusters based on Student T-test         │
   │                                              │
   │       Bonferroni corrected P-vale < 0.05        │
   └────────────────────────────────────────────┘
                        ↓
        ┌──────────────────────────────────┐
        │  Pathway analysis: Gene Ontology   │
        └──────────────────────────────────┘
```

Data analysis workflow

# Principle Component Analysis with filtered gene sets

Bulk tissue data set

LCM data set

| ER+ | |
| --- | --- |
| ER- | |
| ND | |

\* ER-class was based on Enzyme Immunoassay. 10 fmol/mg was used as cutoff.

Figure 7

Figure 8

## Pathway analysis result

Figure 9

Figure 10

Nijmegen (86)

Munich (15)

Bari (27)

Ljubljana (4)

132 LNN patients

Good outcome —48 patients

Poor outcome —84 patients

Prognostic signature

**A.**

| Characteristics | Validation set |
|---|---|
| Number | 132 |
| Age (mean±SD) | 58±11 |
| ≤40 yr | 9 (7%) |
| 41–55 yr | 42 (32%) |
| 56–70 yr (start 55.1) | 63 (48%) |
| >70 yr | 17 (13%) |
| Unknown | 1 |
| Menopausal status | |
| Premenopausal | 31 (23%) |
| Postmenopausal | 101 (77%) |
| T stage | |
| T1 | 77 (58%) |
| T2 | 54 (41%) |
| T3/4 | 1 |
| Grade | |
| Poor | 40 (30%) |
| Moderate | 55 (42%) |
| Good | 16 (12%) |
| Unknown | 21 (16%) |
| Metastasis <5 years | |
| Yes | 24 (18%) |
| No | 108 (82%) |

**B.**

Figure 11

**132 patients**

| | Relapsers | Non-Relapsers |
|---|---|---|
| Positive | 21 | 63 |
| Negative | 3 | 44 |

Sensitivity: 88% (0.69 - 0.96)
Specificity: 41% (0.32 - 0.51)
Freq. of relapse: 18%
PPV: 25%
NPV: 94%
Odds ratio: 4.9 (1.4-17)

Figure 12

**A.**

**B.**

**A.**

**B.**

Figure 13

Figure 14

68

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20020110547 A
- US 20040009491 A
- US 20030236392 A
- WO 0149716 A
- WO 0170979 A
- WO 03025138 A
- WO 03042661 A
- WO 03016475 A
- WO 2004063355 A
- WO 2004047728 A
- WO 2004030615 A
- US 20020131971 A
- US 20030087818 A
- US 20030166064 A
- US 20040005560 A
- WO 0036107 A
- WO 0159063 A
- WO 0160860 A
- WO 0174405 A
- WO 0177288 A
- WO 02059271 A
- WO 0212328 A
- WO 0224956 A
- WO 0229086 A
- WO 2004024097 A
- WO 2004037996 A
- WO 2004039956 A
- WO 2004043361 A
- US 20020064872 A
- US 20020085998 A
- US 20020150581 A
- US 20020156011 A
- US 20020192678 A
- US 20030069180 A
- US 20030073623 A
- US 20030104366 A
- US 20040013663 A
- US 20040058340 A
- WO 0060076 A
- WO 0142467 A
- WO 0155322 A
- WO 0179286 A
- WO 196388 A
- WO 02057414 A
- WO 02059377 A
- WO 02060317 A
- WO 02086443 A
- WO 0231198 A
- WO 0250279 A
- WO 03004989 A
- WO 03062379 A
- WO 2004048938 A
- WO 9938972 A
- US 20030065157 A
- WO 0056880 A
- WO 0058473 A
- WO 02059260 A
- WO 03101283 A
- WO 2004031413 A
- WO 2004060270 A
- WO 9101999 A
- WO 0055350 A
- WO 0175067 A
- WO 0188088 A
- WO 0294629 A
- WO 03078572 A
- WO 2004028479 A
- WO 2004041170 A
- US 20030096982 A
- WO 9514772 A
- WO 9940100 A
- WO 0154472 A
- WO 0157188 A
- WO 03046152 A
- EP 1104808 B1
- WO 9845437 A
- WO 9935158 A
- WO 0142451 A
- WO 0190304 A
- WO 02090526 A
- WO 02095010 A
- WO 02102993 A
- WO 02102994 A

- WO 03004622 A
- WO 04024892 A
- WO 04041170 A
- US 20030194704 A
- US 20040110194 A
- WO 0100828 A
- WO 0204514 A
- WO 02103320 A
- WO 2004018641 A
- WO 2004023973 A
- WO 9423039 A
- US 5874283 A
- WO 0196388 A
- WO 03016500 A
- WO 03072035 A
- WO 2004022059 A
- WO 2004079014 A
- WO 02070737 A
- WO 02081731 A
- WO 02083898 A
- WO 03054152 A
- WO 9219745 A
- US 20030022239 A
- US 20040009478 A
- US 20040034196 A
- WO 0012708 A
- WO 0061610 A
- WO 0078961 A
- WO 0140466 A
- WO 0177168 A
- WO 0194629 A
- WO 02083876 A
- WO 2004024892 A
- WO 2004031414 A
- WO 2004053081 A
- US 20020151681 A
- WO 0055174 A
- WO 03016476 A
- WO 2004039943 A
- US 6060283 A
- US 20040115636 A
- WO 9724441 A
- WO 0021991 A
- WO 02094988 A
- WO 2004003162 A
- WO 9712967 A
- WO 9964576 A
- WO 0146697 A
- WO 02102235 A
- WO 03010336 A
- WO 03027285 A
- US 20030219744 A
- WO 0144448 A
- WO 0153312 A
- WO 0157182 A
- WO 0127158 A
- WO 02068652 A

- WO 02081498 A
- WO 02081517 A
- WO 030000735 A
- WO 03091388 A
- WO 9810067 A
- WO 9958642 A
- WO 0060078 A
- DE 4329177
- US 20020081659 A
- WO 0055320 A
- WO 03003906 A
- WO 03018807 A
- WO 2004042022 A
- WO 2004070062 A
- WO 0063438 A
- WO 0202623 A
- WO 02036766 A
- WO 02074237 A
- WO 0228999 A
- US 20020123619 A
- US 20020168637 A
- WO 0190154 A
- WO 2003094848 A
- WO 2004074301 A
- US 6171787 B
- US 6440694 B
- US 20020055474 A
- US 20020072089 A
- US 20030219767 A
- WO 9733902 A
- WO 9912965 A
- WO 9926976 A
- WO 9928462 A
- WO 9933980 A
- WO 9935170 A
- WO 9950416 A
- WO 9954460 A
- WO 0026244 A
- WO 0032776 A
- WO 012525 A
- WO 0177291 A
- WO 2004020593 A
- WO 2004033637 A
- WO 04037996 A
- US 20040037842 A
- WO 9933981 A
- WO 9947540 A
- WO 0173027 A
- WO 02076488 A
- WO 03038063 A
- WO 03064589 A
- US 6136182 A
- US 5445934 A
- US 5532128 A
- US 5556752 A
- US 5242974 A
- US 5384261 A
- US 5405783 A

- US 5412087 A
- US 5424186 A
- US 5429807 A
- US 5436327 A
- US 5472672 A
- US 5527681 A
- US 5529756 A
- US 5545531 A
- US 5554501 A
- US 5561071 A
- US 5571639 A
- US 5593839 A
- US 5599695 A
- US 5624711 A
- US 5658734 A
- US 5700637 A
- US 6271002 B
- US 6218122 B
- US 6218114 B
- US 6004755 B
- US 20030194734 A
- WO 20030069180 A
- WO 01053312 A
- WO 04030615 A
- WO 20030073623 A
- WO 01054472 A
- WO 04031413 A
- WO 20030087818 A
- WO 01055322 A
- WO 04031414 A
- WO 20030096982 A
- WO 01057182 A
- WO 04033637 A
- WO 20030104366 A
- WO 01057188 A
- WO 20030124128 A
- WO 01059063 A
- WO 03000735 A
- WO 04039943 A
- WO 20030124148 A
- WO 01060860 A
- WO 04039956 A
- WO 20030128884 A
- WO 01070979 A
- WO 20030166064 A
- WO 01073027 A
- WO 04042022 A
- WO 20030194704 A
- WO 01075067 A
- WO 04043361 A
- WO 20030219744 A
- WO 01077168 A
- WO 04047728 A
- WO 20030219767 A
- WO 01077288 A
- WO 04048938 A
- WO 20030236392 A
- WO 01077291 A
- WO 04053081 A
- WO 20040005560 A
- WO 01088088 A
- WO 04060270 A
- WO 20040009478 A
- WO 01090154 A
- WO 04063355 A
- WO 20040009491 A
- WO 01090304 A
- WO 03027258 A
- WO 04070062 A
- WO 20040013663 A
- WO 01094629 A
- WO 04072265 A
- WO 20040034196 A
- WO 02002623 A
- WO 04074301 A
- WO 20040037842 A
- WO 02004514 A
- WO 04076613 A
- WO 20040058340 A
- WO 02012235 A
- WO 04079014 A
- WO 20040110194 A
- WO 02012328 A
- WO 91001999 A
- WO 20040115636 A
- WO 02024956 A
- WO 92019745 A
- WO 5874283 A
- DE 432977
- WO 02028999 A
- WO 94023039 A
- US 6004755 A
- EP 1104808 A
- WO 02029086 A
- WO 95014772 A
- WO 6060283 A
- WO 00021991 A
- WO 02031198 A
- WO 97012967 A
- WO 00026244 A
- WO 97024441 A
- WO 6171787 A
- WO 00032776 A
- WO 02050279 A
- WO 03081201 A
- WO 97033902 A
- WO 6218114 A
- WO 00036107 A
- WO 98010067 A
- WO 6218122 A
- WO 00055320 A
- WO 03094848 A
- WO 98045437 A
- WO 6271002 A
- WO 00055350 A
- WO 99026976 A

- WO 6440694 A
- WO 00056880 A
- WO 04003162 A
- WO 90033980 A
- WO 200200131971 A
- WO 00058473 A
- WO 04018641 A
- WO 99033981 A
- WO 20020055474 A
- WO 00060078 A
- WO 04020497 A
- WO 99035158 A
- WO 20020064872 A
- WO 00061610 A
- WO 04020593 A
- WO 99035170 A
- WO 20030065157 A
- WO 00063438 A
- WO 04022059 A
- WO 99038972 A
- WO 20020072089 A
- WO 00078961 A
- WO 04023973 A
- WO 99047540 A
- WO 20020081659 A

- WO 01000828 A
- WO 04024097 A
- WO 99050416 A
- WO 20020110547 A
- WO 01025256 A
- WO 99054460 A
- WO 20020123619 A
- WO 01027158 A
- WO 04028479 A
- WO 99058642 A
- WO 20020150581 A
- WO 01040466 A
- WO 99064576 A
- WO 20020151681 A
- WO 01042451 A
- WO 20020168637 A
- WO 01042467 A
- WO 20020192678 A
- WO 01044448 A
- WO 20030194734 A
- WO 01046697 A
- WO 02094629 A
- WO 20030022239 A
- WO 01049716 A

**Non-patent literature cited in the description**

- **AHR et al.** Identification of high risk breast-cancer patients by gene-expression profiling. *Lancet,* 2002, vol. 359, 131-132
- **CHANG et al.** Gene expression profiling for the prediction of therapeutic response to docetaxel in patients with breast cancer. *Lancet,* 2003, vol. 362, 362-9
- Effects of radiotherapy and surgery in early breast cancer. An overview of the randomized trials. *N Engl J Med,* 1995, vol. 333, 1444-1455
- Polychemotherapy for early breast cancer: an overview of the randomized trials. *Lancet,* 1998, vol. 352, 930-942
- Tamoxifen for early breast cancer: an overview of randomized trials. *Lancet,* 1998, vol. 351, 1451-1467
- **EFRON.** Censored data and the bootstrap. *J Am Stat Assoc,* 1981, vol. 76, 312-319
- **EIFEL et al.** National Institutes of Health Consensus Development Conference Statement: adjuvant therapy for breast cancer. *J Natl Cancer Inst,* 01 November 2000, vol. 93, 979-989
- **FOEKENS et al.** Prognostic value of estrogen and progesterone receptors measured by enzyme immunoassays in human breast tumor cytosols. *Cancer Res,* 1989, vol. 49, 5823-5828

- **FOEKENS et al.** Prognostic value of receptors for insulin-like growth factor 1, somatostatin, and epidermal growth factor in human breast cancer. *Cancer Res,* 1989, vol. 49, 7002-7009
- **GOLDHIRSCH et al.** Meeting highlights: Updated International Expert Consensus on the Primary Therapy of Early Breast Cancer. *J Clin Oncol,* 2003, vol. 21, 3357-3365
- **GOLUB et al.** Molecular classification of cancer: class discovery and class prediction by gene expression monitoring. *Science,* 1999, vol. 286, 531-537
- **GRUVBERGER et al.** Estrogen receptor status in breast cancer is associated with remarkably distinct gene expression patterns. *Cancer Res,* 2001, vol. 61, 5979-5984
- **HEDENFALK et al.** Gene-expression profiles in hereditary breast cancer. *N Engl J Med,* 2001, vol. 344, 539-548
- **HERRERA-GAYOL et al.** Adhesion proteins in the biology of breast cancer: contribution of CD44. *Exp Mol Pathol,* 1999, vol. 66, 149-156
- **HUANG et al.** Gene expression predictors of breast cancer outcomes. *Lancet,* 2003, vol. 361, 1590-1596
- **KAPLAN et al.** Non-parametric estimation of incomplete observations. *J Am Stat Assoc,* 1958, vol. 53, 457-481

- **KEYOMARSI et al.** Cyclin E and survival in patients with breast cancer. *N Engl J Med,* 2002, vol. 347, 1566-1575
- **LIPSHUTZ et al.** High density synthetic oligonucleotide arrays. *Nat Genet,* 1999, vol. 21, 20-24
- **MA et al.** Gene expression profiles of human breast cancer progression. *Proc Natl Acad Sci USA,* 2003, vol. 100, 5974-5979
- **NTZANI et al.** Predictive ability of DNA microarrays for cancer outcomes and correlates: an empirical assessment. *Lancet,* 2003, vol. 362, 1439-1444
- **PEROU et al.** Molecular portraits of human breast tumors. *Nature,* 2000, vol. 406, 747-752
- **RAMASWAMY et al.** Multiclass cancer diagnosis using tumor gene expression signatures. *Proc Natl Acad Sci USA,* 2001, vol. 98, 15149-15154
- **RAMASWAMY et al.** A molecular signature of metastasis in primary solid tumors. *Nat Genet,* 2003, vol. 33, 1-6
- **RANSOHOFF.** Rules of evidence for cancer molecular-marker discovery and validation. *Nat Rev Cancer,* 2004, vol. 4, 309-314
- **SØRLIE et al.** Gene expression patterns of breast carcinomas distinguish tumor subclasses with clinical implications. *Proc Natl Acad Sci USA,* 2001, vol. 98, 10869-10874
- **SØRLIE et al.** Repeated observation of breast tumor subtypes in independent gene expression data sets. *Proc Natl Acad Sci USA,* 2003, vol. 100, 8418-8423
- **SOTIRIOU et al.** Gene expression profiles derived from fine needle aspiration correlate with response to systemic chemotherapy in breast cancer. *Breast Cancer Res,* 2003, vol. 4, R3
- **SOTIRIOU et al.** Breast cancer classification and prognosis based on gene expression profiles from a population-based study. *Proc Natl Acad Sci USA,* 2003, vol. 100, 10393-10398
- **SU et al.** Molecular classification of human carcinomas by use of gene expression signatures. *Cancer Res,* 2001, vol. 61, 7388-7393
- **VAN DE VIJVER et al.** A gene expression signature as a predictor of survival in breast cancer. *N Engl J Med,* 2002, vol. 347, 1999-2009
- **VAN'T VEER et al.** Gene expression profiling predicts clinical outcome of breast cancer. *Nature,* 2002, vol. 415, 530-536
- **WANG et al.** Gene expression profiles and molecular markers to predict recurrence of Dukes' B colon cancer. *J Clin Oncol,* 2004, vol. 22, 1564-1571
- **WOELFLE et al.** Molecular signature associated with bone marrow micrometastasis in human breast cancer. *Cancer Res,* 2003, vol. 63, 5679-5684